# EUROPEAN PATENT SPECIFICATION

(11) **EP 1 343 766 B1**
(45) Date of publication and mention of the grant of the patent: **27.07.2005**
(21) Application number: 01990192.5
(22) Date of filing: 07.12.2001
(51) Int. Cl.: C07D 215/56, C07D 401/04, C07D 513/04, C07D 471/04, C07D 498/06

(54) **CYCLIZATION PROCESS STEP IN THE MAKING OF QUINOLONES AND NAPHTHYRIDINES**
CYCLISIERUNGSVERFAHRENSSCHRITT BEI DER HERSTELLUNG VON CHINOLONEN UND NAPHTHYRIDINEN
ETAPE DE PROCESSUS DE CYCLISATION DANS LA FABRICATION DE QUINOLONES ET DE NAPHTHYRIDINES

(30) Priority: 14.12.2000 US 255633 P
(43) Date of publication of application: 17.09.2003
(73) Proprietor: THE PROCTER & GAMBLE COMPANY, Cincinnati, Ohio 45202 (US)
(72) Inventor: MUNDLA, Sreenivasa, Reddy, Westfield, IN 46074 (US); RANDALL, Jared, Lynn, Smyrna, NY 13464 (US)
(74) Representative: Clemo, Nicholas Graham
(86) International application number: PCT/US2001/048536
(87) International publication number: WO 2002/048113

(56) References cited:
- WO-A-96/04247
- WO-A-96/04286
- US-A- 5 703 231

## Description

The subject invention relates to processes for making quinolones and quinolone derivatives, which are compounds that are active antibacterial and/or are anti-HIV agents. The invention also relates to useful intermediates in making these compounds.

### BACKGROUND OF THE INVENTION

The chemical and medical literature describes compounds that are said to be antimicrobial, i.e., capable of destroying or suppressing the growth or reproduction of microorganisms, such as bacteria. For example, such antibacterials and other antimicrobials are described in Antibiotics, Chemotherapeutics, and Antibacterial Agents for Disease Control (M. Grayson, editor, 1982), and E. Gale et al., The Molecular Basis of Antibiotic Action 2d edition (1981).

The mechanism of action of these antibacterials vary. However, they are generally believed to function in one or more of the following ways: by inhibiting cell wall synthesis or repair; by altering cell wall permeability; by inhibiting protein synthesis; or by inhibiting synthesis of nucleic acids. For example, beta-lactam antibacterials act through inhibiting the essential penicillin binding proteins (PBPs) in bacteria, which are responsible for cell wall synthesis. As another example, quinolones act, at least in part, by inhibiting synthesis of DNA, thus preventing the cell from replicating.

The pharmacological characteristics of antimicrobials, and their suitability for any given clinical use, vary. For example, the classes of antimicrobials (and members within a class) may vary in 1) their relative efficacy against different types of microorganisms, 2) their susceptibility to development of microbial resistance and 3) their pharmacological characteristics, such as their bioavailability, and biodistribution. Accordingly, selection of an appropriate antibacterial (or other antimicrobial) in a given clinical situation requires analysis of many factors, including the type of organism involved, the desired method of administration, the location of the infection to be treated and other considerations.

Cyclization processes for making intermediate compounds useful in the synthesis of quinolone, naphthyridine, and related compounds are disclosed in a number of references including the following: WO 96/04286, European Patent Application No. 0 168,733 published January 22, 1986; and U.S. Patent No. 5,703,231 issued December 30, 1997. While the methods disclosed in the those publications represent useful advances in quinolone chemistry, Applicants have discovered that the use of certain leaving groups, not contemplated in those or other prior art references, in combination with the use of a silylating reactant provide several advantages relative to the processes disclosed in the prior art. For example, the present process allows the synthesis of various quinolones and related compounds by an intramolecular cyclization process in which the key leaving group on the starting aromatic ring precursor (depicted as XR⁹ in Formula (A) below) is electron donating in nature. The aromatic ring precursor may contain other substituents which may be electron donating or electron withdrawing in nature. Certain prior cyclization methods to form quinolones disclose an electron withdrawing group as the leaving group on the starting aromatic ring and also may require the presence of other electron withdrawing groups at the ortho or para positions on that ring. See, e.g., U.S. Patent No. 5,703,231. Further, when other prior art has discussed the use of methoxy and thiomethyl leaving groups, reaction conditions disclosed are harsh insofar as they use sodium hydride and require high temperatures (140-160°C) in polar solvents.

The present process, in contrast, allows the use of a broader group of starting materials in the manufacture of quinolones, possibly leading to a more efficient and cost effective process. The process also allows the use of less harsh reaction conditions than the methods described in the art generally, which may also provide improved synthetic yields.

Accordingly, the present invention provides an improved means to obtain quinolones and derivatives of quinolones, which themselves may be active or may be intermediates for forming other active molecules.

### SUMMARY OF THE INVENTION

The subject invention relates to a process for making a compound having a structure according to Formula (I), or an optical isomer, diastereomer or enantiomer thereof, or a pharmaceutically-acceptable salt, hydrate, or biohydrolyzable ester, amide or imide thereof: 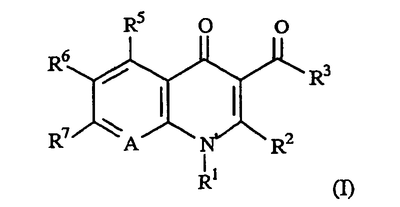 the process comprising reacting one or more organosilicon reagents with a compound having a structure according to Formula (A): 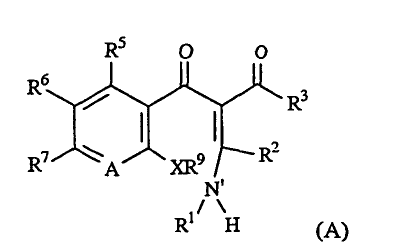 wherein with regard to Formula (I) and Formula (A):
(A)
   (1) A is N or C-R⁸, where R⁸ is selected from hydrogen, alkyl, aryl, halo, a heterocyclic ring, amino, alkylamino, arylamino, alkoxy, nitro, cyano, aryloxy, esters of hydroxy, alkylthio, arylthio, aryloxy, esters of thio, alkylsulfonyl, arylsulfonyl, alkylphosphonyl, arylphosphonyl, alkylacyl, arylacyl, and aryl esters and amides of carboxy;
   (2) R⁷ is selected from hydrogen, alkyl, aryl, a heterocyclic ring, amino, alkylamino, arylamino, halo, nitro, cyano, alkoxy, aryloxy, esters of hydroxy, alkylthio, arylthio, esters of thio, alkylsulfonyl, arylsulfonyl, alkylphosphonyl, arylphosphonyl, alkylacyl, arylacyl, and alkyl and aryl esters and amides of carboxy;
   (3) R⁶ is selected from hydrogen, halo, alkyl, aryl, a heterocyclic ring, amino, alkylamino, arylamino, nitro, cyano, alkoxy, aryloxy, esters of hydroxy, alkylthio, arylthio, esters of thio, alkylsulfonyl, arylsulfonyl, alkylphosphonyl, arylphosphonyl, alkylacyl, arylacyl, and alkyl and aryl esters and amides of carboxy;
   (4) R⁵ is selected from hydrogen, alkyl, aryl, cyano, a heterocyclic ring, amino, alkylamino, arylamino, alkylacyl, arylacyl, and aryl esters and amides of carboxy;
   (5) R¹ is selected from a carbocyclic ring, a heterocyclic ring, lower alkyl, lower alkene, lower alkyne and -CH(R¹⁰)(R¹¹) where R¹⁰ is selected from lower alkyl and phenyl and R¹¹ is -CH₂Y(O=)CR¹² where R¹² is selected from lower alkyl and phenyl and Y is selected from -NH-, -O- and -S-;
   (6) R² is selected from hydrogen, alkyl, aryl, a heterocyclic ring, alkylthio and arylthio; and
   (7) R³ is selected from hydrogen, alkoxy, aryloxy, alkyl and aryl; or
(B) R¹ and R² can join to form a 5- or 6-membered carbocyclic or heterocyclic ring, where A, R³, R⁵, R⁶, R⁷ and R⁸, if present, are as described in (A); or
(C) R⁶ and R⁷ can join to form a 5- or 6-membered carbocyclic or heterocyclic ring, where A, R¹, R², R³, R⁵ and R⁸, if present, are as described in (A);
   and wherein with regard to Formula (A):
(D) X is selected from -O- and -S- and R⁹ is selected from C₁-C₁₀ alkyl, aryl and heteroaryl.

The compounds of Formula (I) may themselves be effective antimicrobial or anti-HIV agents, or they may be further reacted using well known chemistry to provide a molecule having antimicrobial or anti-HIV activity. As such, the compounds of Formula (I) may be useful intermediates in the formation of other active quinolones and quinolone derivatives.

### DETAILED DESCRIPTION OF THE INVENTION

### I. Terms and Definitions:

The following is a list of definitions for terms used herein:
"Acyl" or "carbonyl" is a radical formed by removal of the hydroxy from a carboxylic acid (i.e., R-C(=O)-). "Alkylacyl" is -C(=O)-alkyl and "Arylacyl is -C(=O)-aryl. Preferred acyl groups include (for example) acetyl, formyl, and propionyl.
"Alkyl" is a saturated hydrocarbon chain having 1 to 15 carbon atoms, preferably 1 to 10, more preferably 1 to 4 carbon atoms. "Alkene" is a hydrocarbon chain having at least one (preferably only one) carbon-carbon double bond and having 2 to 15 carbon atoms, preferably 2 to 10, more preferably 2 to 4 carbon atoms. "Alkyne" is a hydrocarbon chain having at least one (preferably only one) carbon-carbon triple bond and having 2 to 15 carbon atoms, preferably 2 to 10, more preferably 2 to 4 carbon atoms. Alkyl, alkene and alkyne chains (referred to collectively as "hydrocarbon chains") may be straight or branched and may be unsubstituted or substituted. Preferred branched alkyl, alkene and alkyne chains have one or two branches, preferably one branch. Preferred chains are alkyl. Alkyl, alkene and alkyne hydrocarbon chains each may be unsubstituted or substituted with from 1 to 4 substituents; when substituted, preferred chains are mono-, di-, or tri-substituted. Alkyl, alkene and alkyne hydrocarbon chains each may be substituted with halo, hydroxy, aryloxy (*e*.*g*., phenoxy), heteroaryloxy, acyloxy (e.g., acetoxy), carboxy, aryl (*e.g.*, phenyl), heteroaryl, cycloalkyl, heterocycloalkyl, spirocycle, amino, amido, acylamino, keto, thioketo, cyano, or any combination thereof. Preferred hydrocarbon groups include methyl, ethyl, propyl, isopropyl, butyl, vinyl, allyl, butenyl, and exomethylenyl.
"Alkoxy" is an oxygen radical having a hydrocarbon chain substituent, where the hydrocarbon chain is an alkyl or alkenyl (i.e., -O-alkyl or -O-alkenyl) that is unsubstituted or substituted as described above. In the case of substituted alkoxy, preferred substituents include 1-5 fluorine atoms. Preferred alkoxy groups include (for example) methoxy, di-fluoro methoxy, ethoxy, penta-fluoro ethoxy, propoxy and allyloxy.

Also, as referred to herein, a "lower" alkoxy, alkyl, alkene or alkyne moiety (e.g., "lower alkyl") is a chain comprised of 1 to 6, preferably from 1 to 4, carbon atoms in the case of alkyl and alkoxy, and 2 to 6, preferably 2 to 4, carbon atoms in the case of alkene and alkyne.

"Alkylphosphonyl" is -PO₃-alkyl (e.g. -PO₃-CH₃).

"Alkylsulfonyl" is -SO₂-alkyl (e.g., -SO₂-CH₃).

"Alkylthio" is -S-alkyl (e.g. -S-CH₃).

"Amino" refers to -NH₂. "Alkylamino" is an amino substituted with at least one alkyl moiety (e.g., -NH(CH₃). "Arylamino" is an amino substituted with at least one aryl moiety (e.g., -NH(C₆H₅).

"Aryl" is an aromatic hydrocarbon ring. Aryl rings are monocyclic or fused bicyclic ring systems. Monocyclic aryl rings contain 6 carbon atoms in the ring. Monocyclic aryl rings are also referred to as phenyl rings. Bicyclic aryl rings contain from 8 to 17 carbon atoms, preferably 9 to 12 carbon atoms, in the ring. Bicyclic aryl rings include ring systems wherein one ring is aryl and the other ring is aryl, cycloalkyl, or heterocycloakyl. Preferred bicyclic aryl rings comprise 5-, 6- or 7-membered rings fused to 5-, 6-, or 7-membered rings. Aryl rings may be unsubstituted or substituted with from 1 to 4 substituents on the ring. Aryl may be substituted with halo, cyano, nitro, hydroxy, carboxy, amino, acylamino, alkyl, heteroalkyl, haloalkyl, phenyl, aryloxy, alkoxy, heteroalkyloxy, carbamyl, haloalkyl, methylenedioxy, heteroaryloxy, or any combination thereof. Preferred aryl rings include naphthyl, tolyl, xylyl, and phenyl. The most preferred aryl ring radical is phenyl.

"Aryloxy" is an oxygen radical having an aryl substituent (i.e., -O-aryl). Preferred aryloxy groups include (for example) phenoxy, napthyloxy, methoxyphenoxy, and methylenedioxyphenoxy.

"Arylphosphonyl" is -PO₃-aryl (e.g., - PO₃-C₆H₅).

"Arylsulfonyl" is -SO₂-aryl (e.g., -SO₂-C₆H₅).

"Arylthio" is -S-aryl (e.g., -S-C₆H₅).

"Biohydrolyzable amides" are aminoacyl, acylamino, or other amides of the compounds of the invention, where the amide does not essentially interfere, preferably does not interfere, with the activity of the compound, or where the amide is readily converted in vivo by a host to yield an active compound.

"Biohydrolyzable imides" are imides of compounds of the invention, where the imide does not essentially interfere, preferably does not interfere, with the activity of the compound, or where the imide is readily converted in vivo by a host to yield an active compound. Preferred imides are hydroxyimides.

"Biohydrolyzable esters" are esters of compounds of the invention, where the ester does not essentially interfere, preferably does not interfere, with the antimicrobial activity of the compound, or where the ester is readily converted in a host to yield an active compound. Many such esters are known in the art, as described in U.S. Patent No. 4,783,443, issued to Johnston and Mobashery on November 8, 1988 (incorporated by reference herein). Such esters include lower alkyl esters, lower acyloxy-alkyl esters (such as acetoxymethyl, acetoxyethyl, aminocarbonyloxymethyl, pivaloyloxymethyl and pivaloyloxyethyl esters), lactonyl esters (such as phthalidyl and thiophthalidyl esters), lower alkoxyacyloxyalkyl esters (such as methoxycarbonyloxymethyl, ethoxycarbonyloxyethyl and isopropoxycarbonyloxyethyl esters), alkoxyalkyl esters, choline esters and alkylacylaminoalkyl esters (such as acetamidomethyl esters).

"Carbocyclic ring" encompasses both cycloalkyl and aryl moieties, as those terms are defined herein.

"Carbonyl" is -C(=O)-.

"Cycloalkyl" is a saturated or unsaturated hydrocarbon ring. Cycloalkyl rings are not aromatic. Cycloalkyl rings are monocyclic, or are fused, spiro, or bridged bicyclic ring systems. Monocyclic cycloalkyl rings contain from about 3 to about 9 carbon atoms, preferably from 3 to 7 carbon atoms, in the ring. Bicyclic cycloalkyl rings contain from 7 to 17 carbon atoms, preferably from 7 to 12 carbon atoms, in the ring. Preferred bicyclic cycloalkyl rings comprise 4-, 5-, 6- or 7-membered rings fused to 5-, 6-, or 7-membered rings. Cycloalkyl rings may be unsubstituted or substituted with from 1 to 4 substituents on the ring. Cycloalkyl may be substituted with halo, cyano, alkyl, heteroalkyl, haloallcyl, phenyl, keto, hydroxy, carboxy, amino, acylamino, aryloxy, heteroaryloxy, or any combination thereof. Preferred cycloalkyl rings include cyclopropyl, cyclopentyl, and cyclohexyl.

"Halo" or "halogen" is fluoro, chloro, bromo or iodo. Preferred halo are fluoro, chloro and bromo; more preferred typically are chloro and fluoro, especially fluoro.

"Haloalkyl" is a straight, branched, or cyclic hydrocarbon substituted with one or more halo substituents. Preferred are C₁-C₁₂ haloalkyls; more preferred are C₁-C₆ haloalkyls; still more preferred still are C₁-C₃ haloalkyls. Preferred halo substituents are fluoro and chloro. The most preferred haloalkyl is trifluoromethyl.

"Heteroatom" is a nitrogen, sulfur, or oxygen atom. Groups containing more than one heteroatom may contain different heteroatoms.

"Heteroalkyl" is a saturated or unsaturated chain containing carbon and at least one heteroatom, wherein no two heteroatoms are adjacent. Heteroalkyl chains contain from 2 to 15 member atoms (carbon and heteroatoms) in the chain, preferably 2 to 10, more preferably 2 to 5. For example, alkoxy (i.e., -O-alkyl or -O-heteroalkyl) radicals are included in heteroalkyl. Heteroalkyl chains may be straight or branched. Preferred branched heteroalkyl have one or two branches, preferably one branch. Preferred heteroalkyl are saturated. Unsaturated heteroalkyl have one or more carbon-carbon double bonds and/or one or more carbon-carbon triple bonds. Preferred unsaturated heteroalkyls have one or two double bonds or one triple bond, more preferably one double bond. Heteroalkyl chains may be unsubstituted or substituted with from 1 to 4 substituents. Preferred substituted heteroalkyl are mono-, di-, or tri-substituted. Heteroalkyl may be substituted with lower alkyl, haloalkyl, halo, hydroxy, aryloxy, heteroaryloxy, acyloxy, carboxy, monocyclic aryl, heteroaryl, cycloalkyl, heterocycloalkyl, spirocycle, amino, acylamino, amido, keto, thioketo, cyano, or any combination thereof.

"Heteroaryl" is an aromatic ring containing carbon atoms and from 1 to about 6 heteroatoms in the ring. Heteroaryl rings are monocyclic or fused bicyclic ring systems. Monocyclic heteroaryl rings contain from about 5 to about 9 member atoms (carbon and heteroatoms), preferably 5 or 6 member atoms, in the ring. Bicyclic heteroaryl rings contain from 8 to 17 member atoms, preferably 8 to 12 member atoms, in the ring. Bicyclic heteroaryl rings include ring systems wherein one ring is heteroaryl and the other ring is aryl, heteroaryl, cycloalkyl, or heterocycloalkyl. Preferred bicyclic heteroaryl ring systems comprise 5-, 6-or 7-membered rings fused to 5-, 6-, or 7-membered rings. Heteroaryl rings may be unsubstituted or substituted with from 1 to 4 substituents on the ring. Heteroaryl may be substituted with halo, cyano, nitro, hydroxy, carboxy, amino, acylamino, alkyl, heteroalkyl, haloalkyl, phenyl, alkoxy, aryloxy, heteroaryloxy, or any combination thereof. Preferred heteroaryl rings include, but are not limited to, the following:

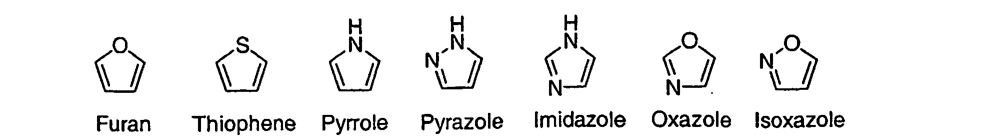

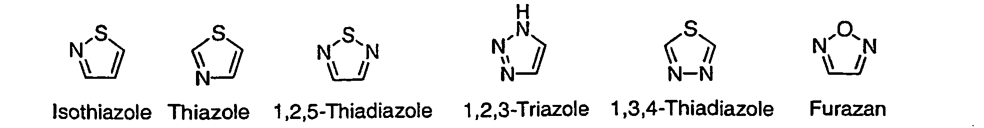

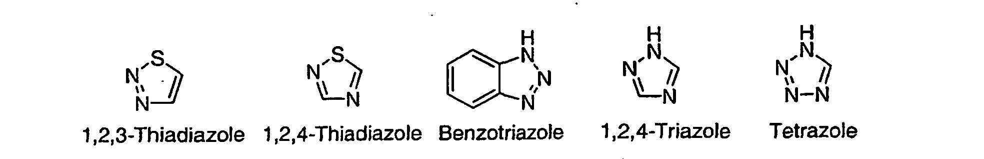

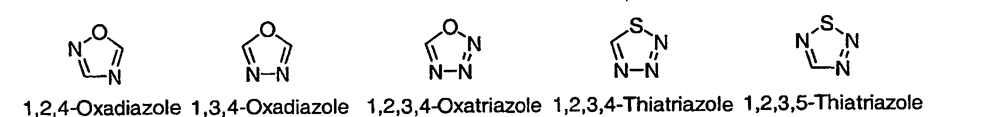

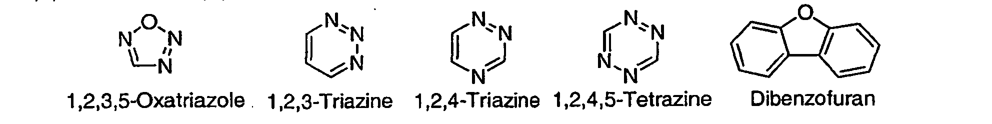

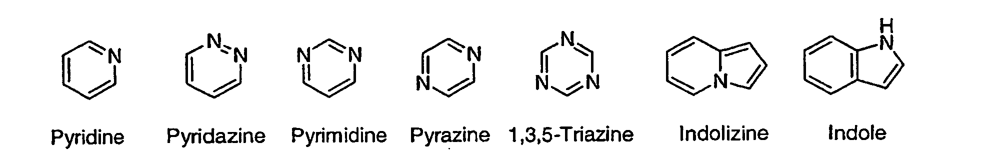

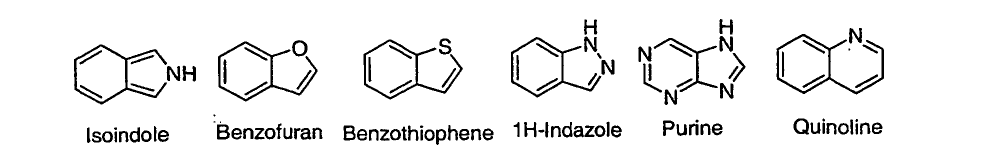

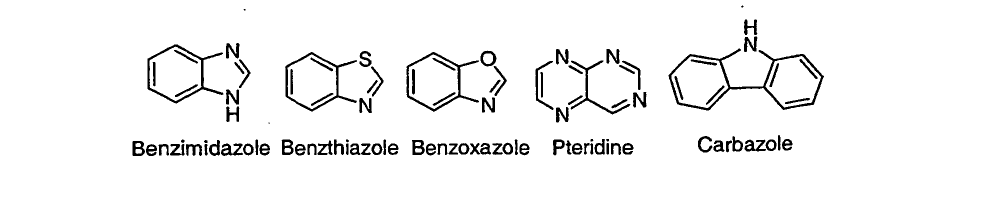

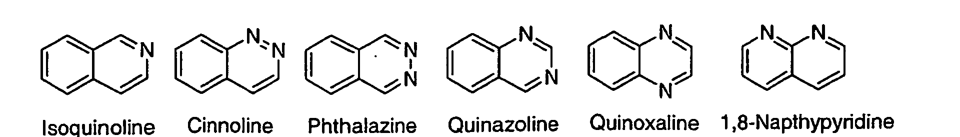

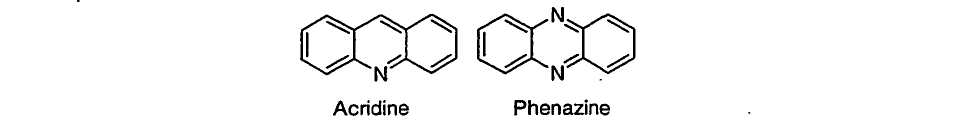

"Heteroaryloxy" is an oxygen radical having a heteroaryl substituent (i.e., -O-heteroaryl). Preferred heteroaryloxy groups include (for example) pyridyloxy, furanyloxy, (thiophene)oxy, (oxazole)oxy, (thiazole)oxy, (isoxazole)oxy, pyrmidinyloxy, pyrazinyloxy, and benzothiazolyloxy.

"Heterocycloalkyl" is a saturated or unsaturated ring containing carbon atoms and from 1 to about 4 (preferably 1 to 3) heteroatoms in the ring. Heterocycloalkyl rings are not aromatic. Heterocycloalkyl rings are monocyclic or bicyclic ring systems. Monocyclic heterocycloalkyl rings contain from about 3 to about 9 member atoms (carbon and heteroatoms), preferably from 5 to 7 member atoms, in the ring. Bicyclic heterocycloalkyl rings contain from 7 to 17 member atoms, preferably 7 to 12 member atoms, in the ring. Bicyclic heterocycloalkyl rings contain from about 7 to about 17 ring atoms, preferably from 7 to 12 ring atoms. Bicyclic heterocycloalkyl rings may be fused, spiro, or bridged ring systems. Preferred bicyclic heterocycloalkyl rings comprise 5-, 6- or 7-membered rings fused to 5-, 6-, or 7-membered rings. Heterocycloalkyl rings may be unsubstituted or substituted with from 1 to 4 substituents on the ring. Heterocycloalkyl may be substituted with halo, cyano, hydroxy, carboxy, keto, thioketo, amino, acylamino, acyl, amido, alkyl, heteroallcyl, haloalkyl, phenyl, alkoxy, aryloxy or any combination thereof. Preferred substituents on heterocycloalkyl include halo and haloalkyl. Preferred heterocycloalkyl rings include, but are not limited to, the following:

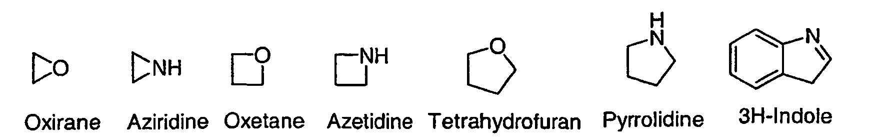

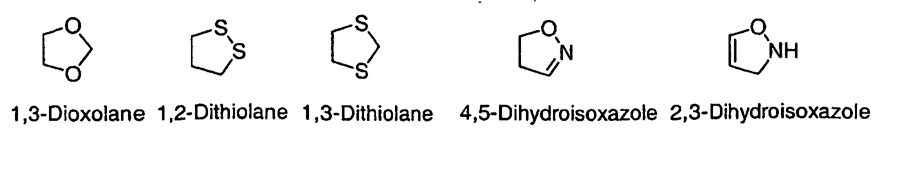

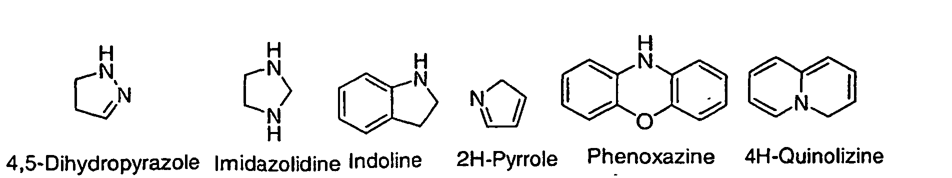

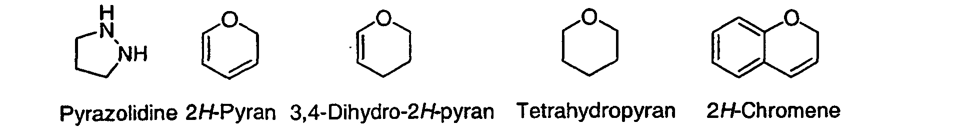

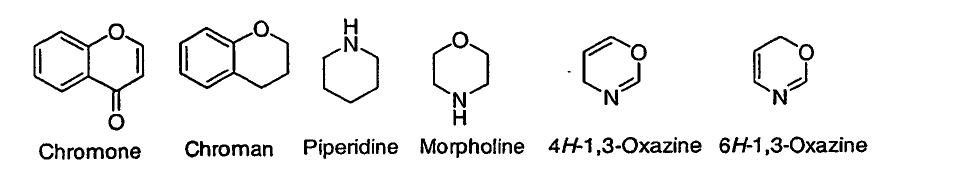

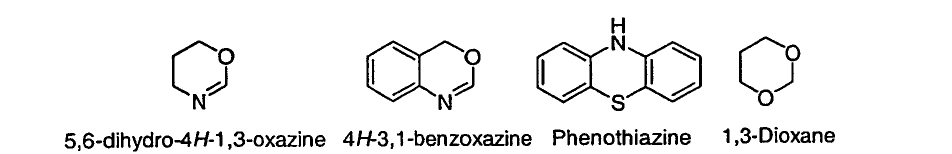

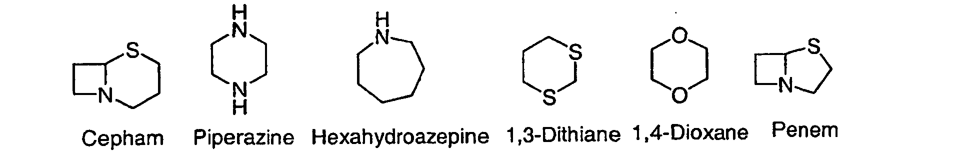

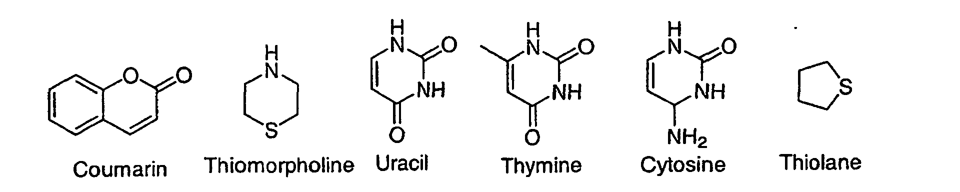

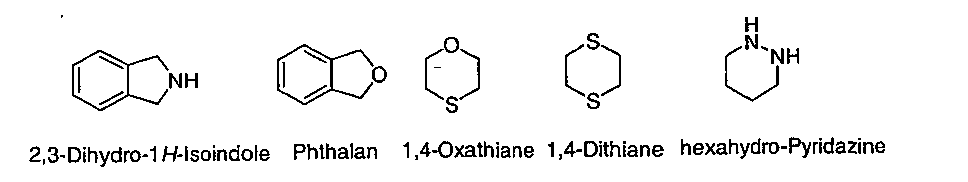

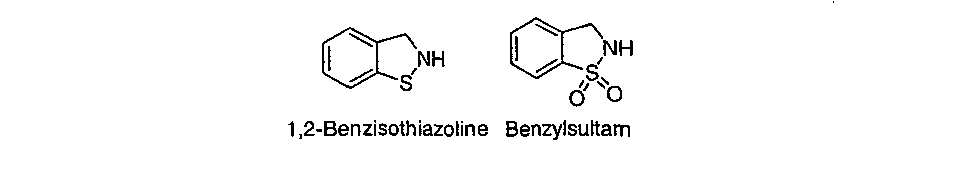

"Heterocyclic ring" encompasses both heterocycloalkyl and heteroaryl moieties, as those terms are defined herein.

"Host" is a substrate capable of sustaining a microbe, preferably it is a living organism, more preferably an animal, more preferably a mammal, more preferably still a human.

The terms "optical isomer", "stereoisomer", and "diastereomer" have the standard art recognized meanings (see, e.g., Hawley's Condensed Chemical Dictionary, 11th Ed.). The illustration of specific protected forms and other derivatives of the compounds of the instant invention is not intended to be limiting. The application of other useful protecting groups, salt forms, etc. is within the ability of the skilled artisan.

The compounds of the invention may have one or more chiral centers. As a result, one may selectively prepare one optical isomer, including diastereomer and enantiomer, over another, for example by use of chiral starting materials, catalysts or solvents, one may prepare both stereoisomers or both optical isomers, including diastereomers and enantiomers at once (a racemic mixture). Since the compounds of the invention may exist as racemic mixtures, mixtures of optical isomers, including diastereomers and enantiomers, or stereoisomers, they may be separated using known methods, such as chiral resolution, chiral chromatography and the like.

In addition, it is recognized that one optical isomer, including diastereomer and enantiomer, or stereoisomer may have favorable properties over the other. Thus when disclosing and claiming the invention, when one racemic mixture is disclosed, it is clearly contemplated that both optical isomers, including diastereomers and enantiomers, or stereoisomers substantially free of the other are disclosed and claimed as well.

An "organosilicon reagent" is any silicon-containing reagent that is commonly utilized in silylation reactions, that is, reactions which substitute a hydrogen atom bound to a heteroatom (e.g., -OH, =NH, -SH, etc.) with a silyl group, usually a trialkylsilyl group, including reactions of a tautomer of a heteroatom system to form a silyl derivative (e.g., silyl emol ethers), thus forming a silicon-heteroatom bond. Many such compounds are known in the art, as described in the following articles: E. Plueddemann, "Silylating Agents", in: Kirk-Othmer, 3d ed., Vol. 20, "Encyclopedia of Chemical Technology" (1982); I. Fleming, "Organic Silicon Chemistry", in: Vol. 3, "Comprehensive Organic Chemistry" (D. Jones, editor, 1979); B. Cooper, "Silylation in Organic Synthesis", Proc. Biochem. 9 (1980); B. Cooper, "Silylation as a Protective Method in Organic Synthesis", Chem. Ind. 794 (1978); J. Rasmussen, "O-Silylated Enolates―Versatile Intermediates for Organic Synthesis" 91 Synthesis (1977). Representative organosilicon reagents useful in the present process include, but are not limited to, chlorotrimethylsilane, N,O-bis(trimethylsilyl)acetamide, N,O-bis(trimethylsilyl)trifluoroacetamide, 1,3-bis(trimethylsilyl)urea, 1,1,1,3,3,3-hexametbyldisilazane, N-methyl-N-trimethylsilyltrifluoroacetamide, 1-trimethylsilylimidazole, trimethylsilyl trifluoromethanesulfonate, tert-butyldimethylchlorosilane, 1-(tert-butyldimethylsilyl)imidazole, ethyl(trimethylsilyl)acetate, N-tert-butyldimethyl-N-methyltrifluoroacetamide, tertbutyldimethylsilyl trifluoromethanesulfonate, tert-butyldiphenylchlorosilane, tert-butylmethoxyphenylbromosilane, dimethylphenylchlorosilane, triethylchlorosilane, triethylsilyl trifluoromethane-sulfonate, and triphenylchlorosilane. Of the various organosilicon reagents useful herein, N,O-bis(trimethylsilyl)acetamide, N,O-bis(trimethylsilyl)trifluoroacetamide, N-methyl-N-trimethylsilyltrifluoroacetamide and tert-butyldiphenylchlorosilane are particularly preferred. More than one organosilicon reagent may be used in the present process.

A "pharmaceutically-acceptable salt" is a cationic salt formed at any acidic (e.g., carboxyl) group, or an anionic salt formed at any basic (e.g., amino, alkylamino, dialkylamino, morphylino, and the like) group on the compound of the invention. Since many of the compounds of the invention are zwitterionic, either salt is possible and acceptable. Many such salts are known in the art. Preferred cationic salts include the alkali metal salts (such as sodium and potassium), alkaline earth metal salts (such as magnesium and calcium) and organic salts, such as ammonio. Preferred anionic salts include halides, sulfonates, carboxylates, phosphates, and the like. Clearly contemplated in such salts are addition salts that may provide an optical center, where once there was none. For example, a chiral tartrate salt may be prepared from the compounds of the invention, and this definition includes such chiral salts. Salts contemplated are nontoxic in the amounts administered to the patient-animal, mammal or human.

The compounds made by the present process may be sufficiently basic to form acid-addition salts. The compounds are useful both in the free base form and the form of acid-addition salts, and both forms are within the purview of the invention. The acid-addition salts are in some cases a more convenient form for use. In practice, the use of the salt form inherently amounts to the use of the base form of the active. Acids used to prepare acid-addition salts include preferably those which produce, when combined with the free base, medicinally acceptable salts. These salts have anions that are relatively innocuous to the animal organism, such as a mammal, in medicinal doses of the salts so that the beneficial property inherent in the free base are not vitiated by any side effects ascribable to the acid's anions.

Examples of appropriate acid-addition salts include, but are not limited to hydrochloride, hydrobromide, hydroiodide, sulfate, hydrogensulfate, acetate, trifluoroacetate, nitrate, citrate, fumarate, formate, stearate, succinate, maleate, malonate, adipate, glutarate, lactate, propionate, butyrate, tartrate, methanesulfonate, trifluoromethanesulfonate, p-toluenesulfonate, dodecyl sulfate, cyclohexanesulfamate, and the like. However, other appropriate medicinally acceptable salts within the scope of the invention are those derived from other mineral acids and organic acids. The acid-addition salts of the basic compounds are prepared by several methods. For example, the free base can be dissolved in an aqueous alcohol solution containing the appropriate acid and the salt is isolated by evaporation of the solution. Alternatively, they may be prepared by reacting the free base with an acid in an organic solvent so that the salt separates directly. Where separation of the salt is difficult, it can be precipitated with a second organic solvent, or can be obtained by concentration of the solution.

Although medicinally acceptable salts of the basic compounds are preferred, all acid-addition salts are within the scope of the present invention. All acid-addition salts are useful as sources of the free base form, even if the particular salt per se is desired only as an intermediate product. For example, when the salt is formed only for purposes of purification or identification, or when it is used as an intermediate in preparing a medicinally acceptable salt by ion exchange procedures, these salts are clearly contemplated to be a part of this invention.

Such salts are well understood by the skilled artisan, and the skilled artisan is able to prepare any number of salts given the knowledge in the art. Furthermore, it is recognized that the skilled artisan may prefer one salt over another for reasons of solubility, stability, formulation ease and the like. Determination and optimization of such salts is within the purview of the skilled artisan's practice.

As used herein, a "quinolone derivative" includes prodrugs of a quinolone, or an active drug made from a quinolone. Preferably, such derivatives include lactams (e.g., cephems, carbacephems, penems, monolactams, etc.) covalently linked to the quinolone optionally via a spacer. Such derivatives and methods to make and use them are apparent to the skilled artisan, given the teachings of this specification.

"Spirocycle" is an alkyl or heteroalkyl diradical substituent of alkyl or heteroalkyl wherein said diradical substituent is attached geminally and wherein said diradical substituent forms a ring, said ring containing 4 to 8 member atoms (carbon or heteroatom), preferably 5 or 6 member atoms.

A "solvate" is a complex formed by the combination of a solute (e.g., a quinolone) and a solvent (e.g., water). See J. Honig et al., The Van Nostrand Chemist's Dictionary, p. 650 (1953). Pharmaceutically-acceptable solvents used according to this invention include those that do not interfere with the biological activity of the quinolone (e.g., water, ethanol, acetic acid, N,N-dimethylformamide and others known or readily determined by the skilled artisan).

While alkyl, heteroalkyl, cycloalkyl, and heterocycloalkyl groups may be substituted with hydroxy, amino, and amido groups as stated above, the following are not envisioned in the invention:
1. Enols (OH attached to a carbon bearing a double bond).
2. Amino groups attached to a carbon bearing a double bond (except for vinylogous amides).
3. More than one hydroxy, amino, or amido attached to a single carbon (except where two nitrogen atoms are attached to a single carbon atom and all three atoms are member atoms within a heterocycloalkyl ring).
4. Hydroxy, amino, or amido attached to a carbon that also has a heteroatom attached to it.
5. Hydroxy, amino, or amido attached to a carbon that also has a halogen attached to it.

The illustration of the use of specific protected forms and other derivatives of the Formula 1 compounds in the present process are not intended to be limiting. The application of other useful protecting groups, salt forms, etc. is within the ability of the skilled artisan.

### II. Preferred Compounds Made By the Subject Process:

The subject invention relates to a process comprising the following process step: 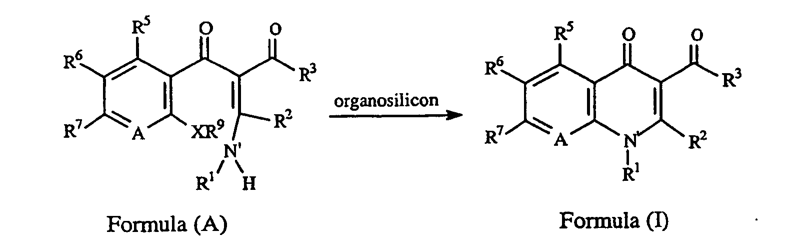 where R¹, R², R³, R⁵, R⁶, R⁷, A, X and R⁹ are as defined in the Summary of the Invention section above.

With reference to Formula (I) and Formula (A), the description above indicates that in one embodiment (defined in sub-part (A)), the nucleus of the final compounds of Formula (I) will include two fused rings as depicted. Alternatively, the nucleus of the Formula (I) compounds will, upon cyclization via the present process, include three fused rings, as defined in sub-parts (B) and (C). These alternative embodiments are depicted as Formula (B) and Formula (C), respectively, below.

In the above structures, R⁵ is selected from hydrogen, alkyl, aryl, cyano, a heterocyclic ring, amino, alkylamino, arylamino, alkylacyl, arylacyl, and aryl esters and amides of carboxy. Preferred R⁵ is selected from hydrogen, C₁ to about C₄ alkyl, phenyl, amino and C₁ to about C₄ mono- or dialkylamino. More preferred R⁵ is selected form hydrogen, amino, methyl, ethyl, methylamino and dimethylamino. Alkyl and aryl portions of the R⁵ moieties are preferably unsubstituted or substituted with fluoro.

In the above structures, R⁶ is selected from hydrogen, halo, alkyl, aryl, a heterocyclic ring, amino, alkylamino, arylamino, nitro, cyano, alkoxy, aryloxy, esters of hydroxy, alkylthio, arylthio, esters of thio, alkylsulfonyl, arylsulfonyl, alkylphosphonyl, arylphosphonyl, alkylacyl, arylacyl, and alkyl and aryl esters and amides of carboxy. Preferred R⁶ is selected from hydrogen, halo, nitro, C₁ to about C₄ alkylamino, C₁ to about C₄ alkoxy, and C₁ to about C₄ esters of hydroxy. More preferred R⁶ is selected from hydrogen, fluoro, chloro, methyl, methylamino, dimethylamino, nitro, methoxy and acetoxy. Alkyl and aryl portions of the R⁶ moieties are preferably unsubstituted or substituted with fluoro.

In the above structures, R⁷ is selected from hydrogen, alkyl, aryl, a heterocyclic ring, amino, alkylamino, arylamino, halo, nitro, cyano, alkoxy, aryloxy, esters of hydroxy, alkylthio, arylthio, esters of thio, alkylsulfonyl, arylsulfonyl, alkylphosphonyl, arylphosphonyl, alkylacyl, arylacyl, and alkyl and aryl esters and amides of carboxy. Preferred R⁷ is selected from hydrogen, halo, nitro, C₁ to about C₄ alkyl, unsubstituted amino, C₁ to about C₄ mono- or di-alkylamino, phenyl, naphthyl, a heterocyclic ring having one ring with 5 or 6 ring atoms or two fused rings with 8-10 ring atoms, C₁ to about C₄ alkylthio, phenylthio, phenoxy and C₁ to about C₄ esters of hydroxy. More preferred R⁷ is selected from hydrogen, fluoro, chloro, bromo, nitro, unsubstituted amino, methylamino, dimethylamino and trifluoroacetoxy. Alkyl and aryl portions of the R⁷ moieties are preferably unsubstituted or substituted with one or more fluoro atoms.

In the above structures, A is N or C-R⁸, preferably C-R⁸. R⁸ is selected from hydrogen, alkyl, aryl, halo, a heterocyclic ring, amino, alkylamino, arylamino, alkoxy, nitro, cyano, aryloxy, esters of hydroxy, alkylthio, arylthio, esters of thio, alkylsulfonyl, arylsulfonyl, alkylphosphonyl, arylphosphonyl, alkylacyl, arylacyl, and aryl esters and amides of carboxy. Preferred R⁸ is selected form hydrogen, halo, about C₁-C₄ alkyl, phenyl, about C₁-C₄ alkoxy, about C₁-C₄ alkylthio, and phenoxy. More preferred R⁸ is selected from hydrogen, fluoro, chloro, methoxy, di- and trifluoromethoxy, methylthio, di- and trifluoromethylthio, methyl, ethyl, cyclopropyl and phenyl.

In the above structures, R¹ is selected from a carbocyclic ring, a heterocyclic ring, lower alkyl, lower alkene, lower alkyne, and -CH(R¹⁰)(R¹¹) where R¹⁰ is selected from lower alkyl and phenyl and R¹¹ is -CH₂Y(O=)CR¹² where R¹² is selected from lower alkyl and phenyl and Y is selected from -NH-, -O- and -S-. Preferred R¹ is selected from C₁-C₄ alkyl, C₃-C₆ cycloalkyl and aryl. More preferred R¹ is selected from cyclopropyl, ethyl, 2,4-difluorophenyl, 2-methyl-1-acetoxypropane, 2-methyl-1-thioacetoxypropane. Akyl, cycloalkyl and aryl portions of the R¹ moieties are preferably unsubstituted or substituted with fluoro.

In the above structures, R² is selected from hydrogen, alkyl, aryl, a heterocyclic ring, allcylthio and arylthio. Preferred R² is selected from hydrogen, C₁-C₄ alkyl, C₁-C₄ alkylthio and phenyl. More preferred R² is hydrogen and methylthio.

In the above structures, R³ is selected from hydrogen, alkoxy, aryloxy, alkyl and aryl. Preferred R³ is selected from hydrogen, about C₁-C₄ alkoxy and phenoxy. Most preferred are hydrogen, methoxy and ethoxy.

In Formula (A), X is selected from -O- and -S- and R⁹ is selected from C₁-C₁₀ alkyl, aryl and heteroaryl. Preferred XR⁹ moieties are selected from alkoxy and alkylthio having from about 1 to about 10 carbon atoms, phenoxy and phenylthio. More preferred XR⁹ moieties are selected from methoxy, ethoxy, methylthio, ethylthio, phenoxy and phenylthio, all unsubstituted.

With respect to compounds defined in sub-part (A) of Formula (I), where the compounds include only two fused rings as the compound's nucleus, preferred compounds made according to the present process are those listed in Table I.

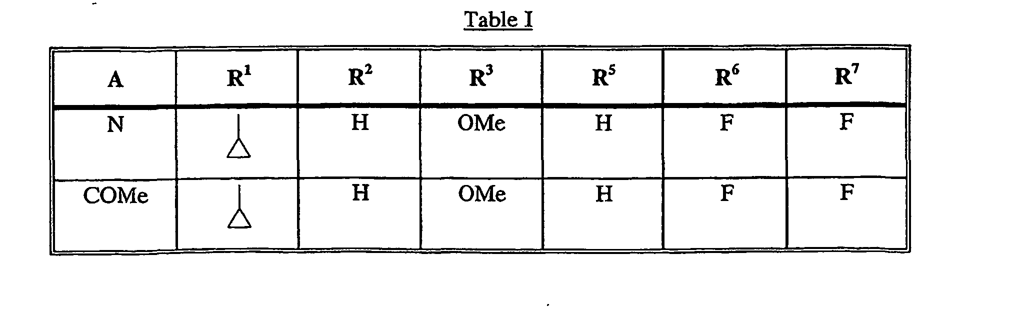

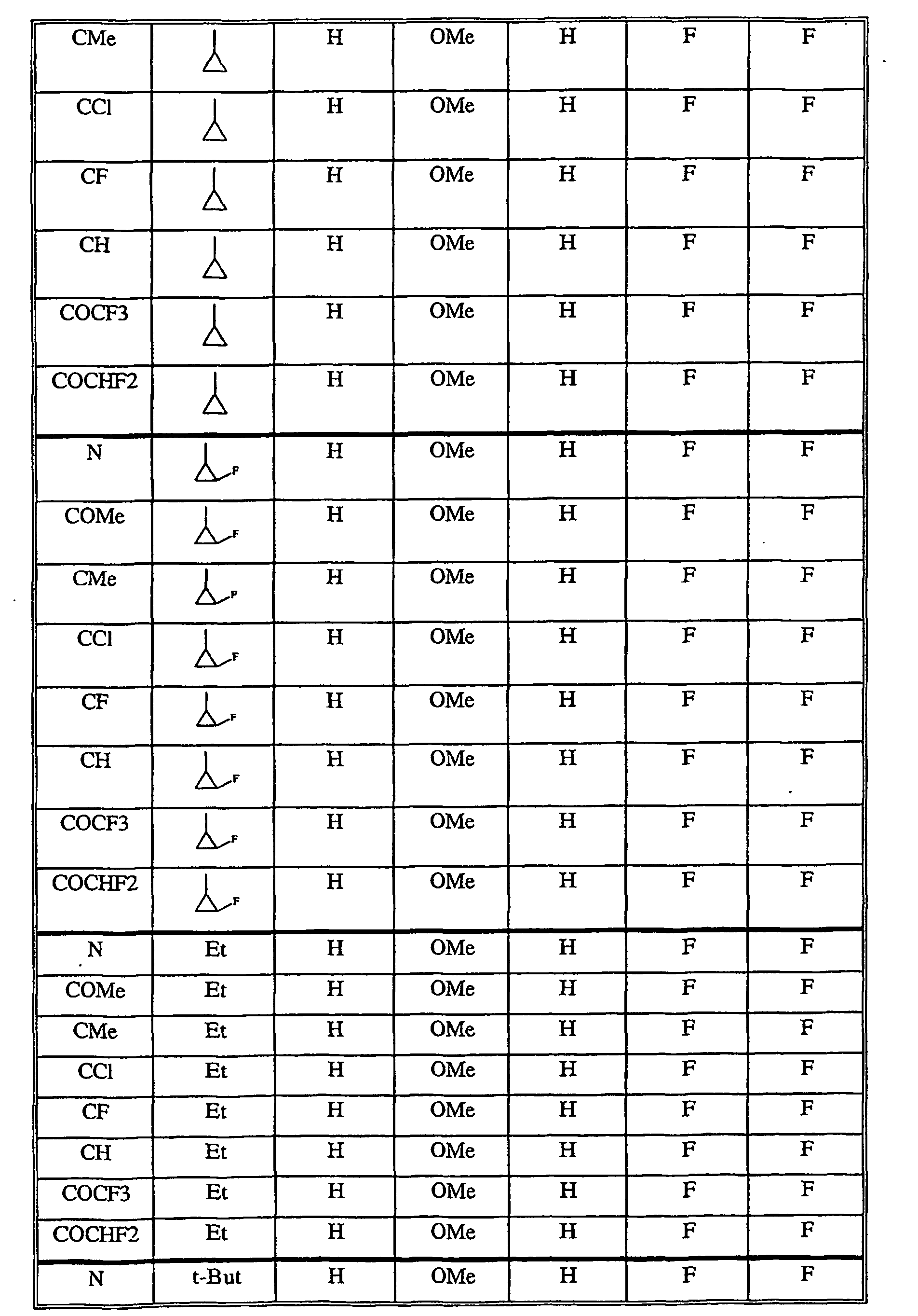

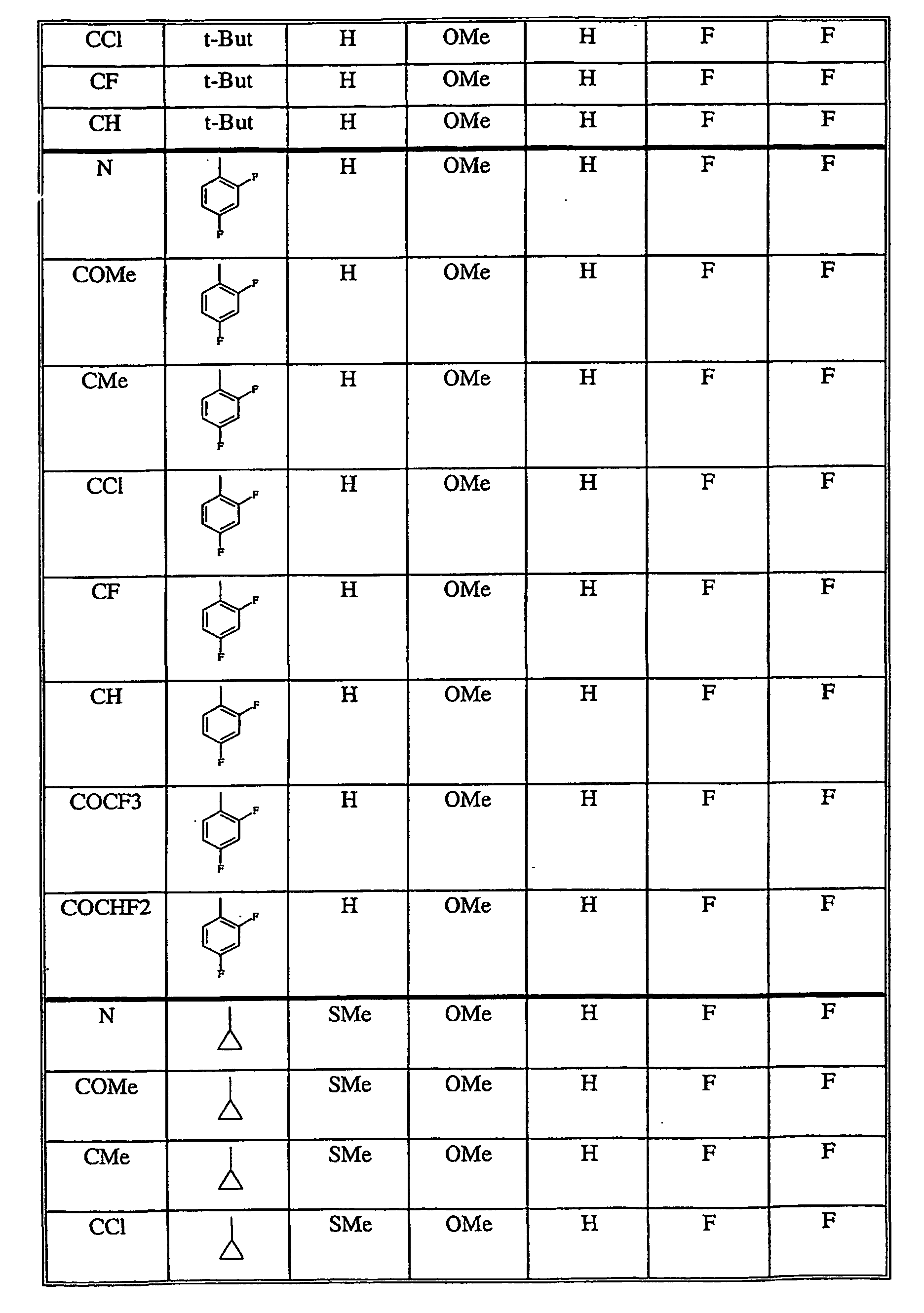

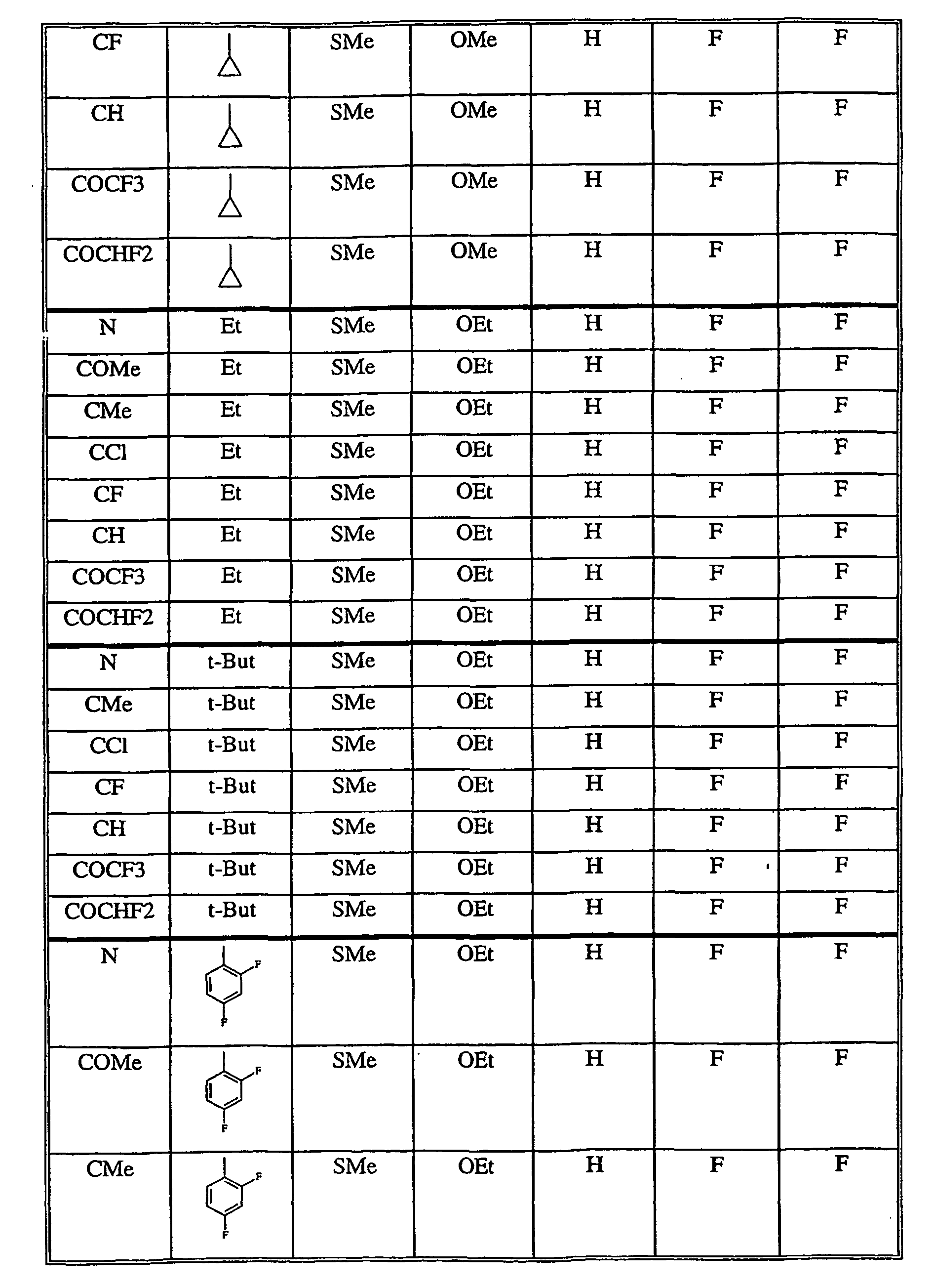

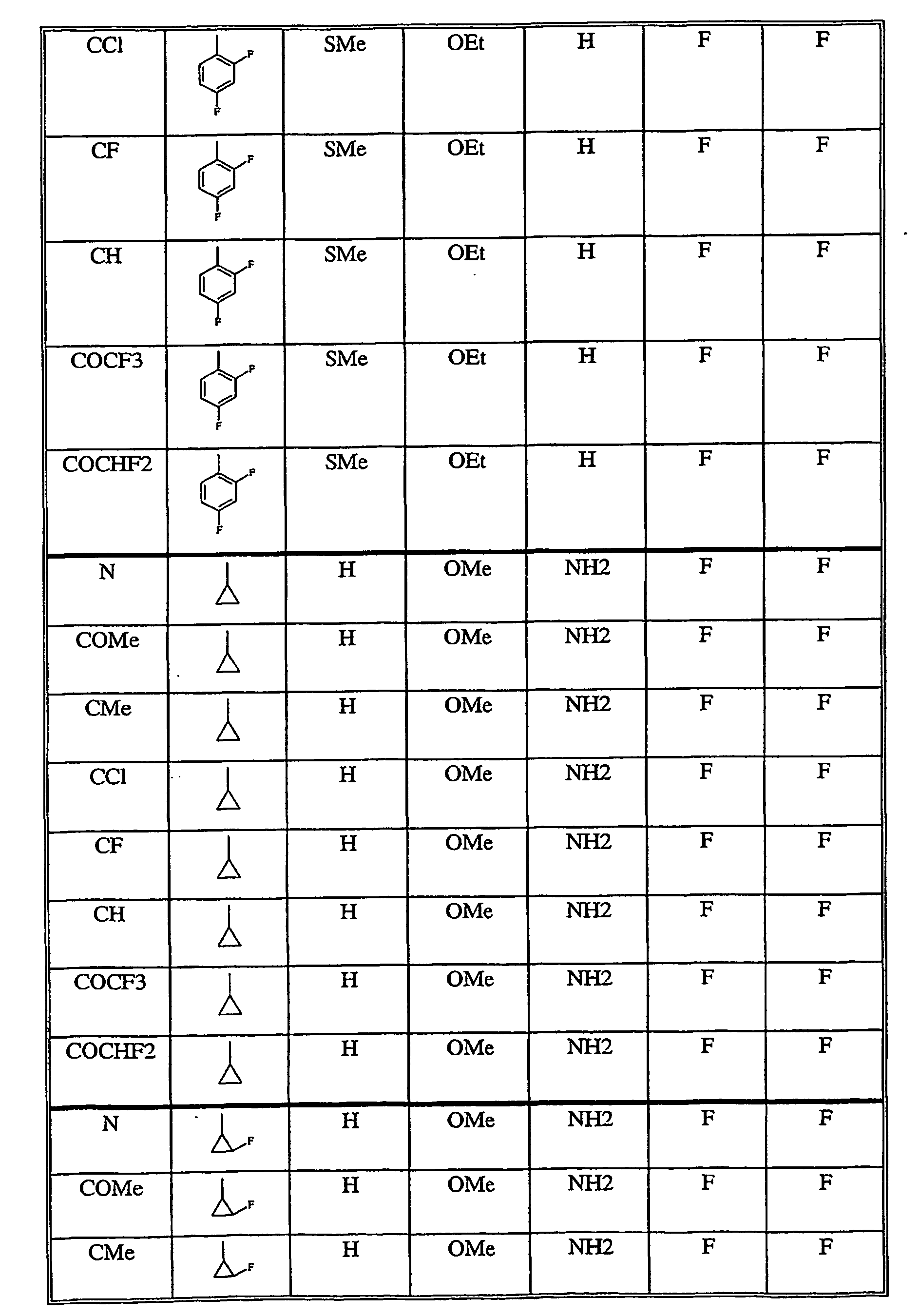

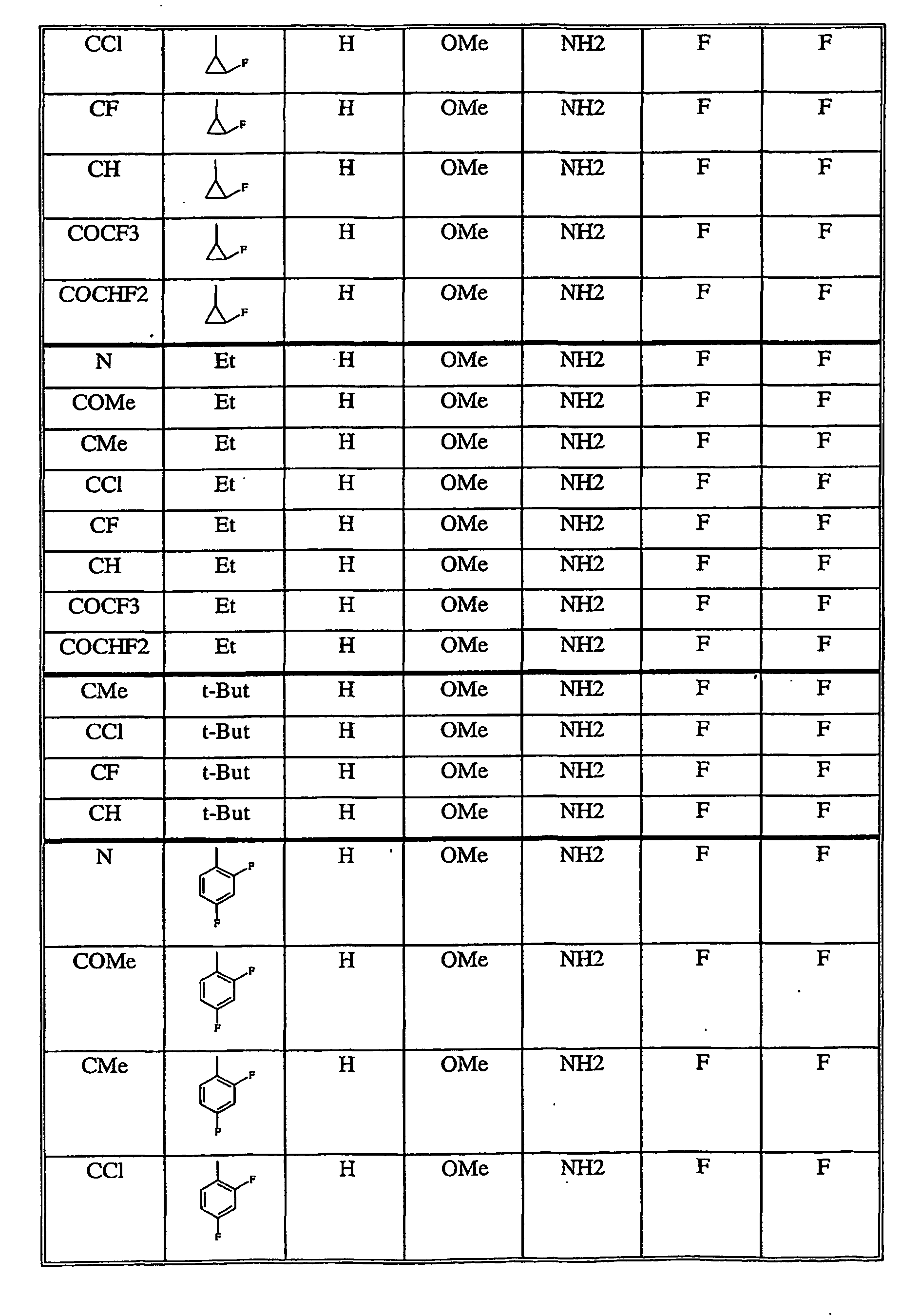

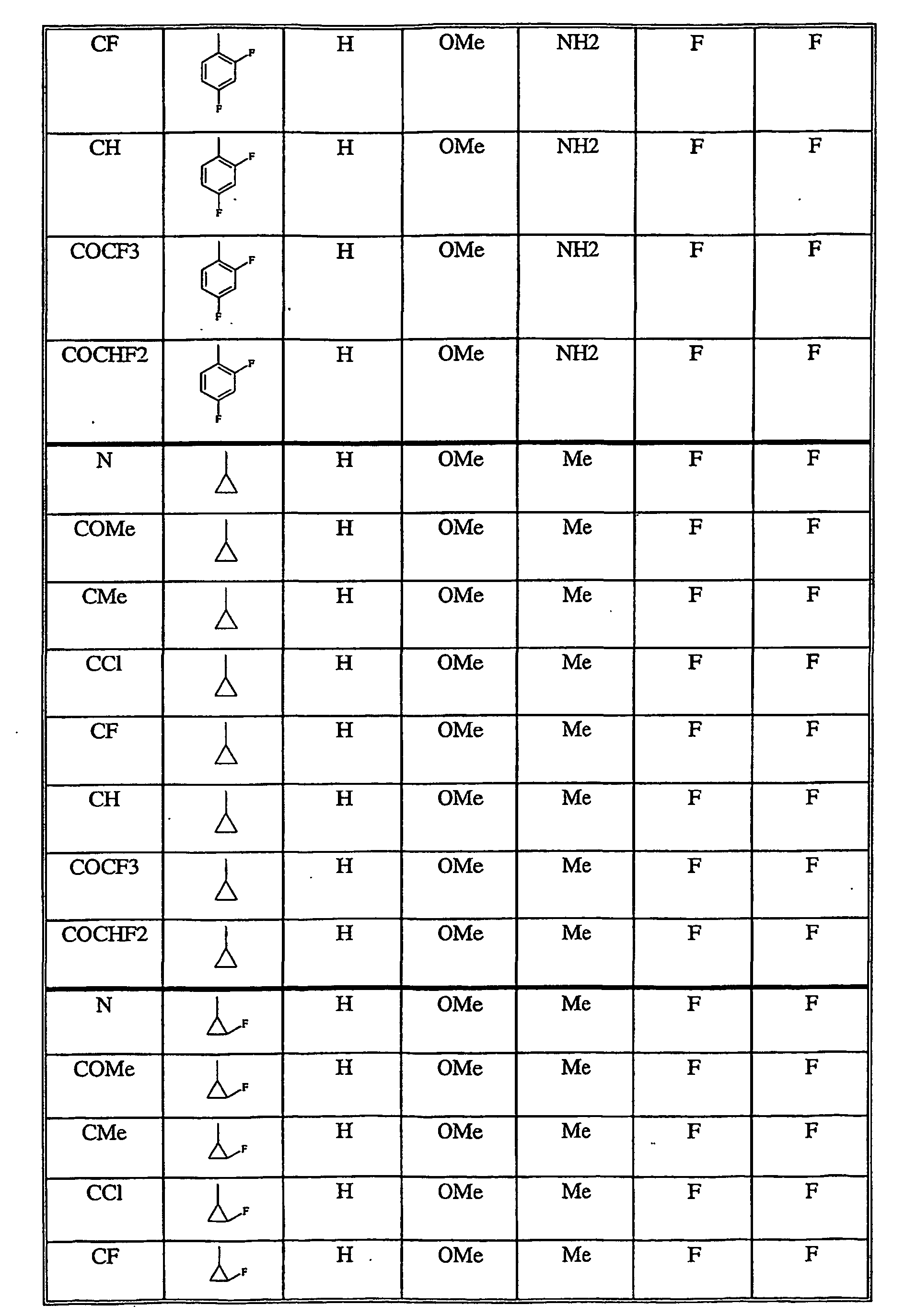

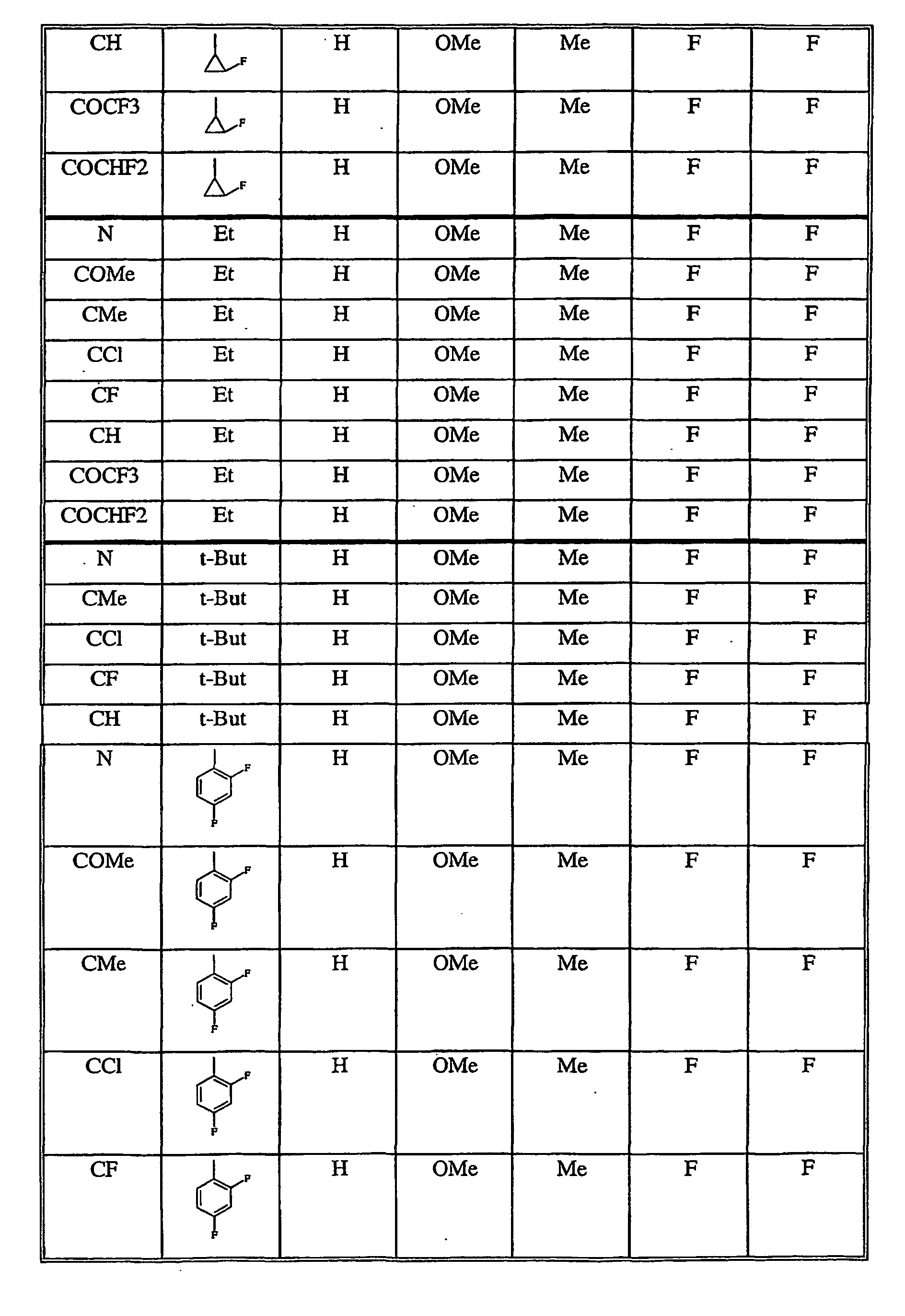

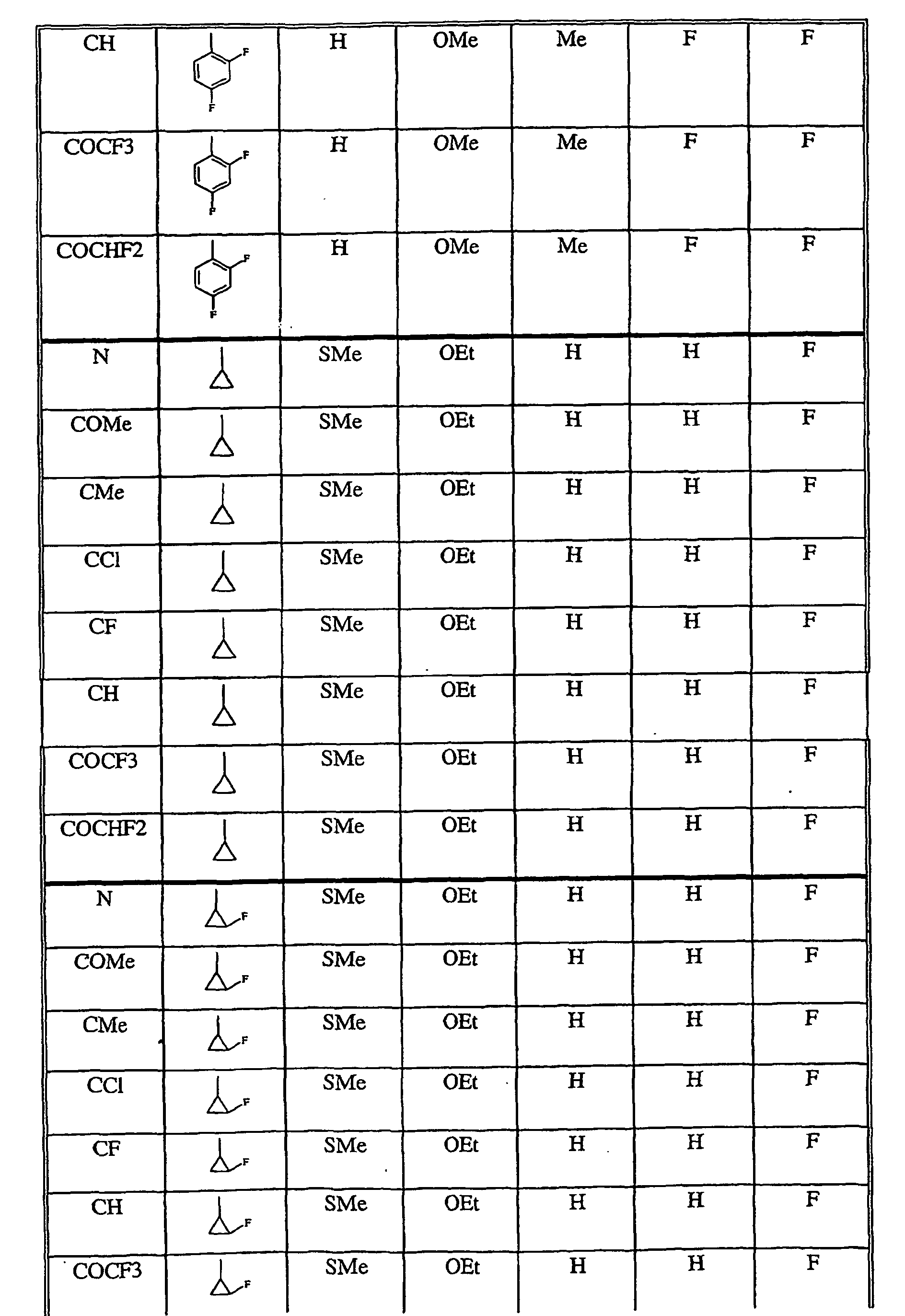

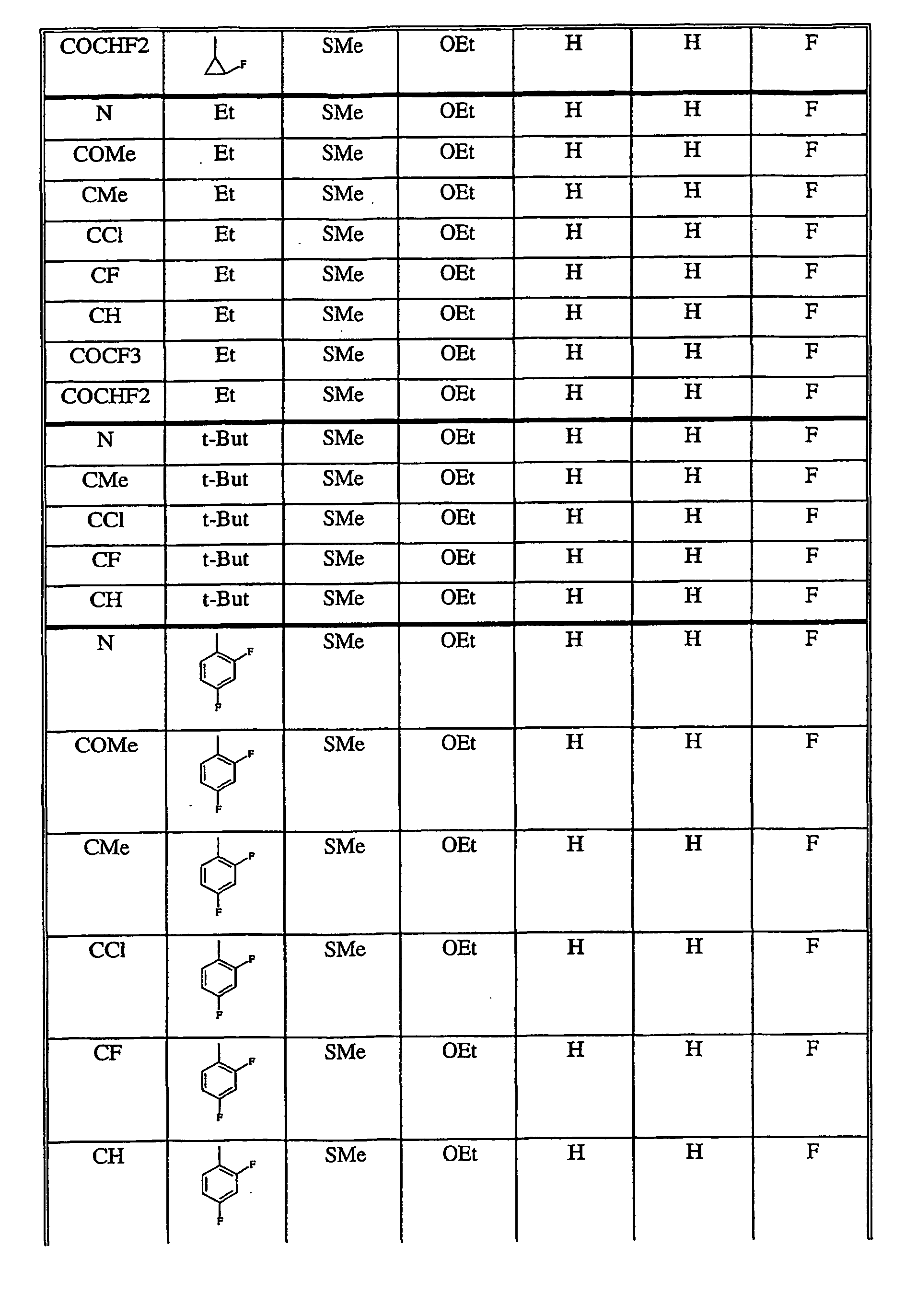

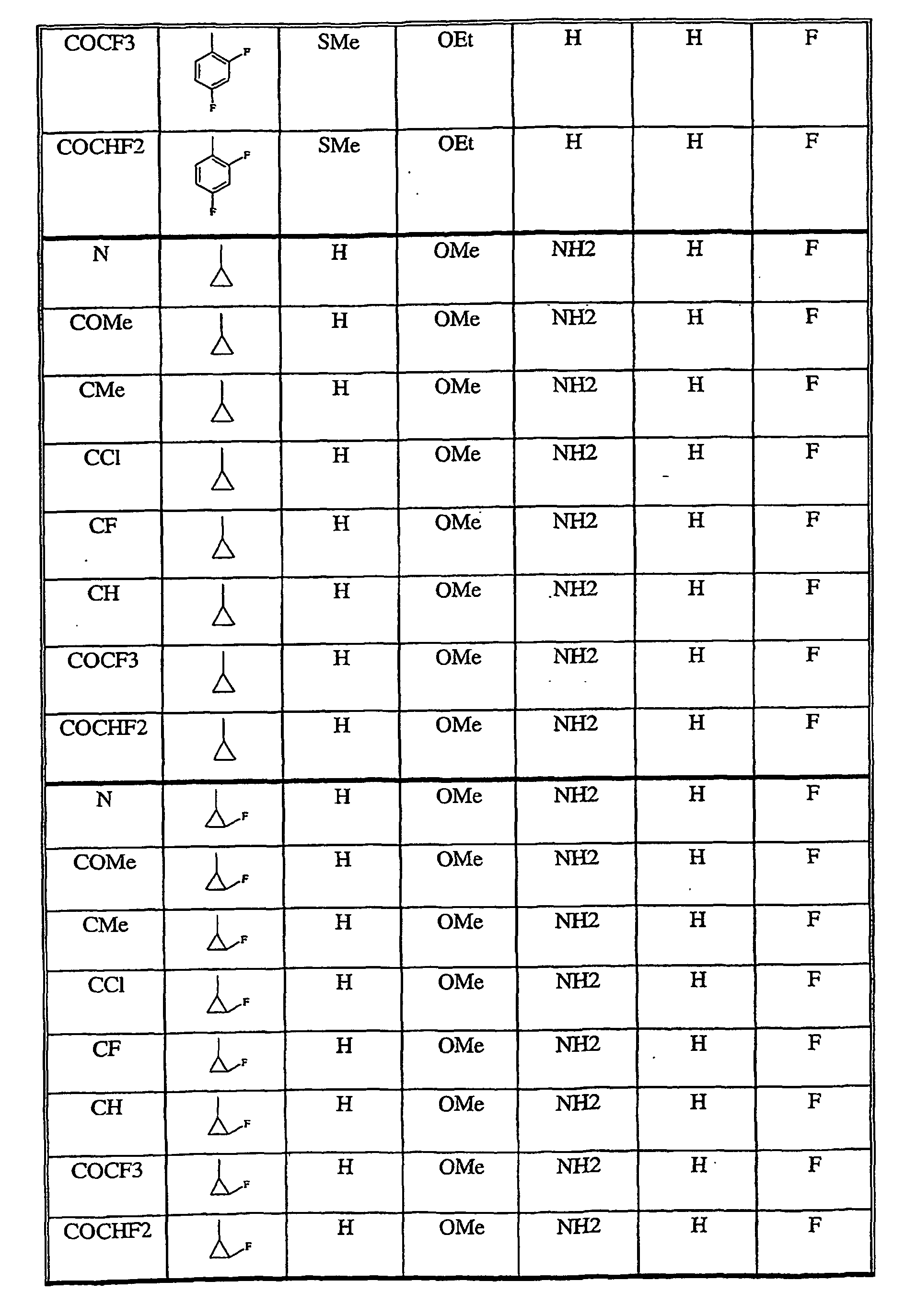

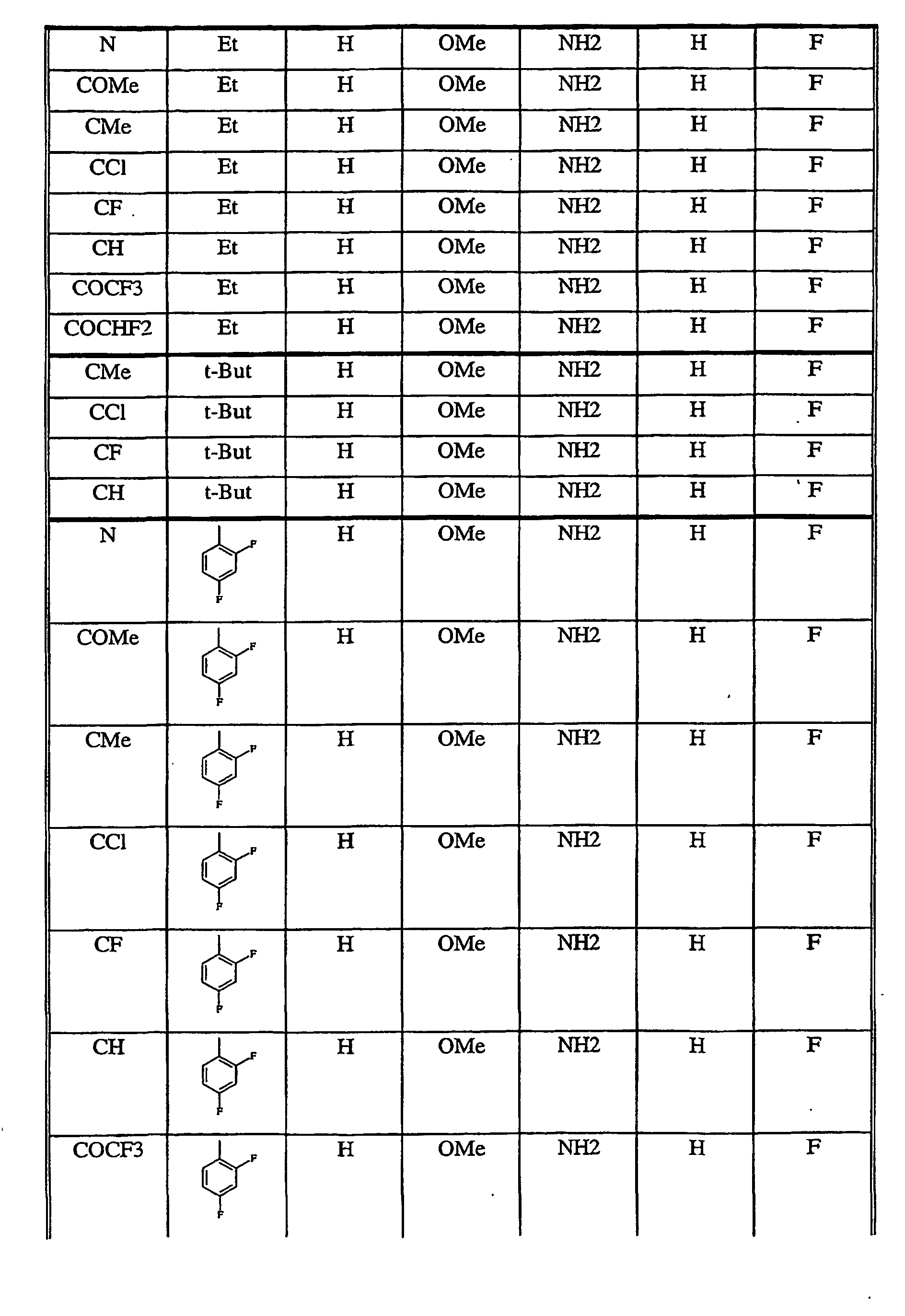

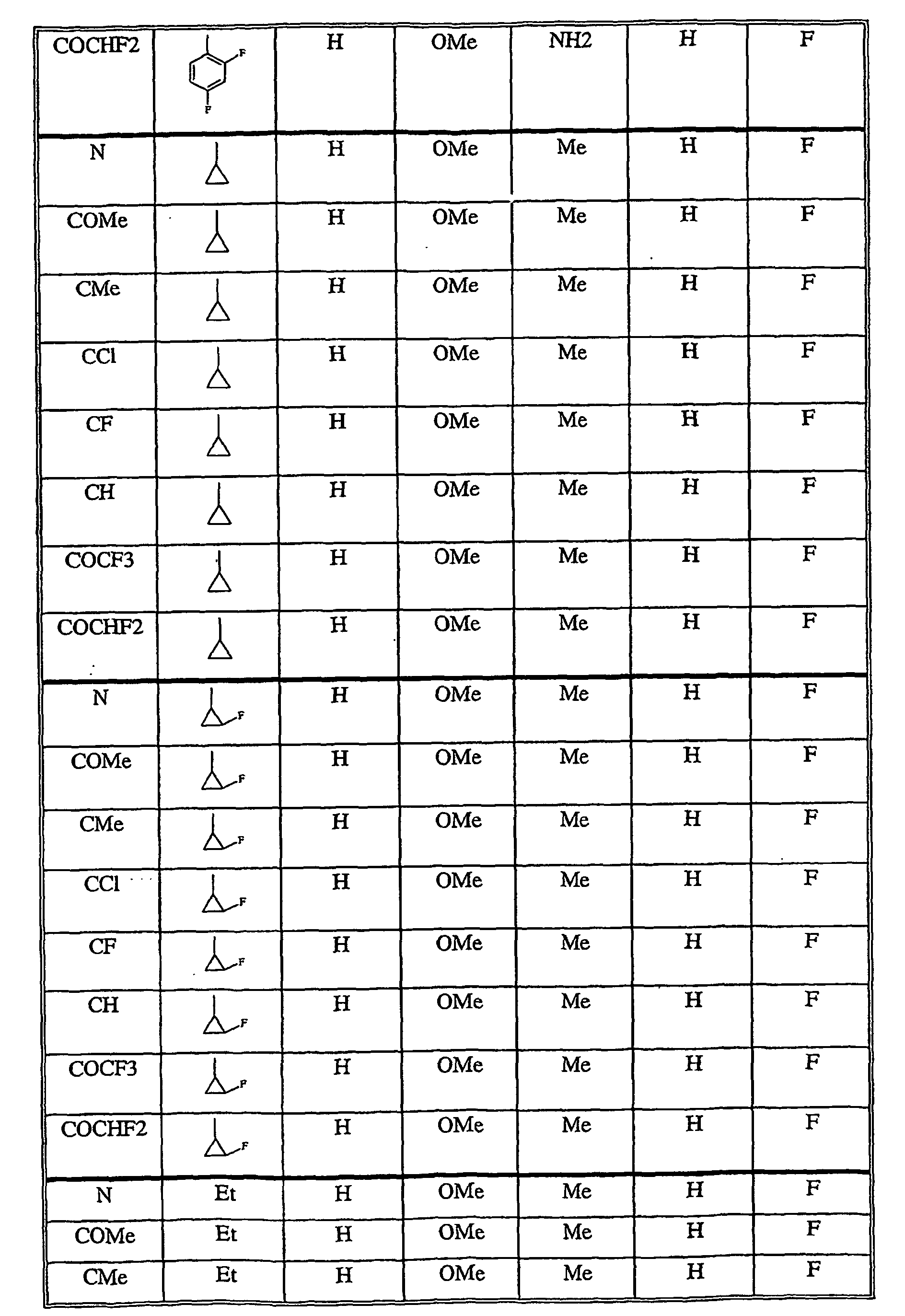

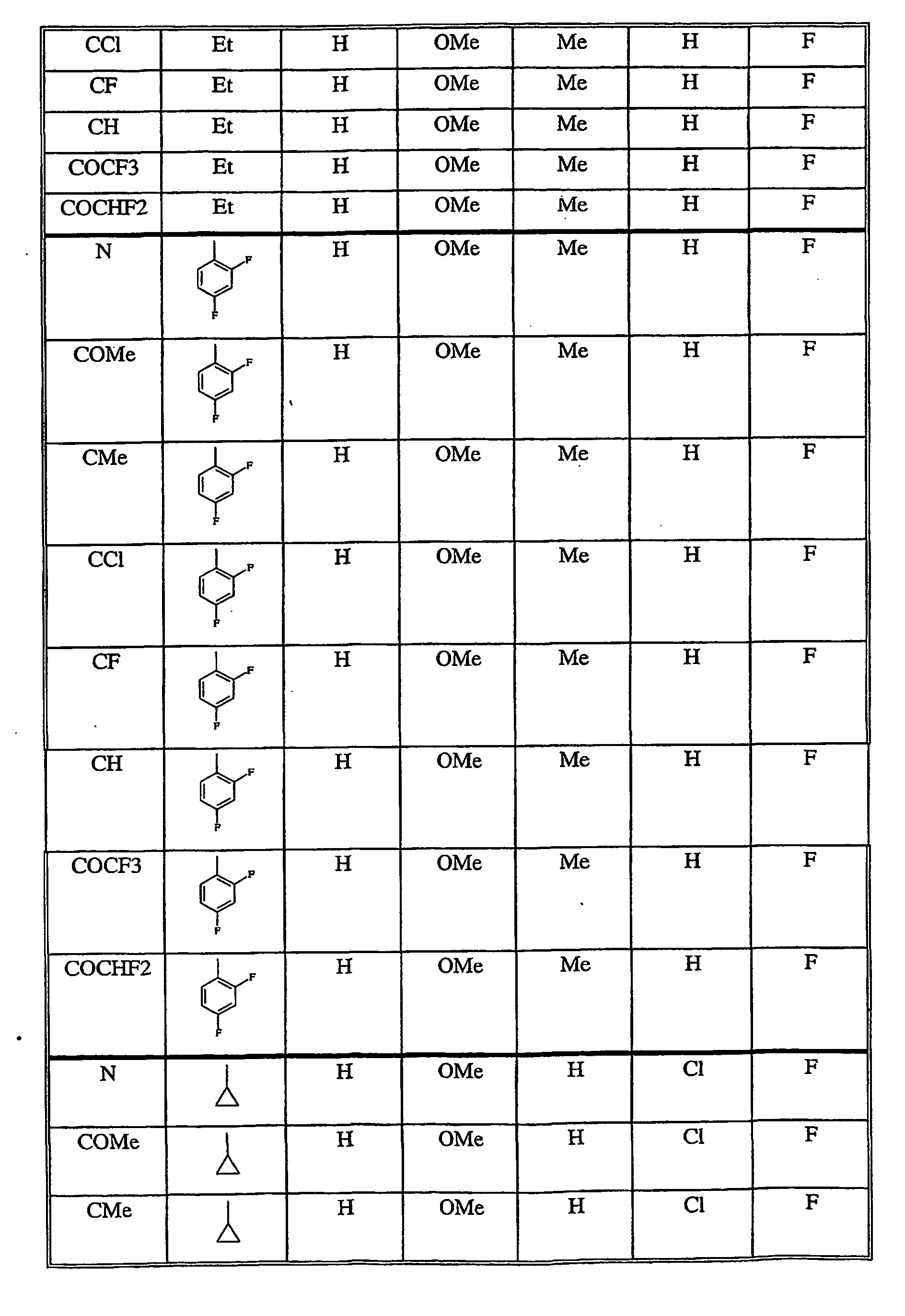

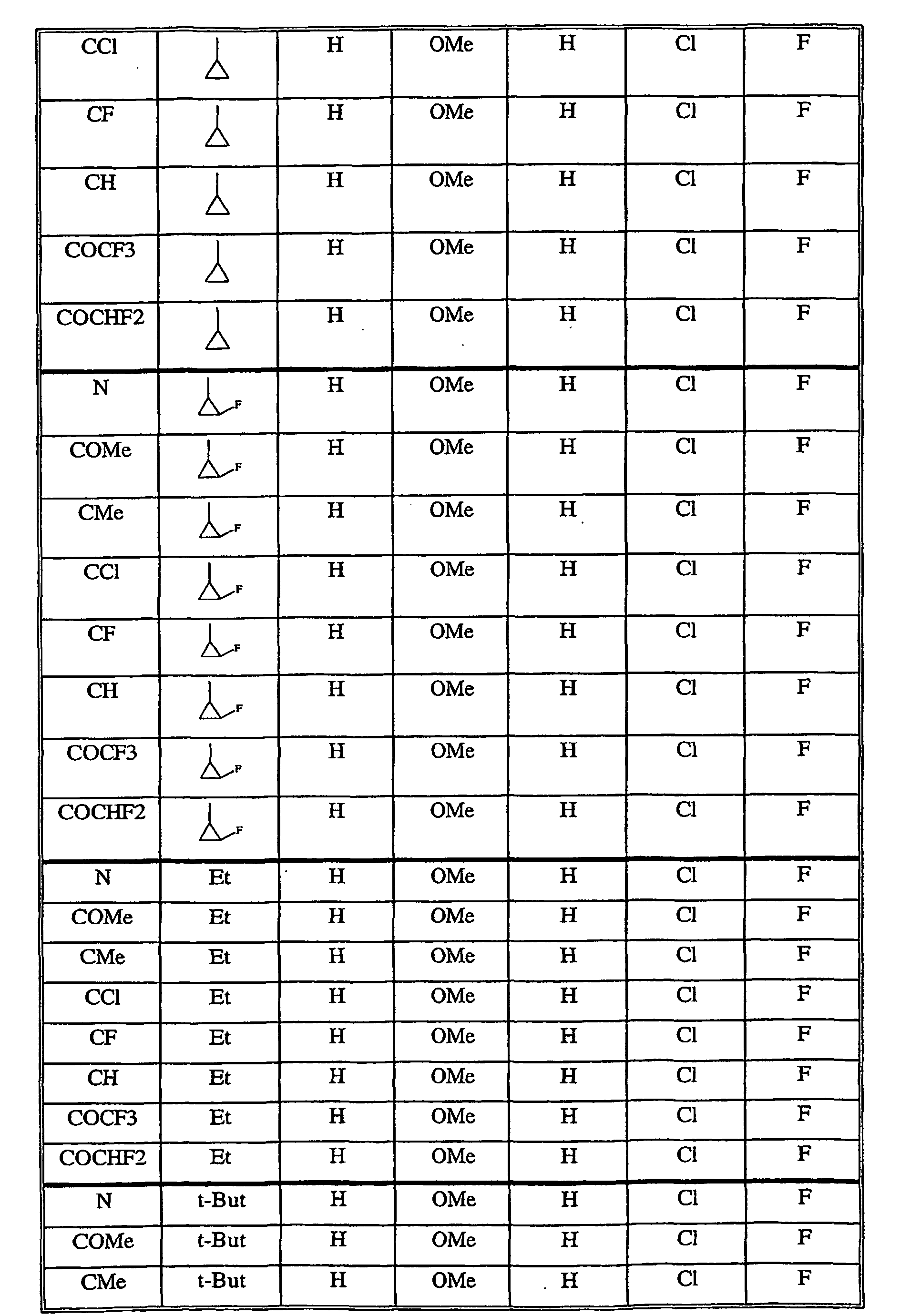

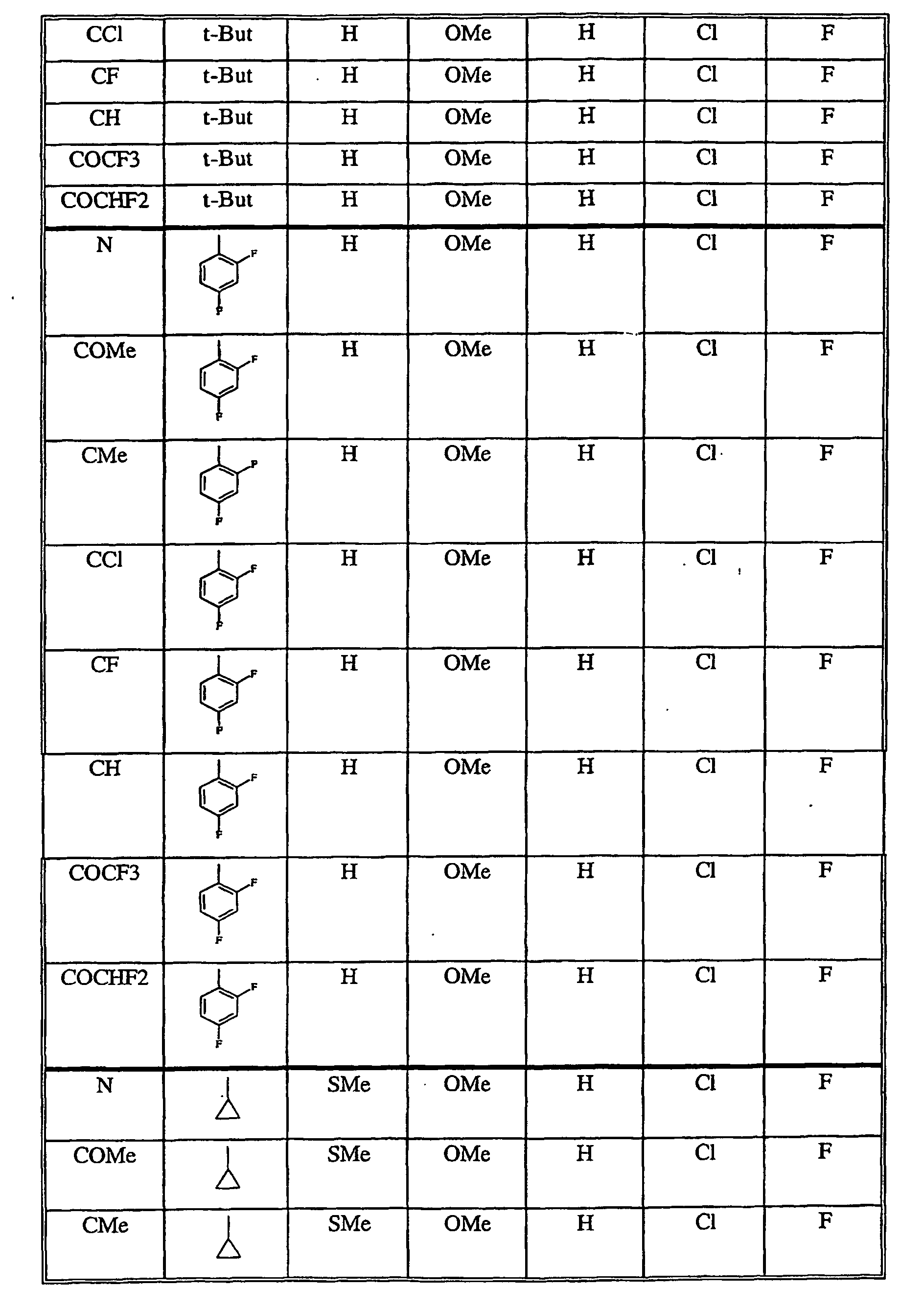

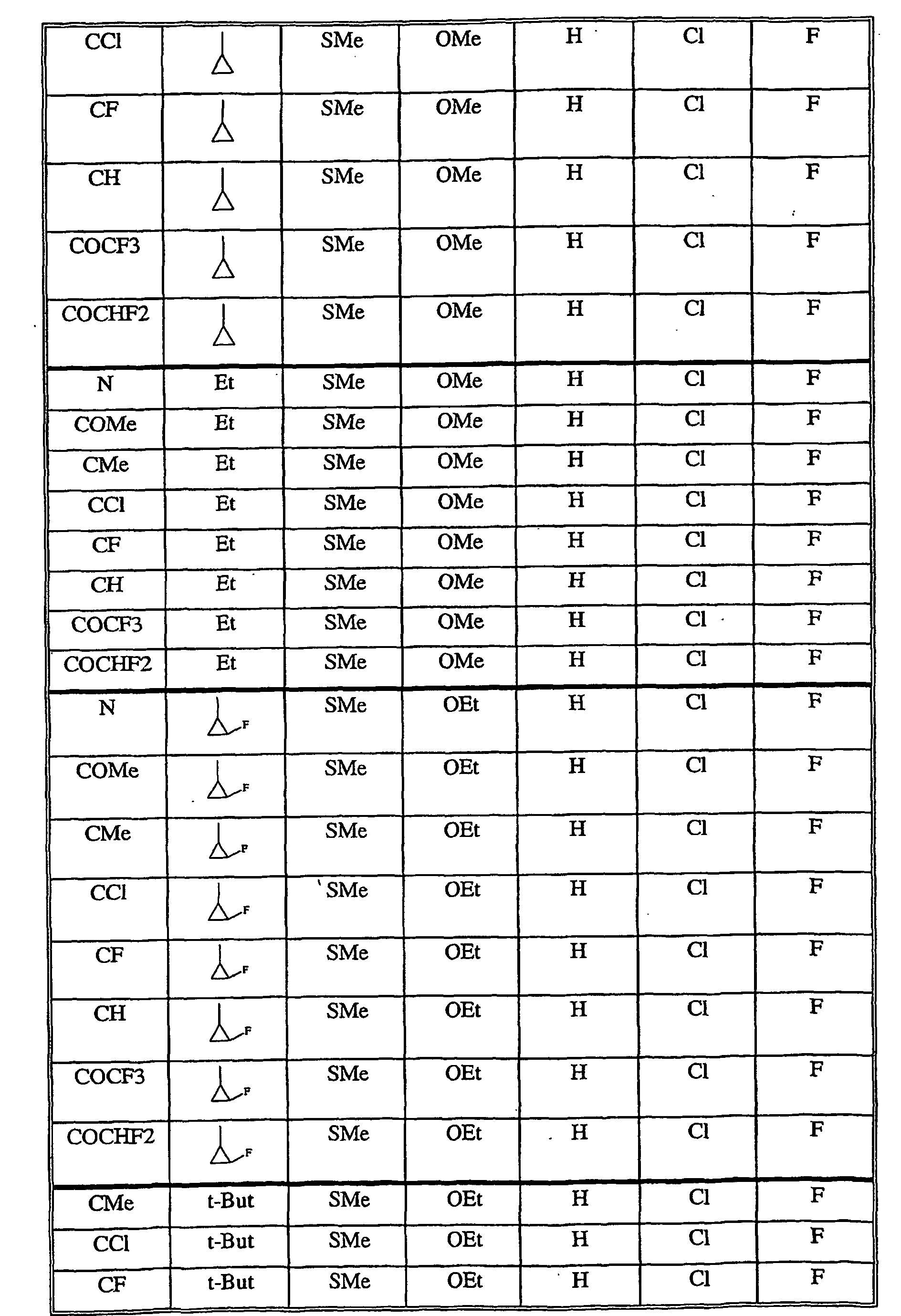

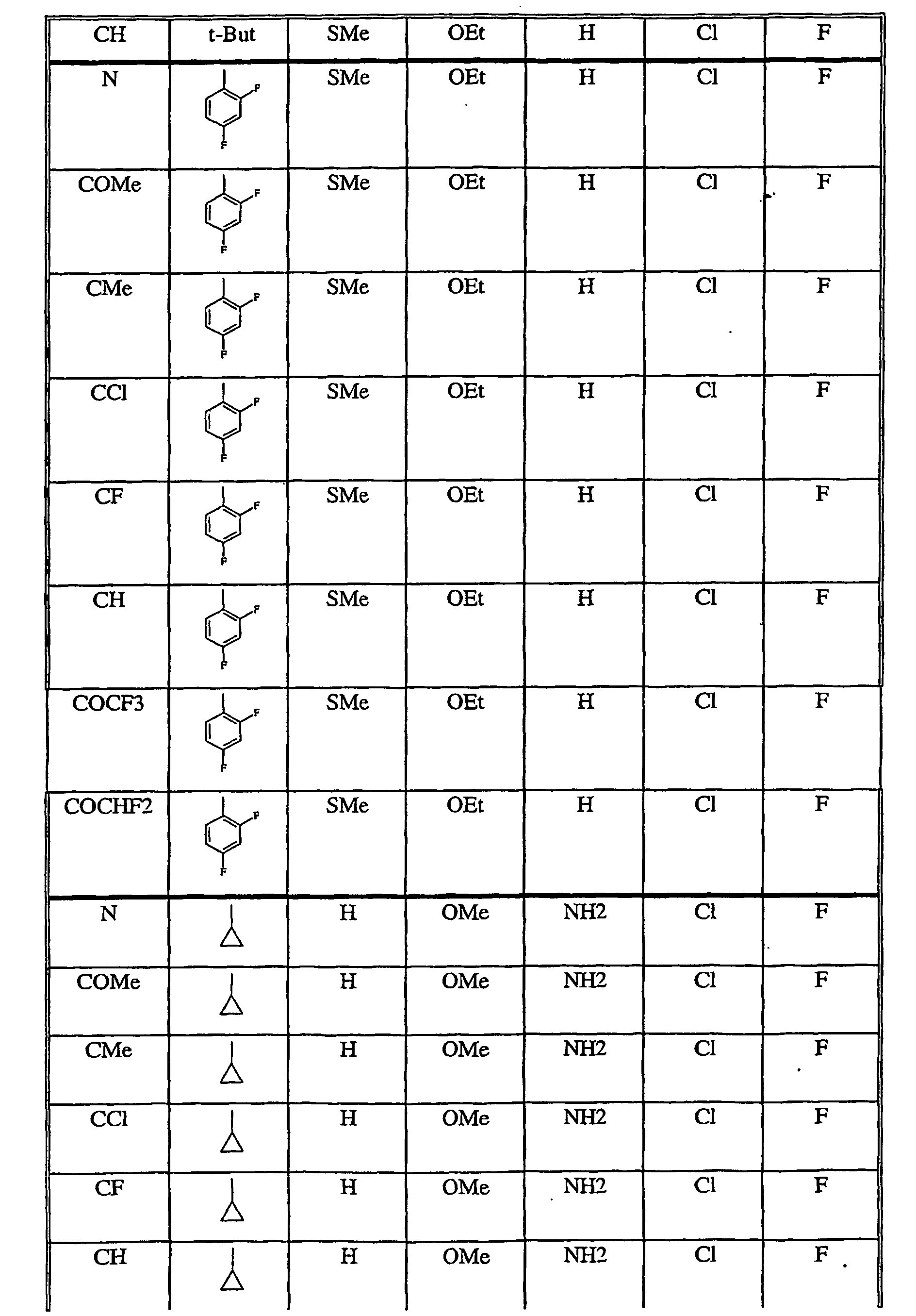

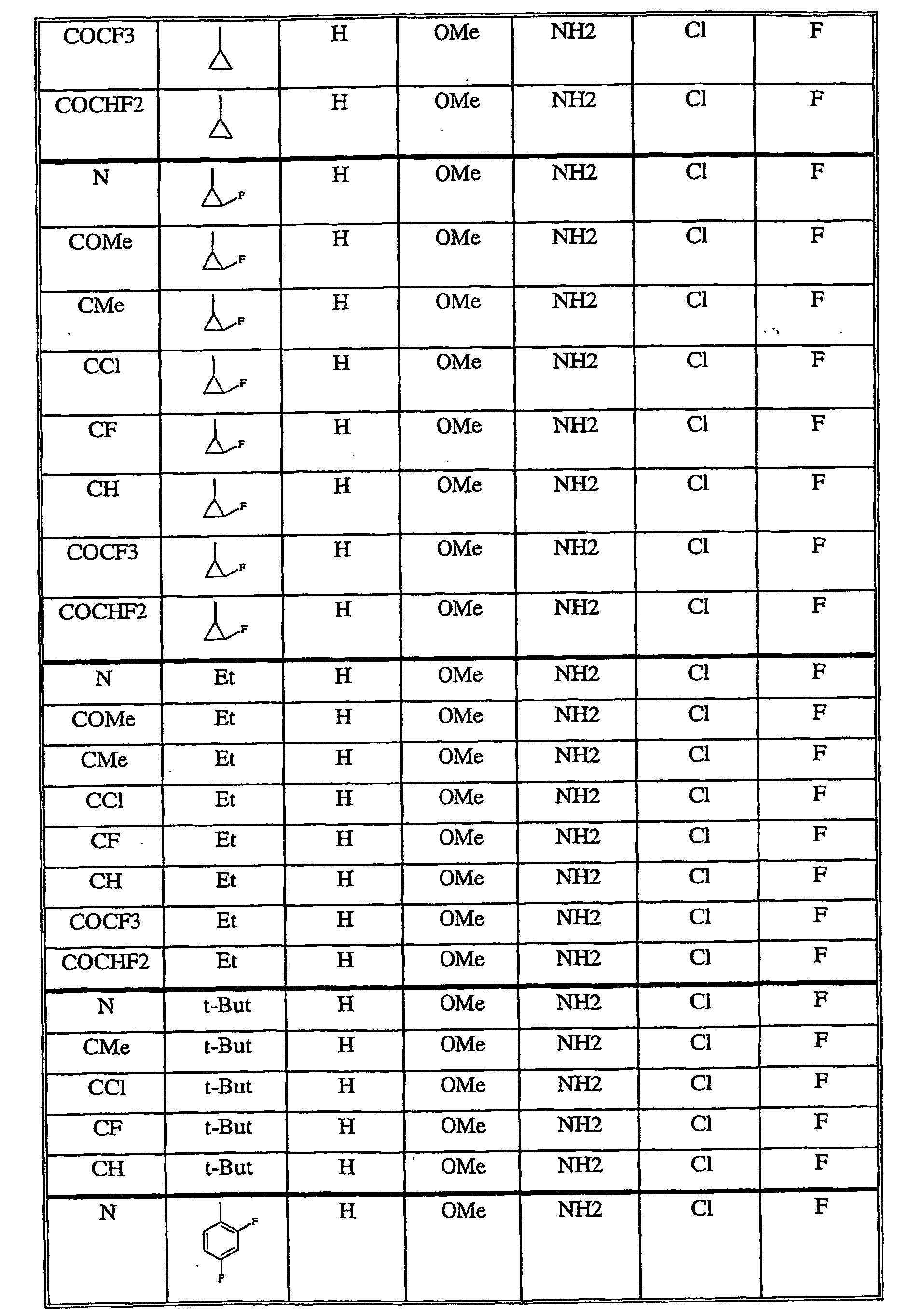

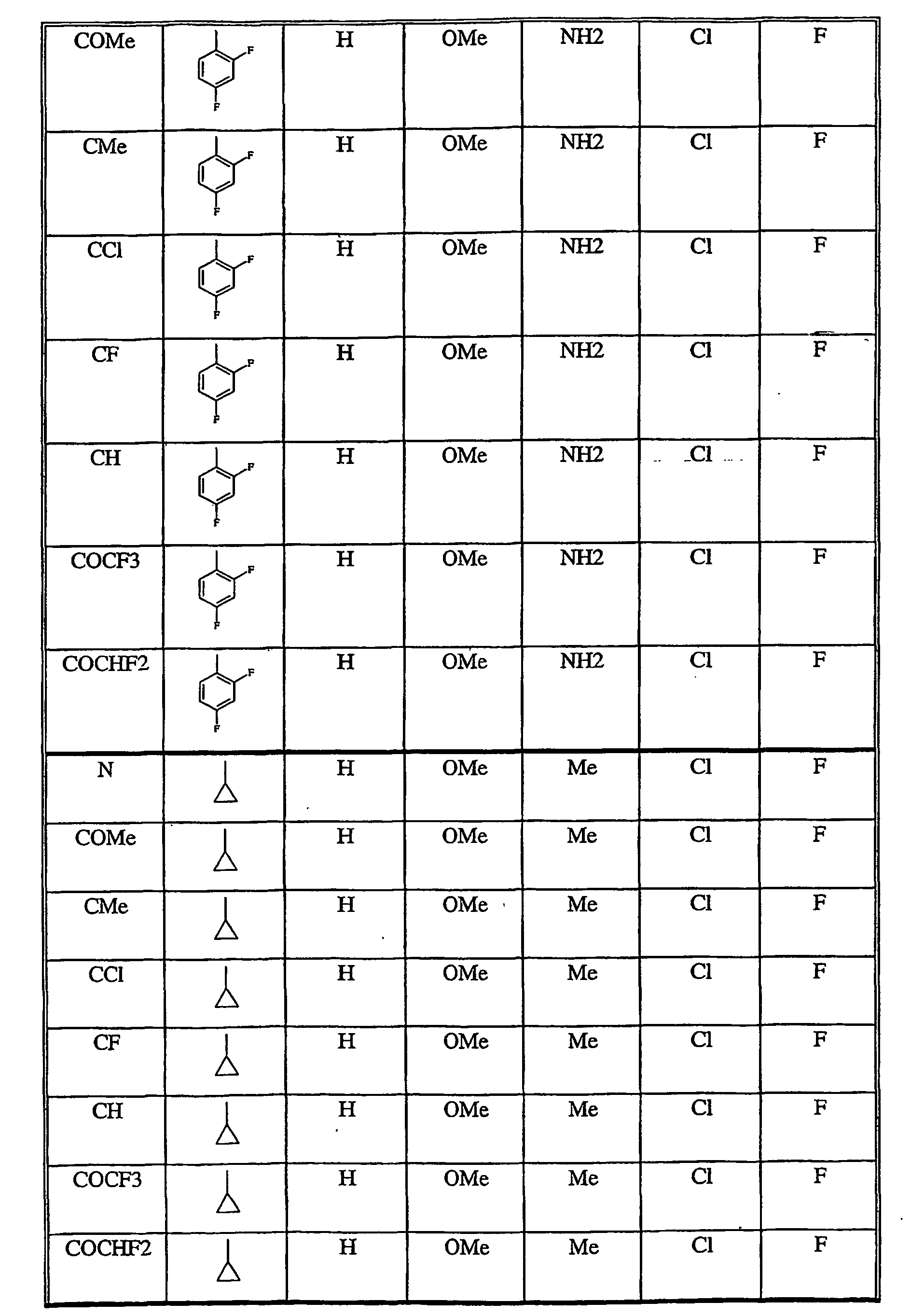

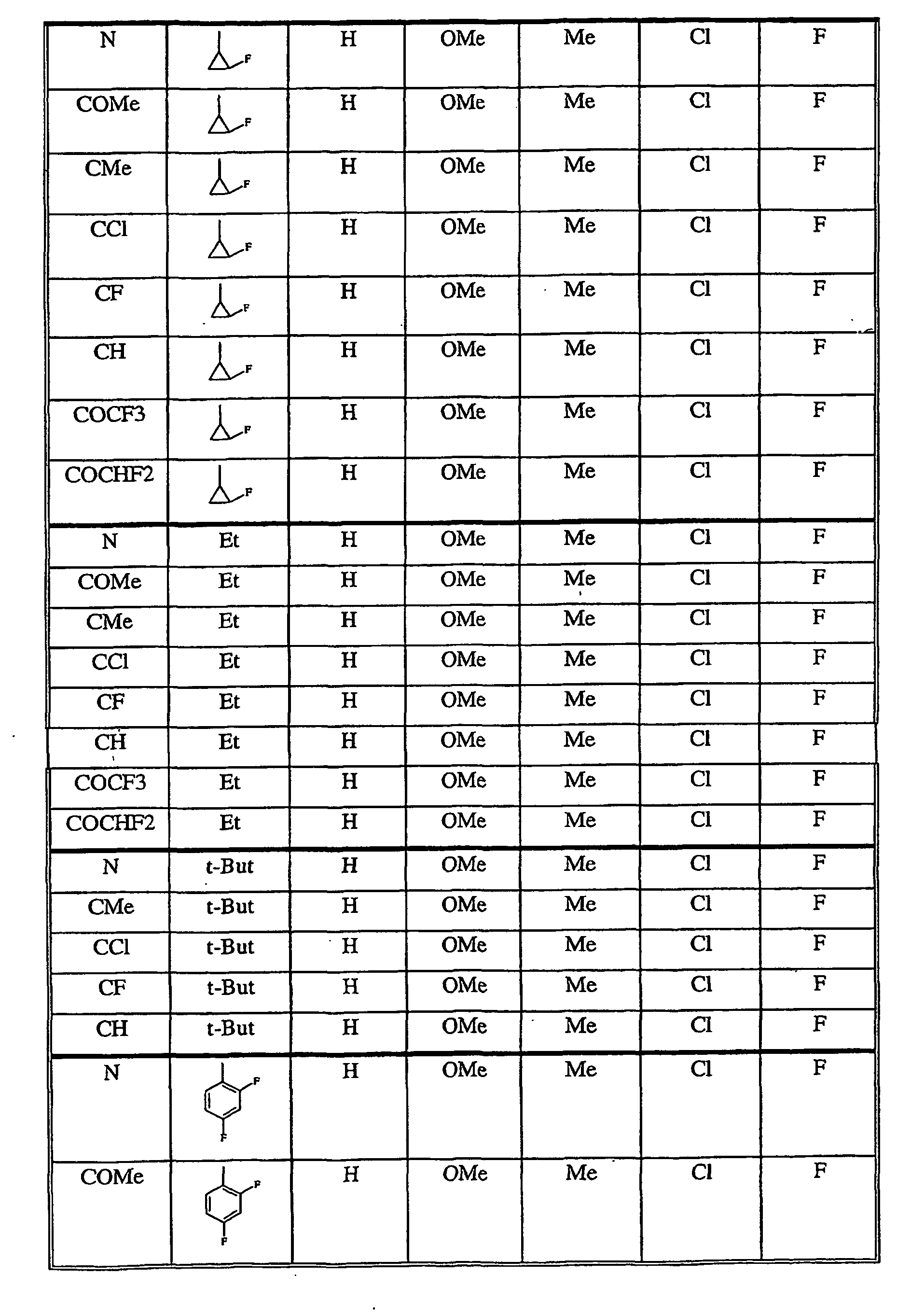

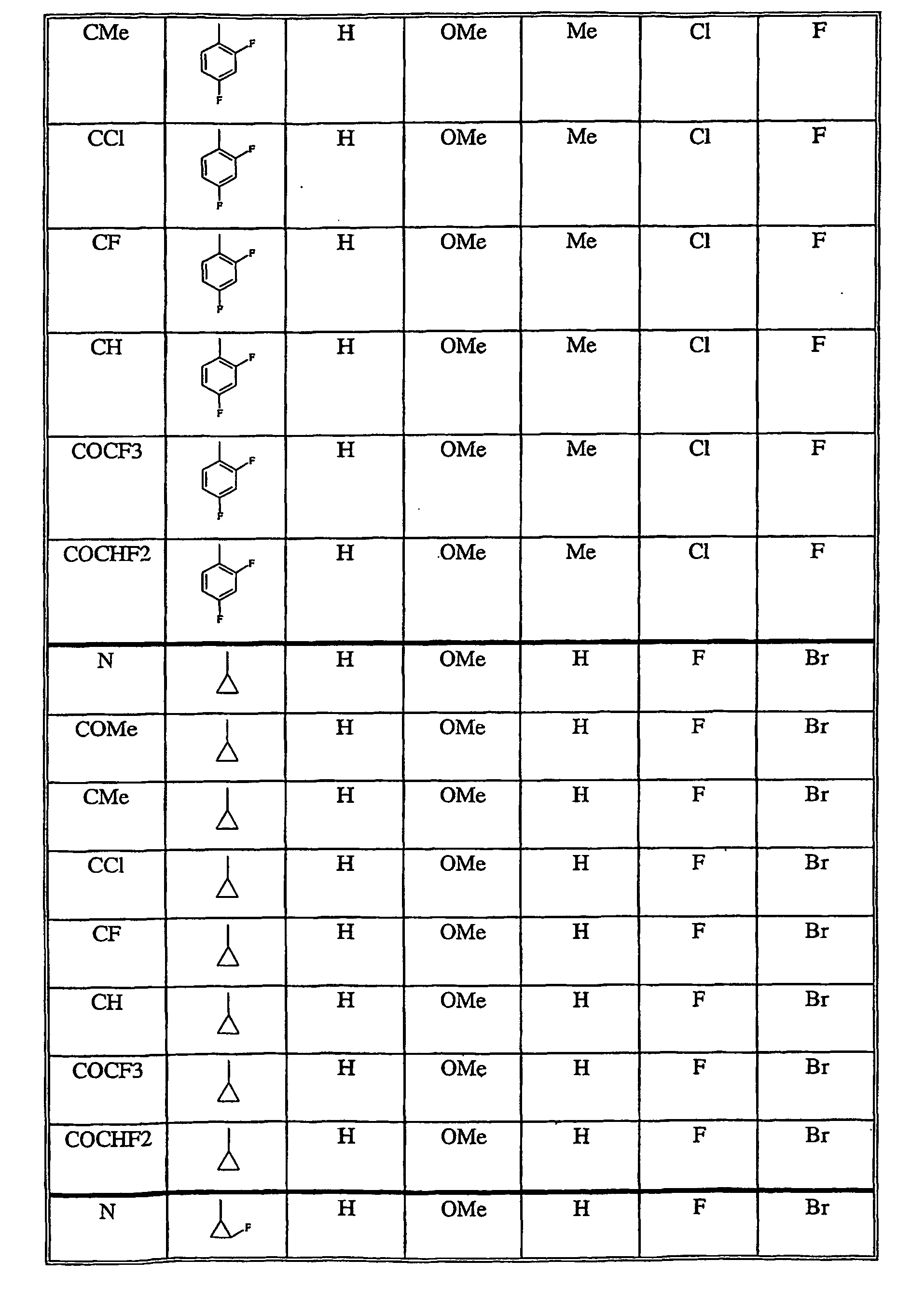

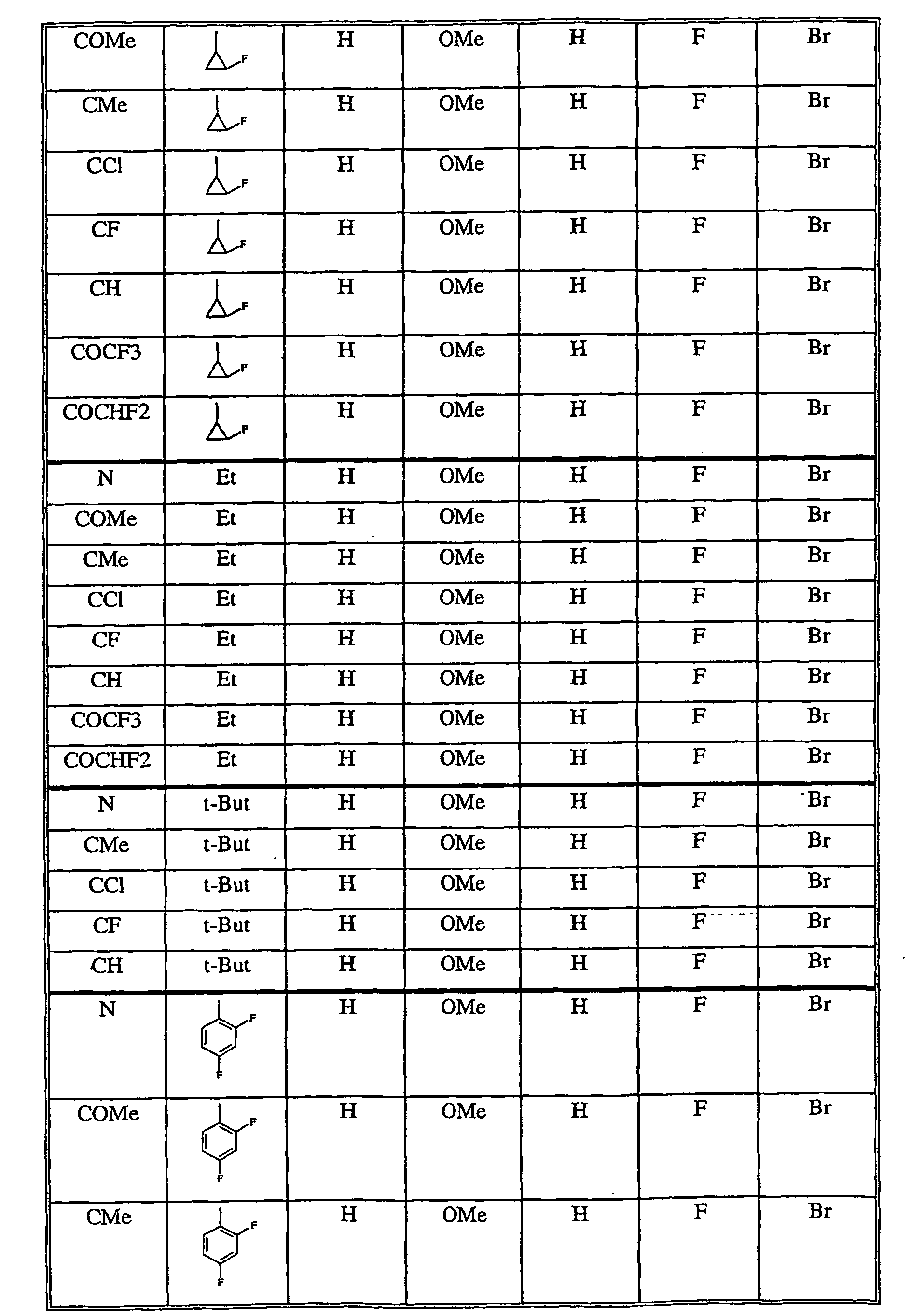

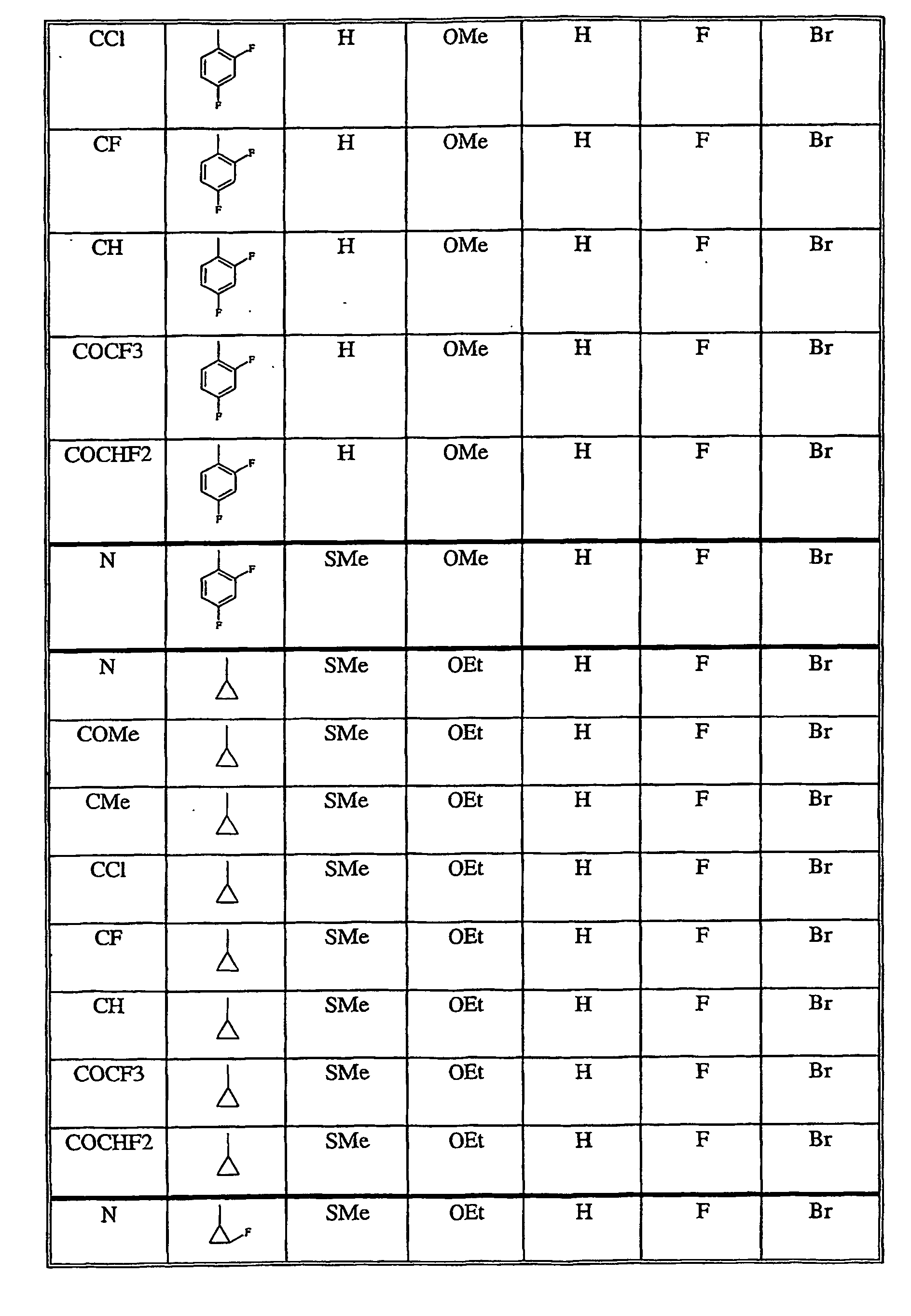

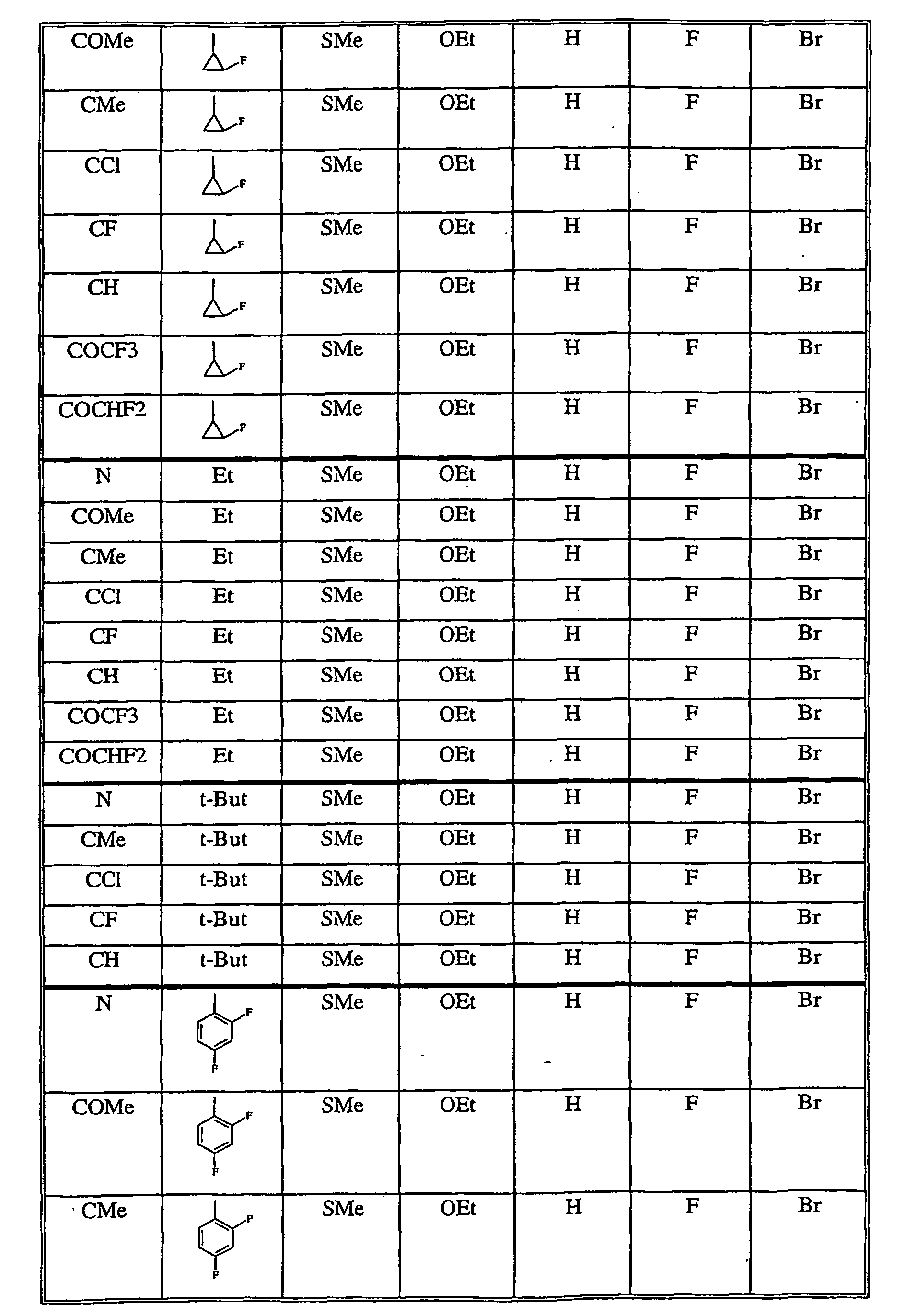

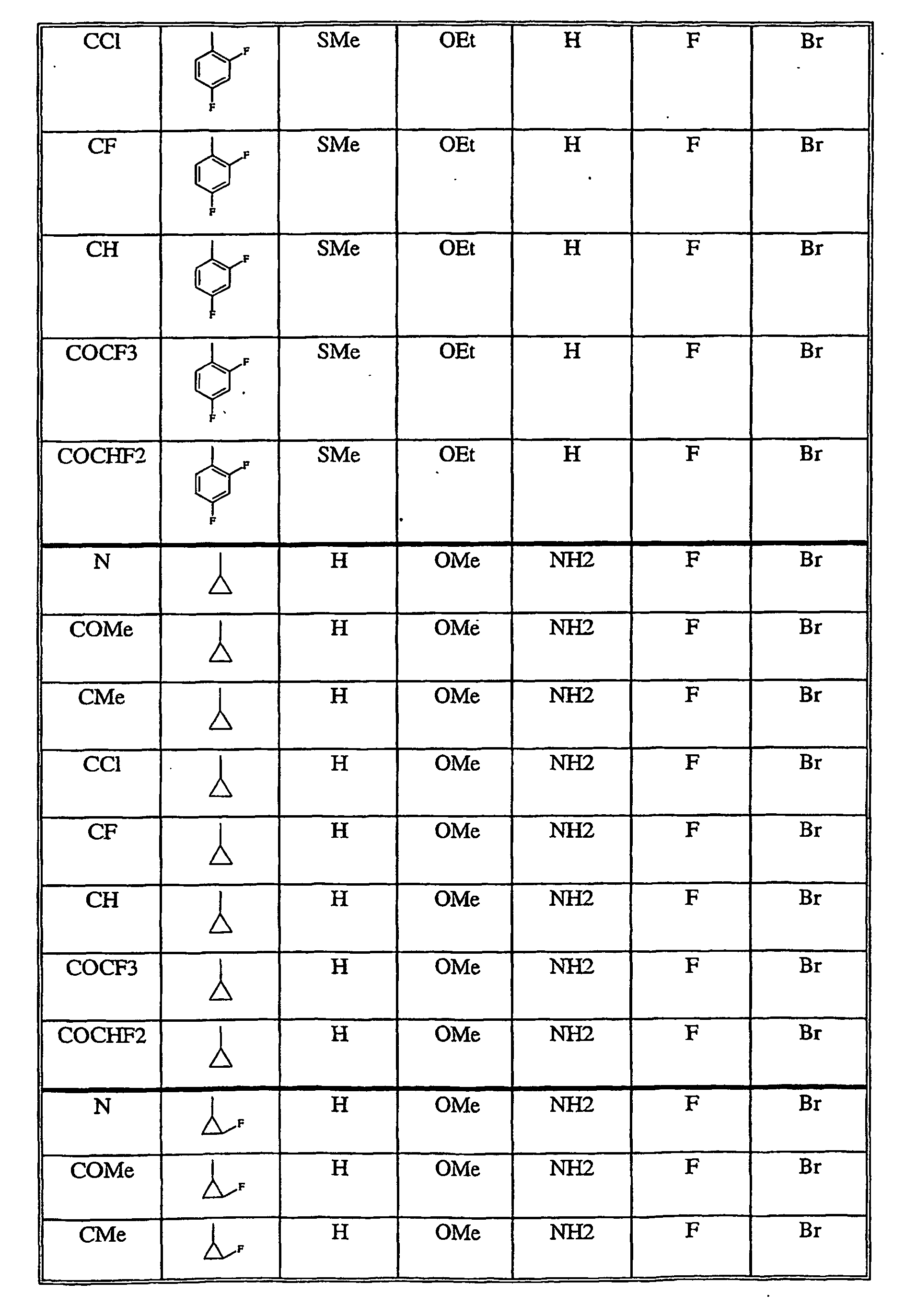

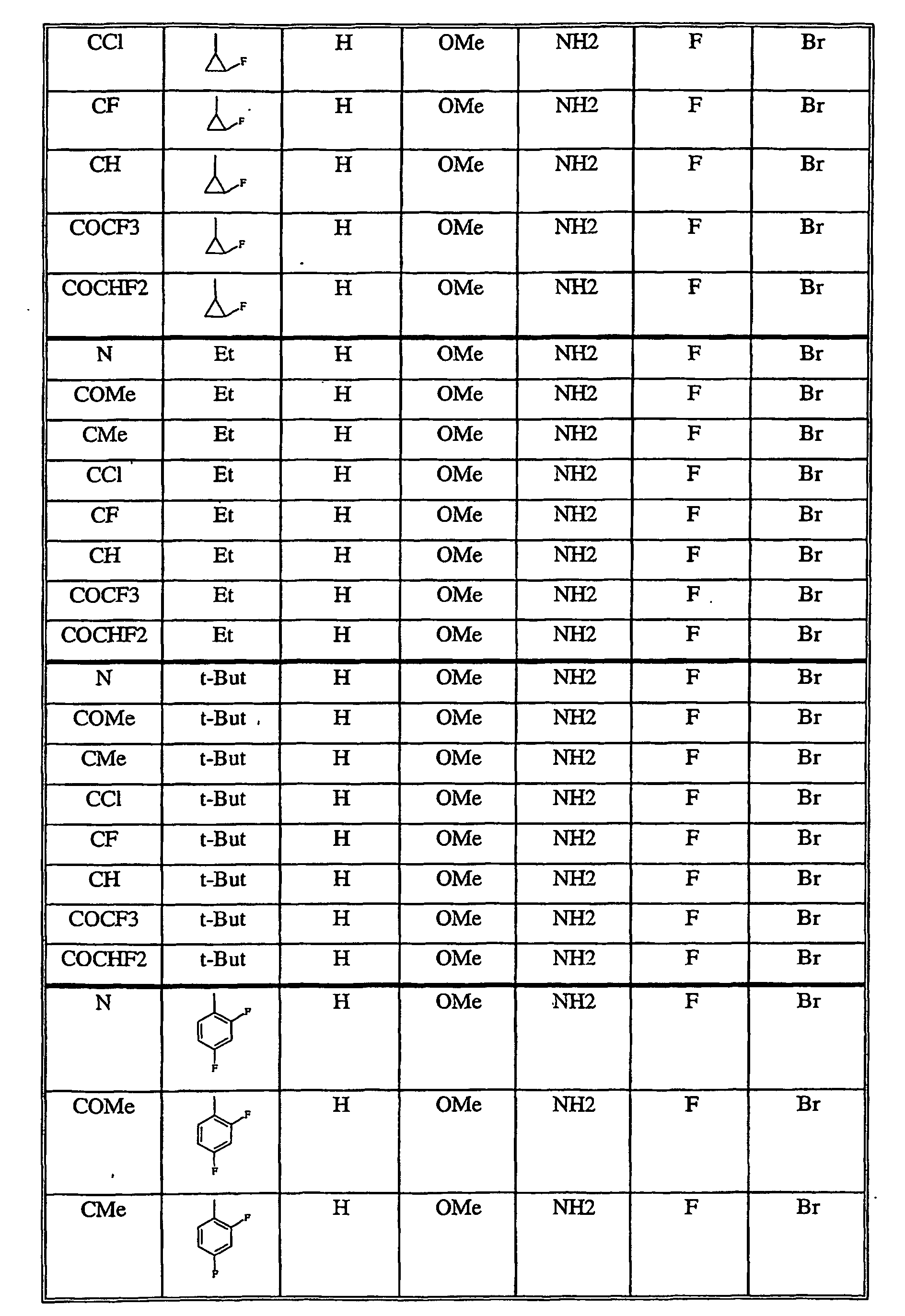

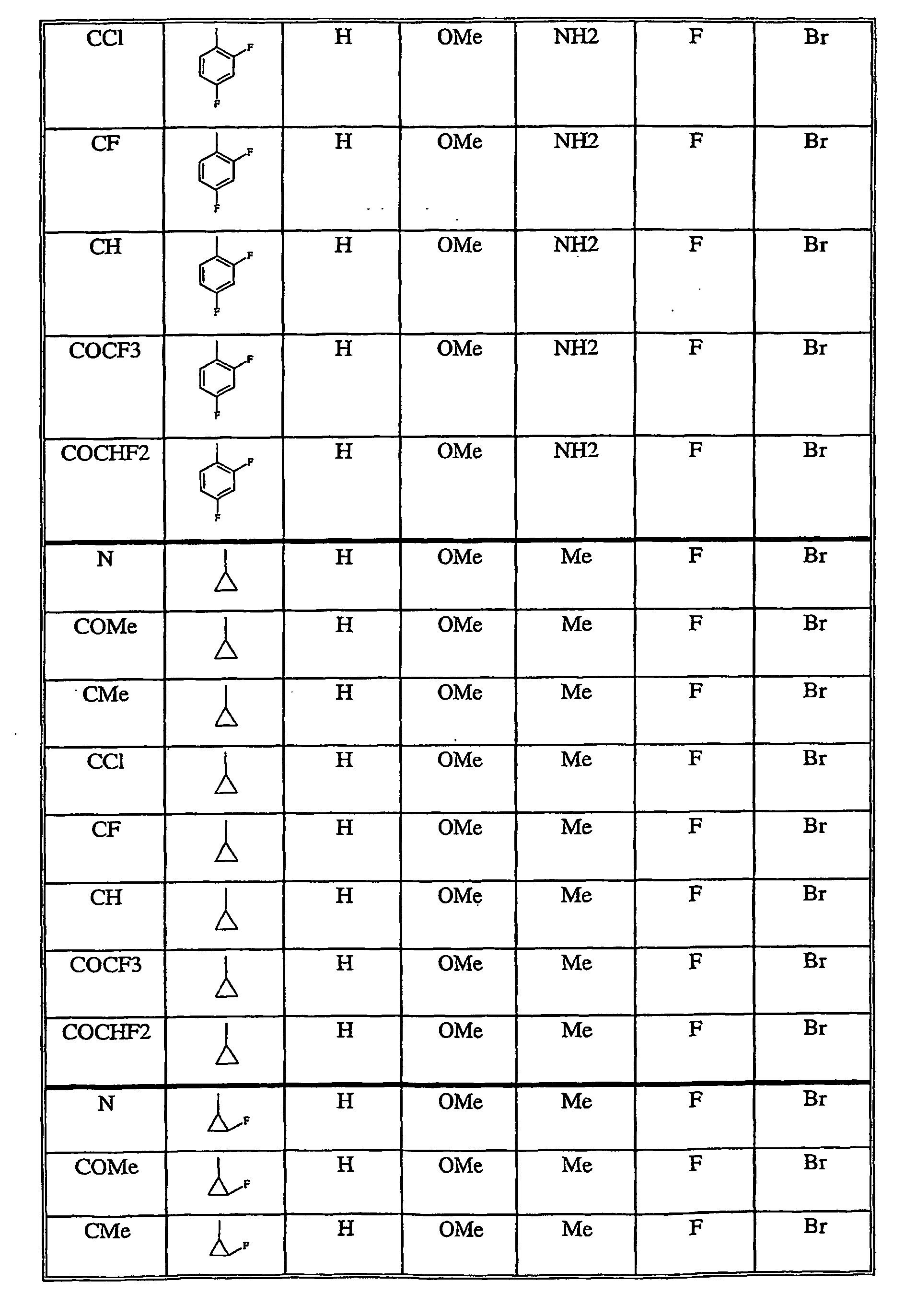

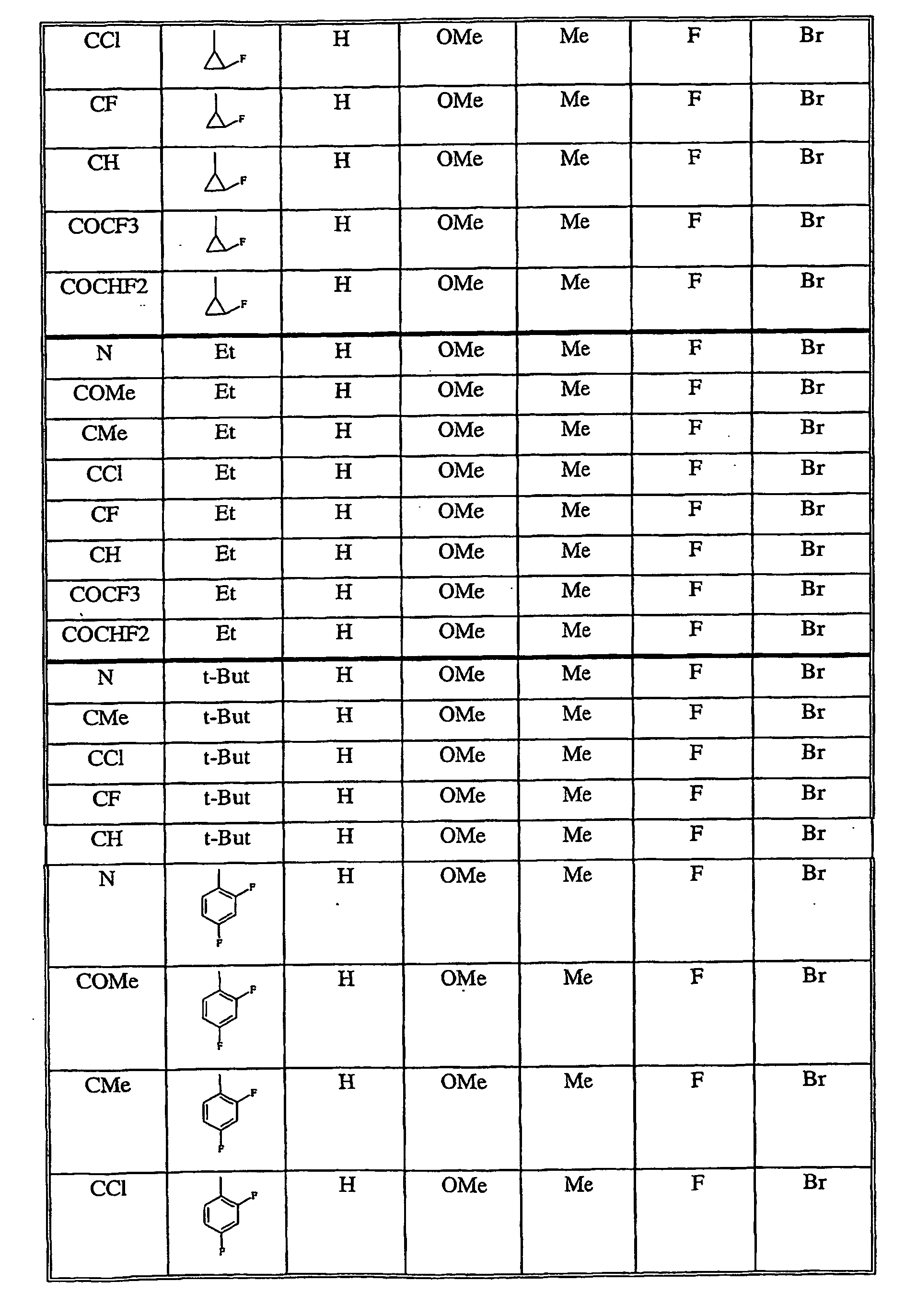

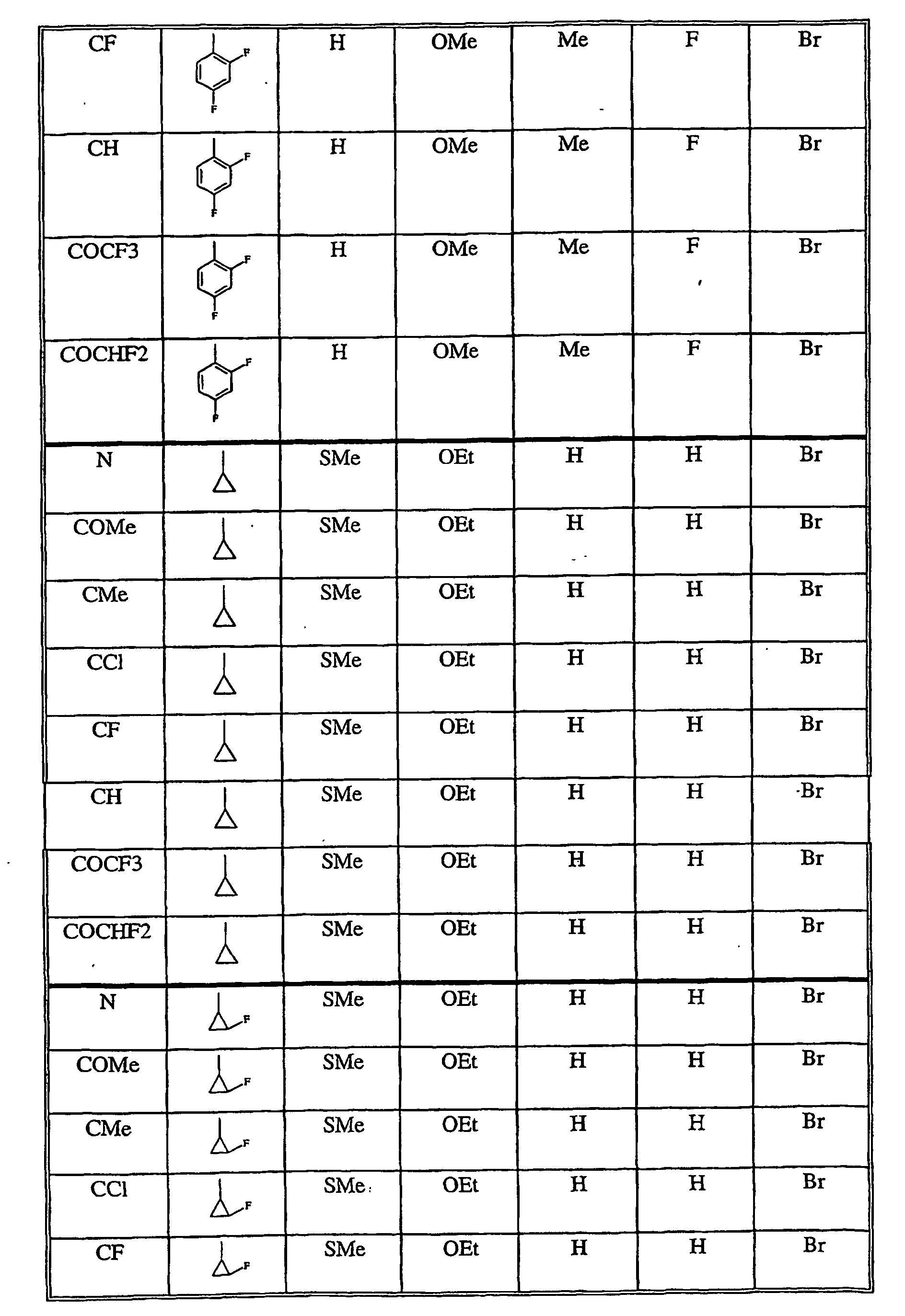

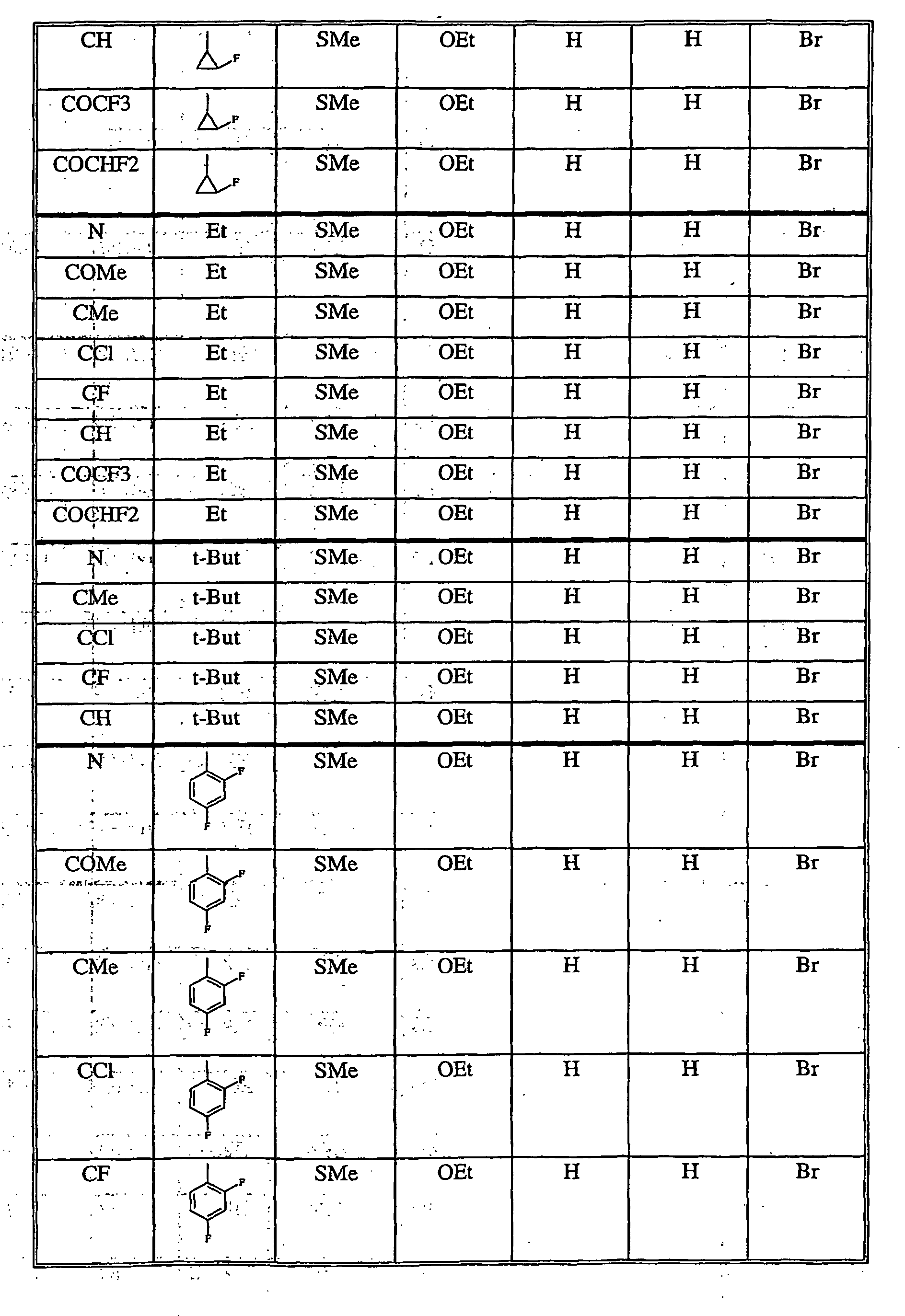

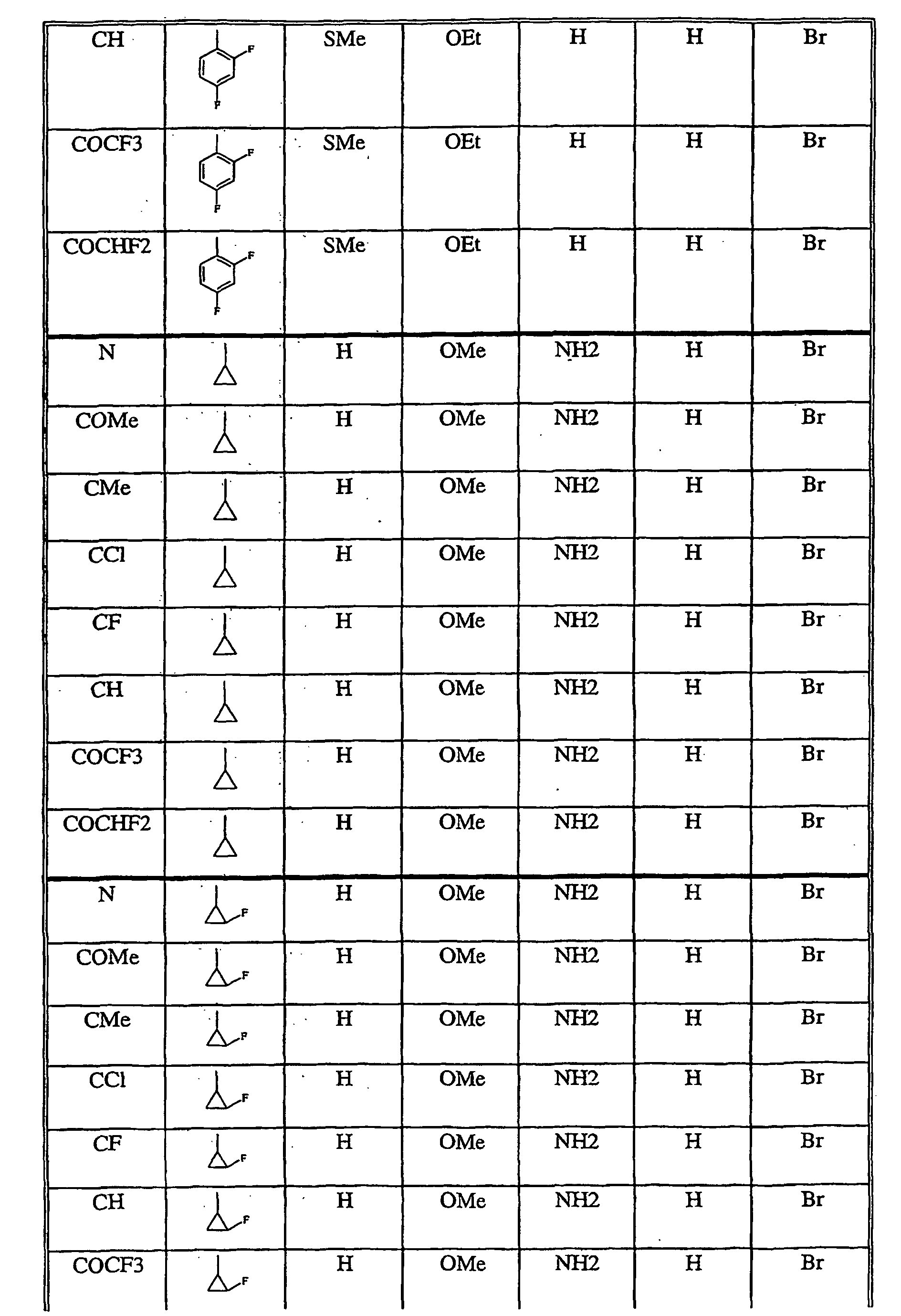

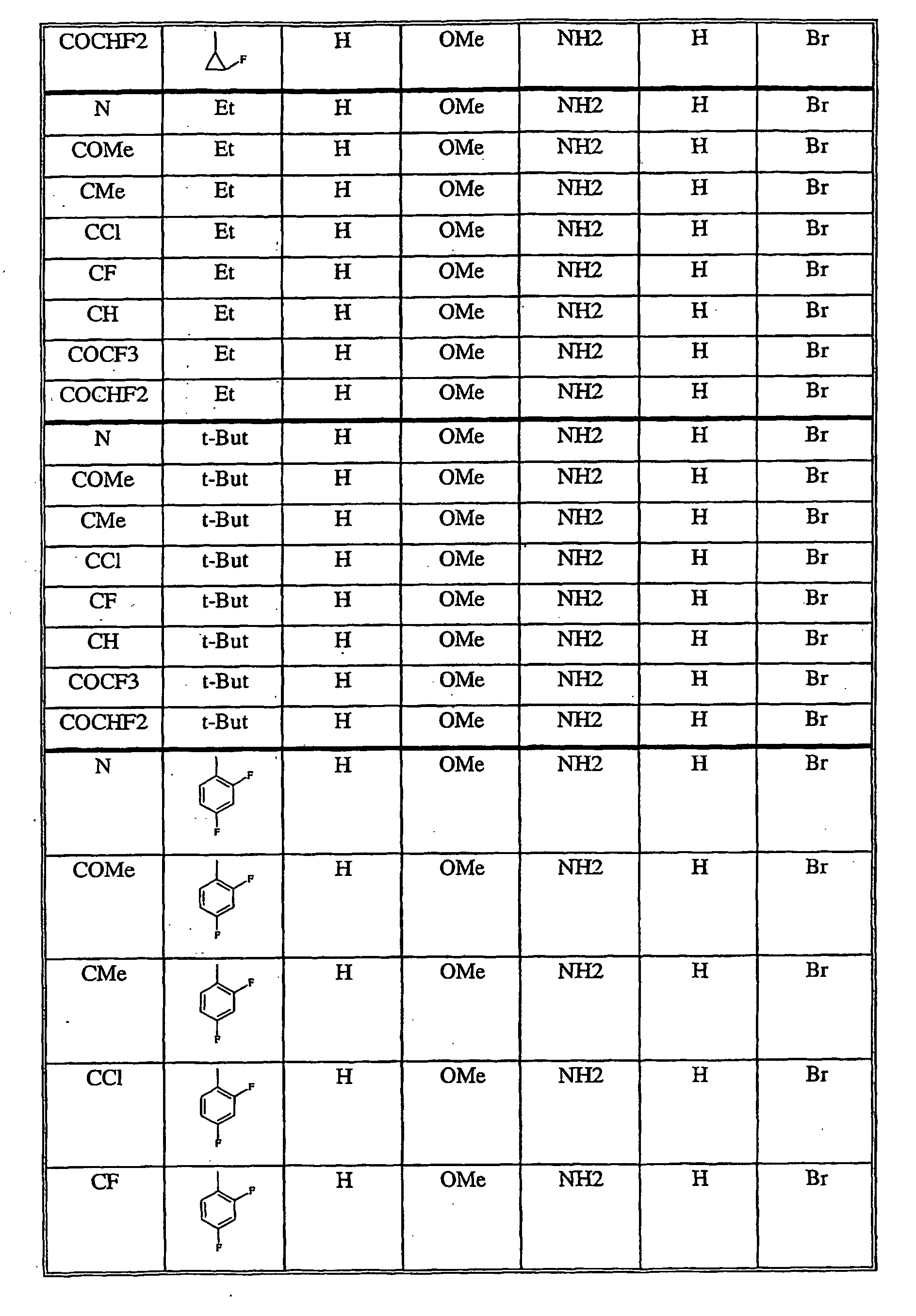

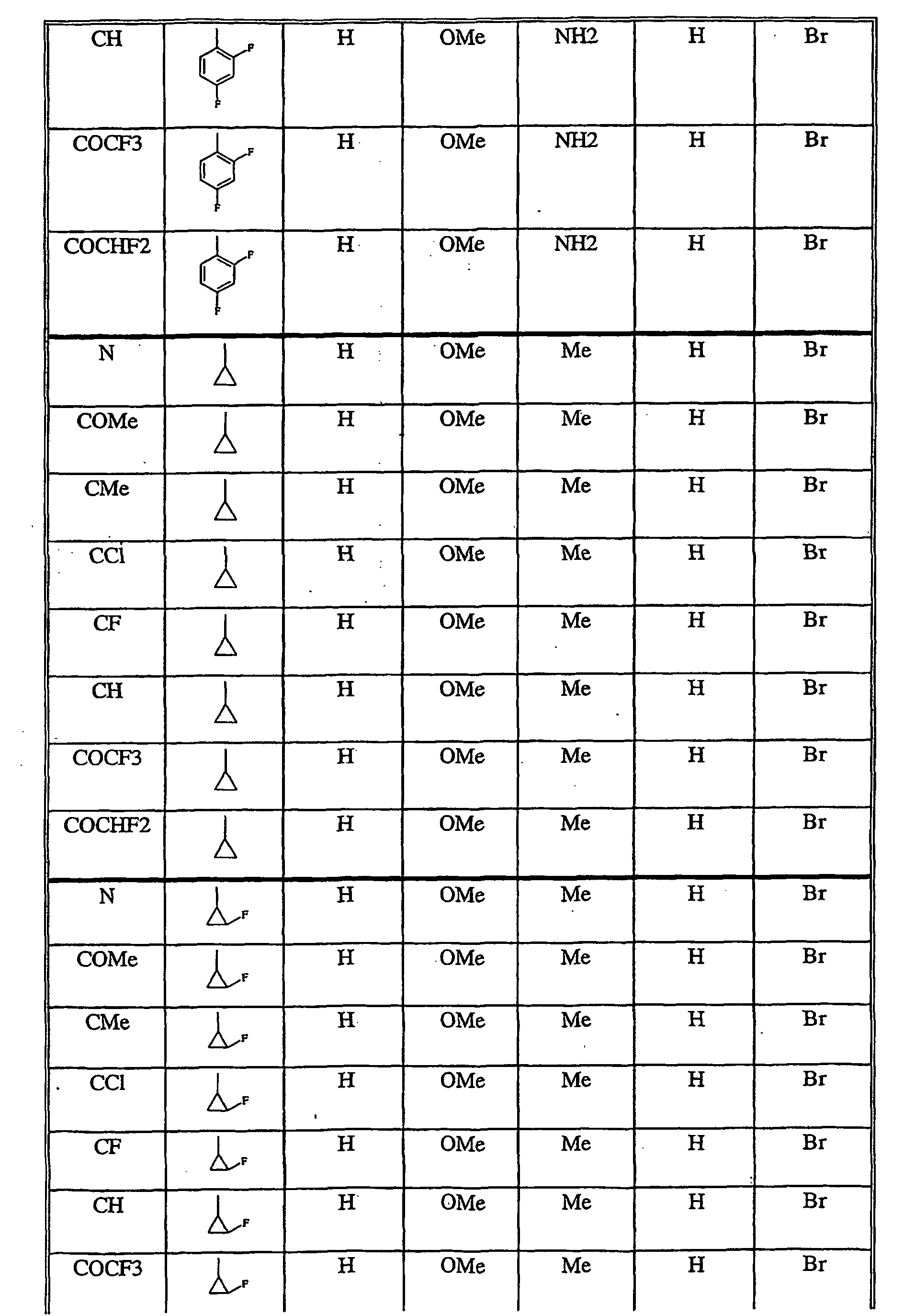

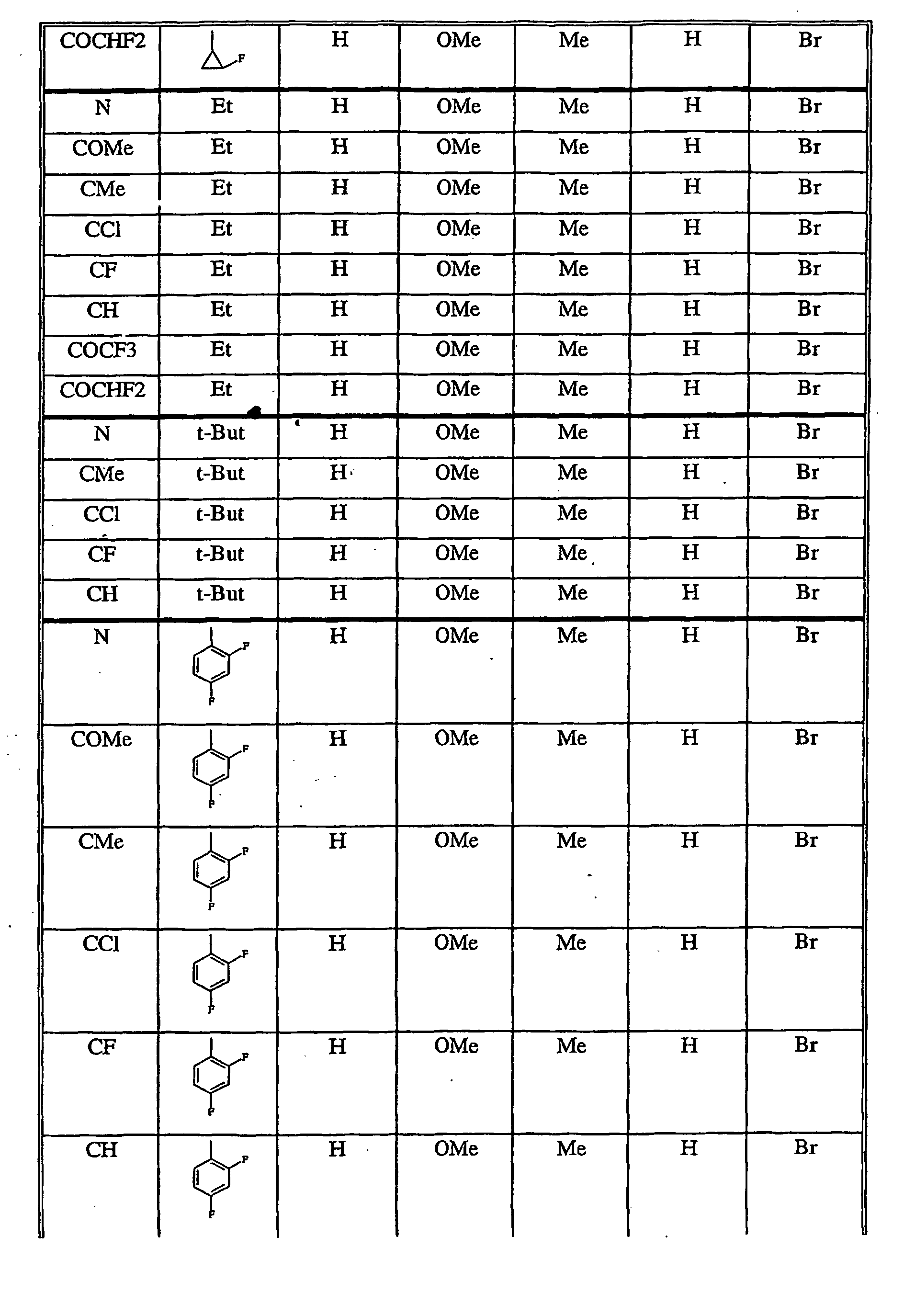

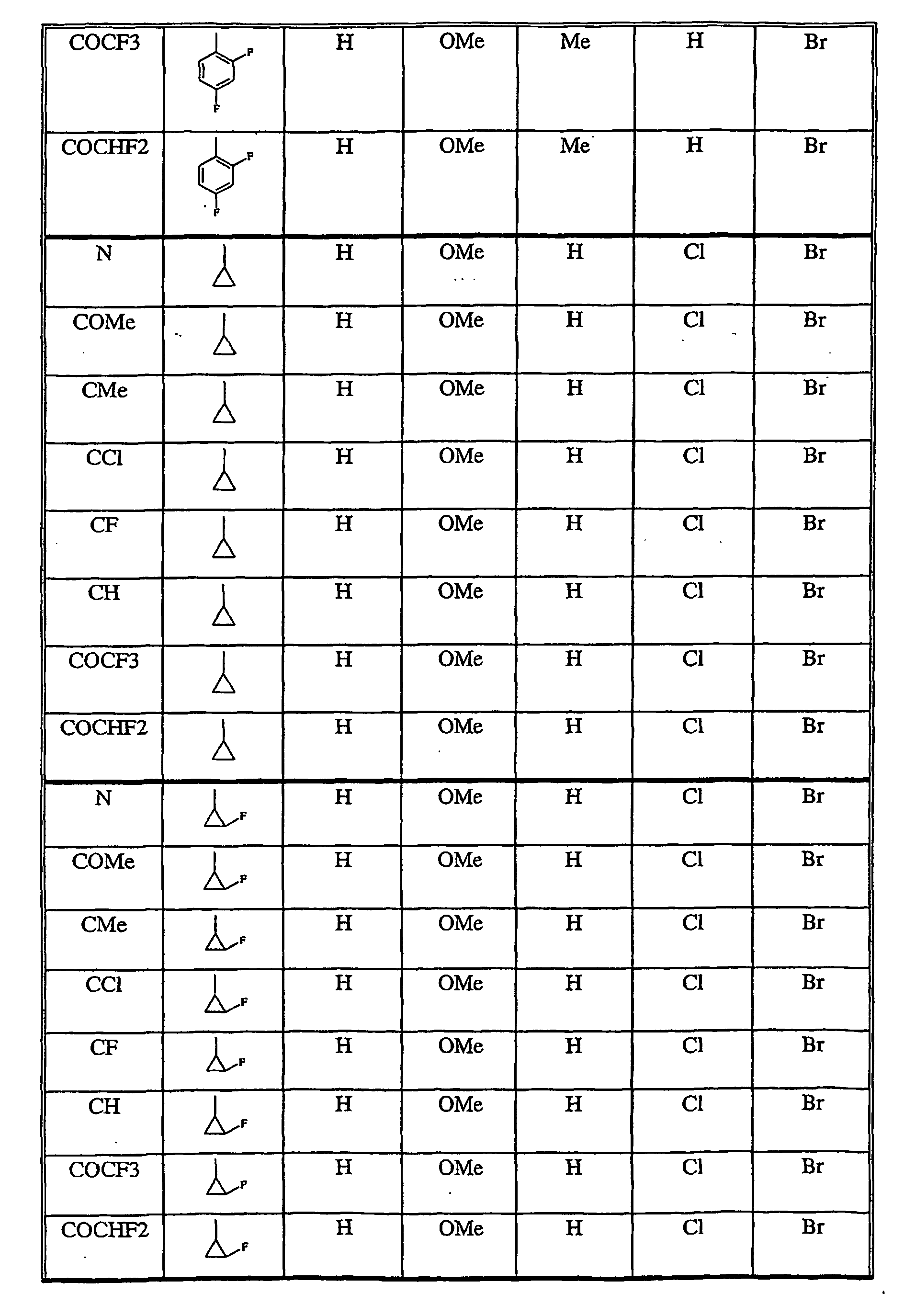

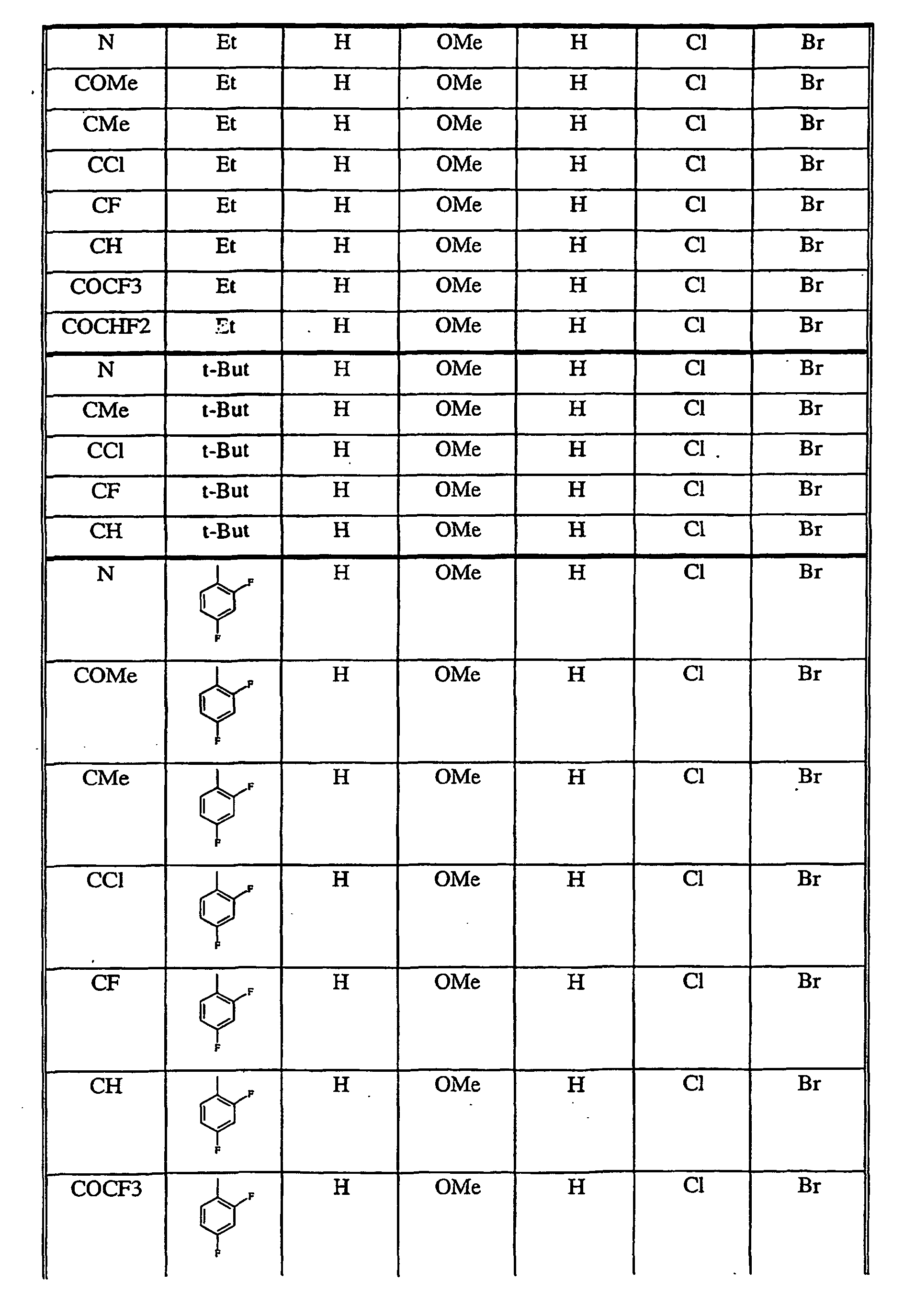

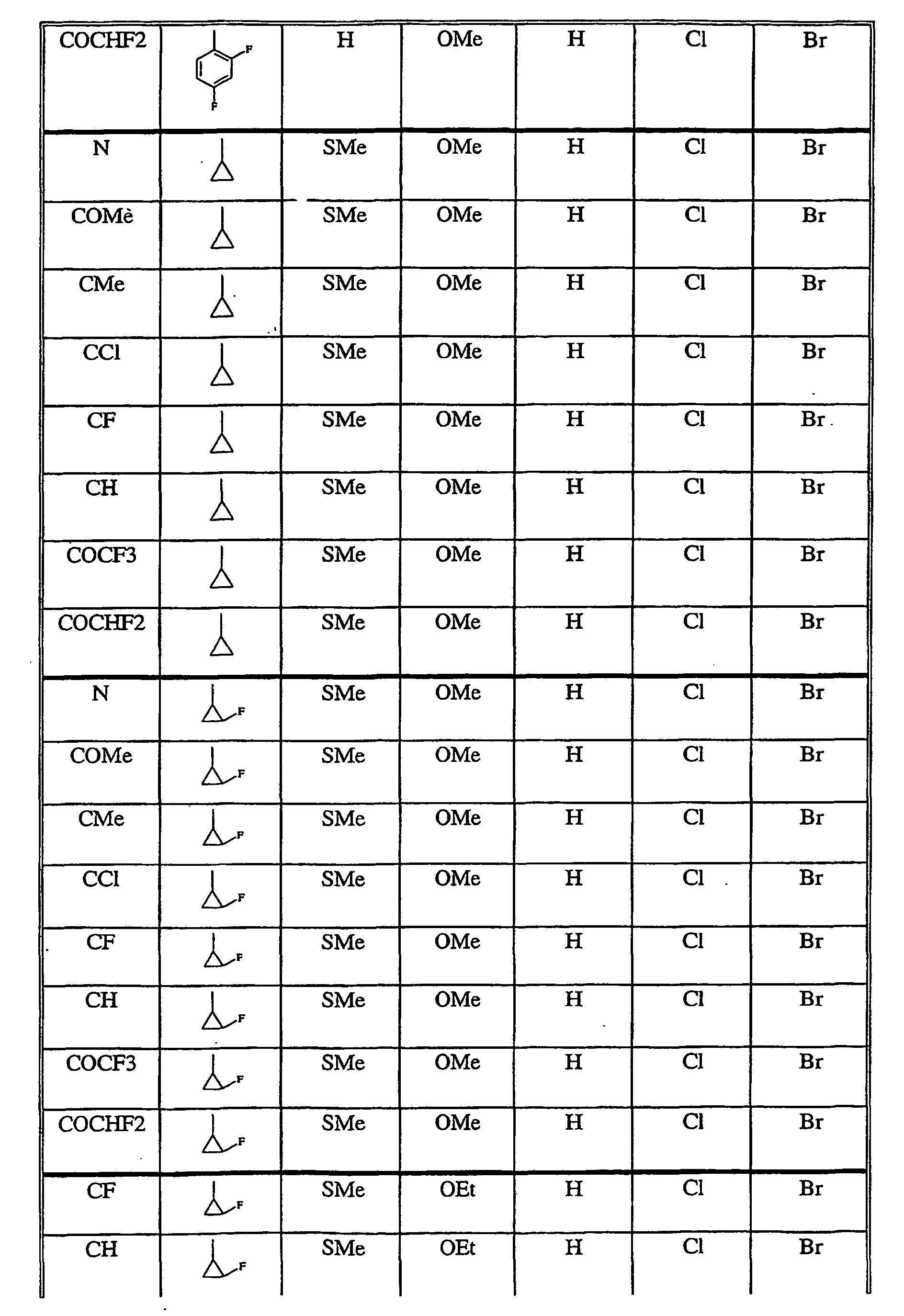

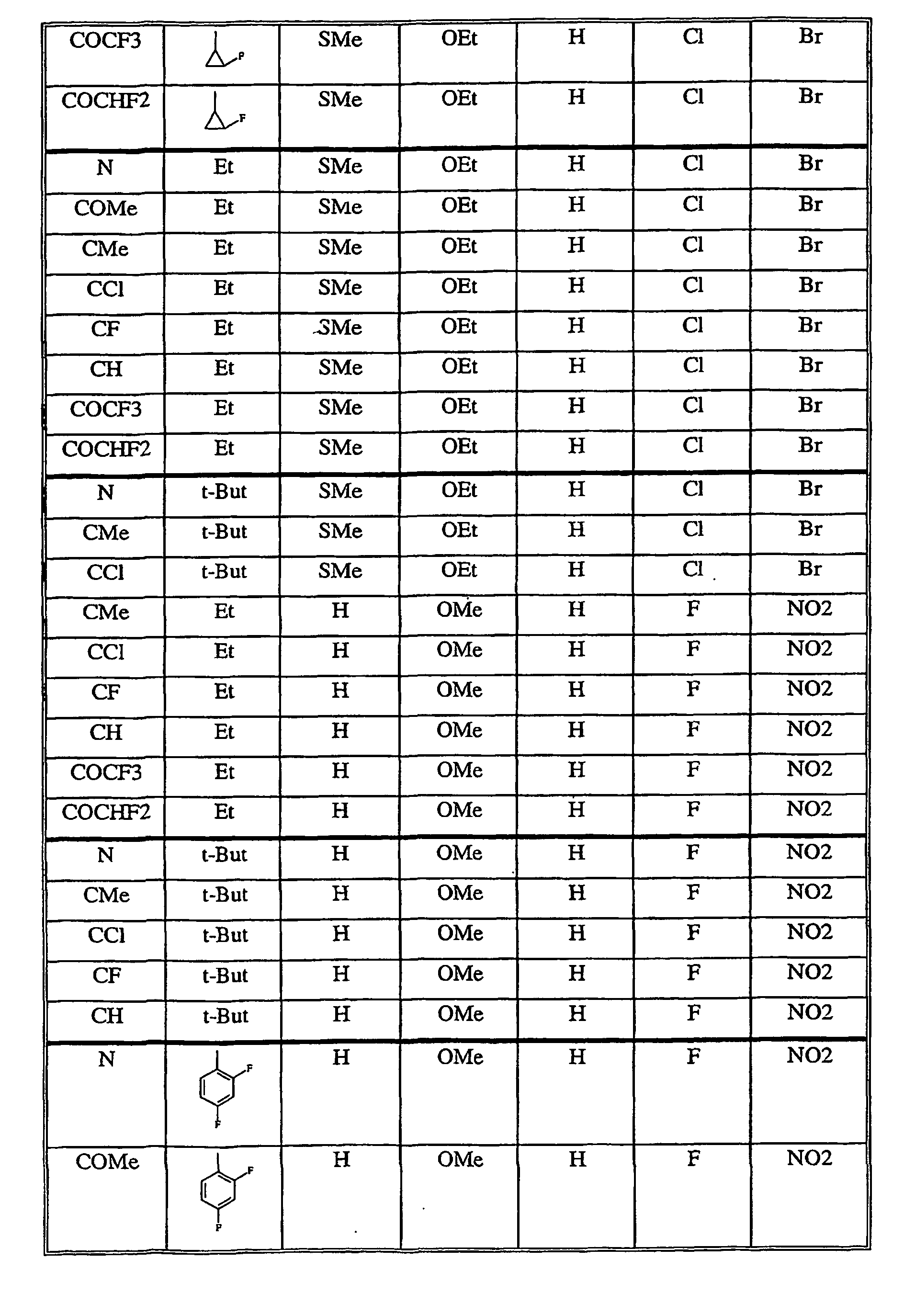

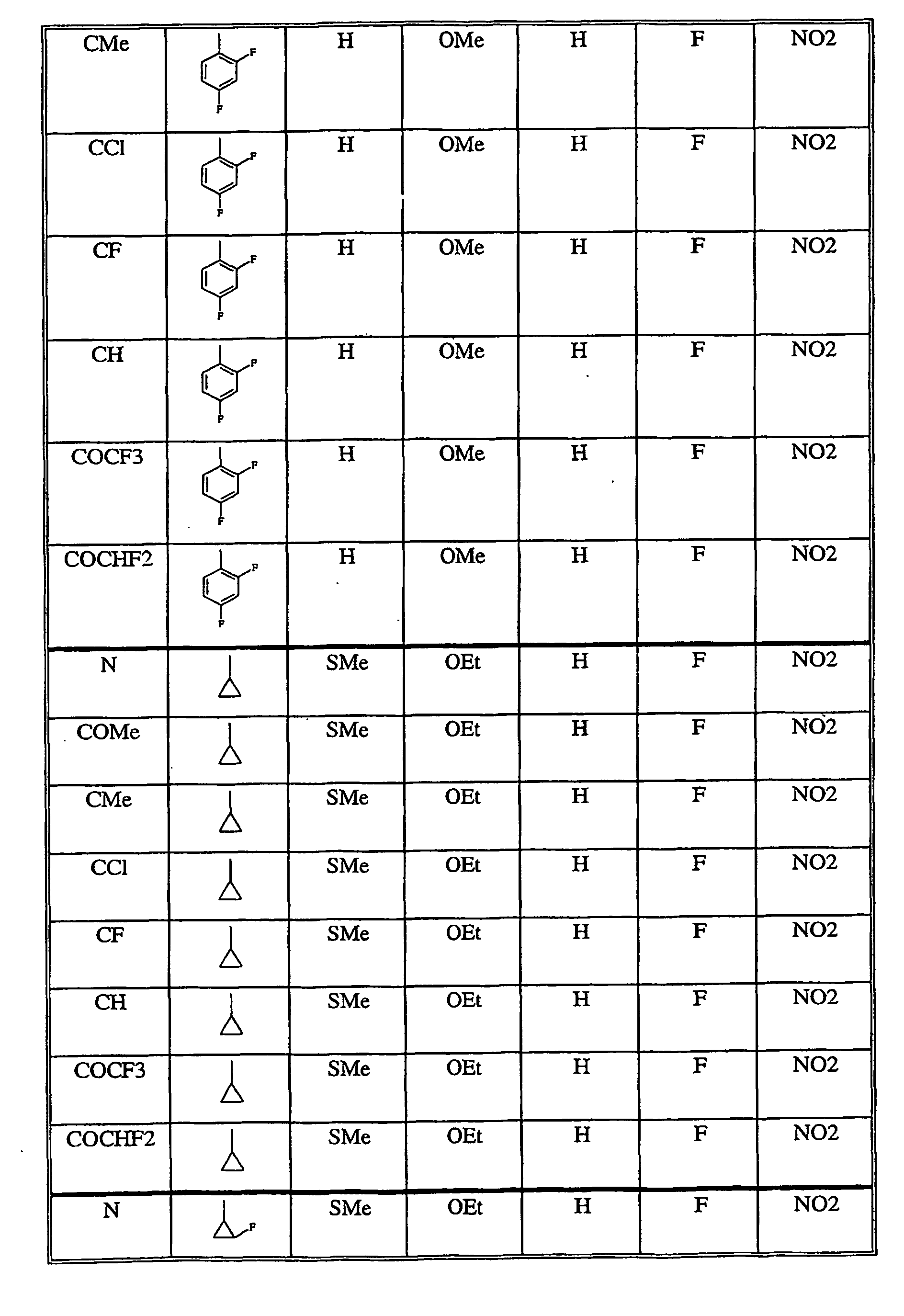

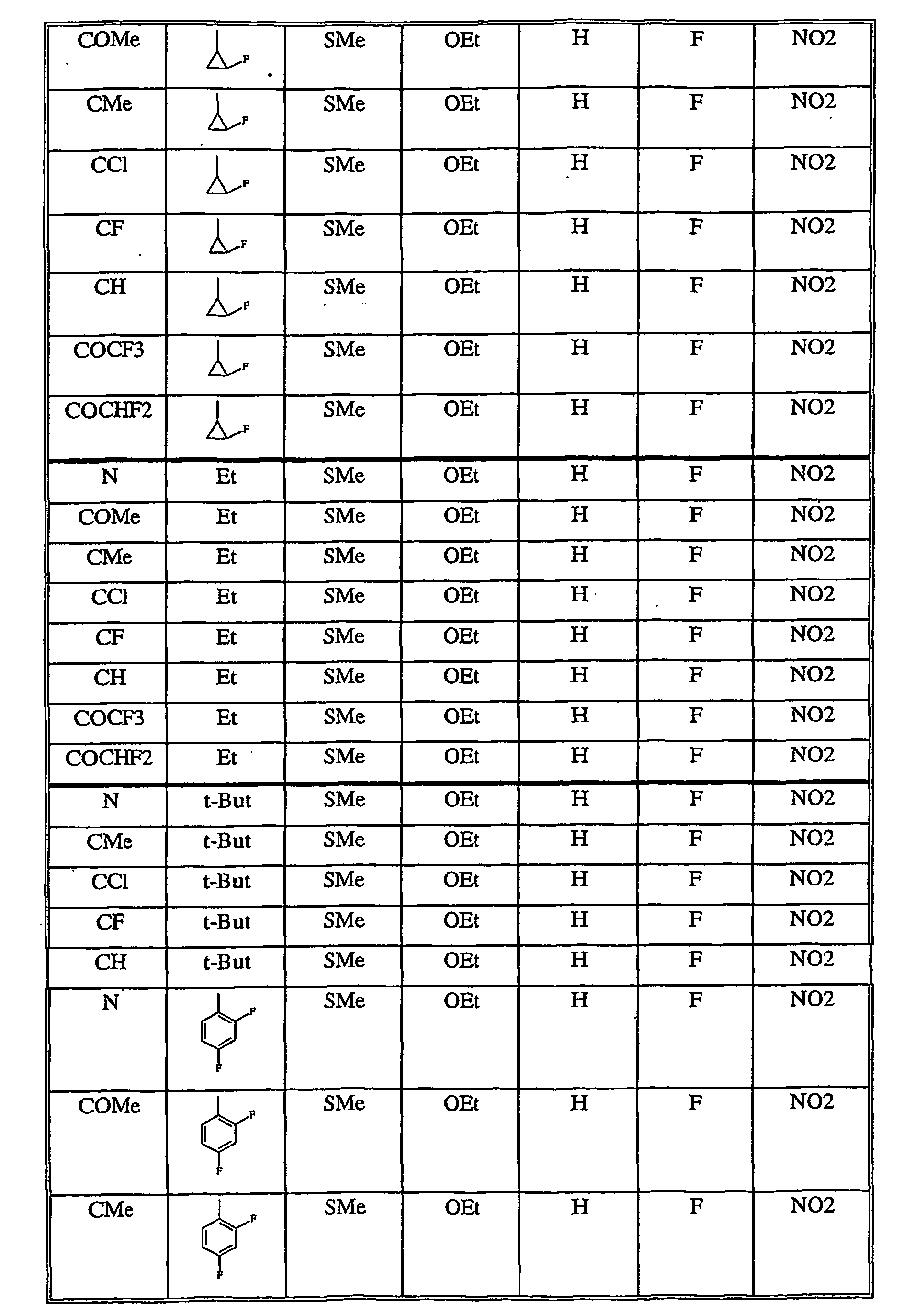

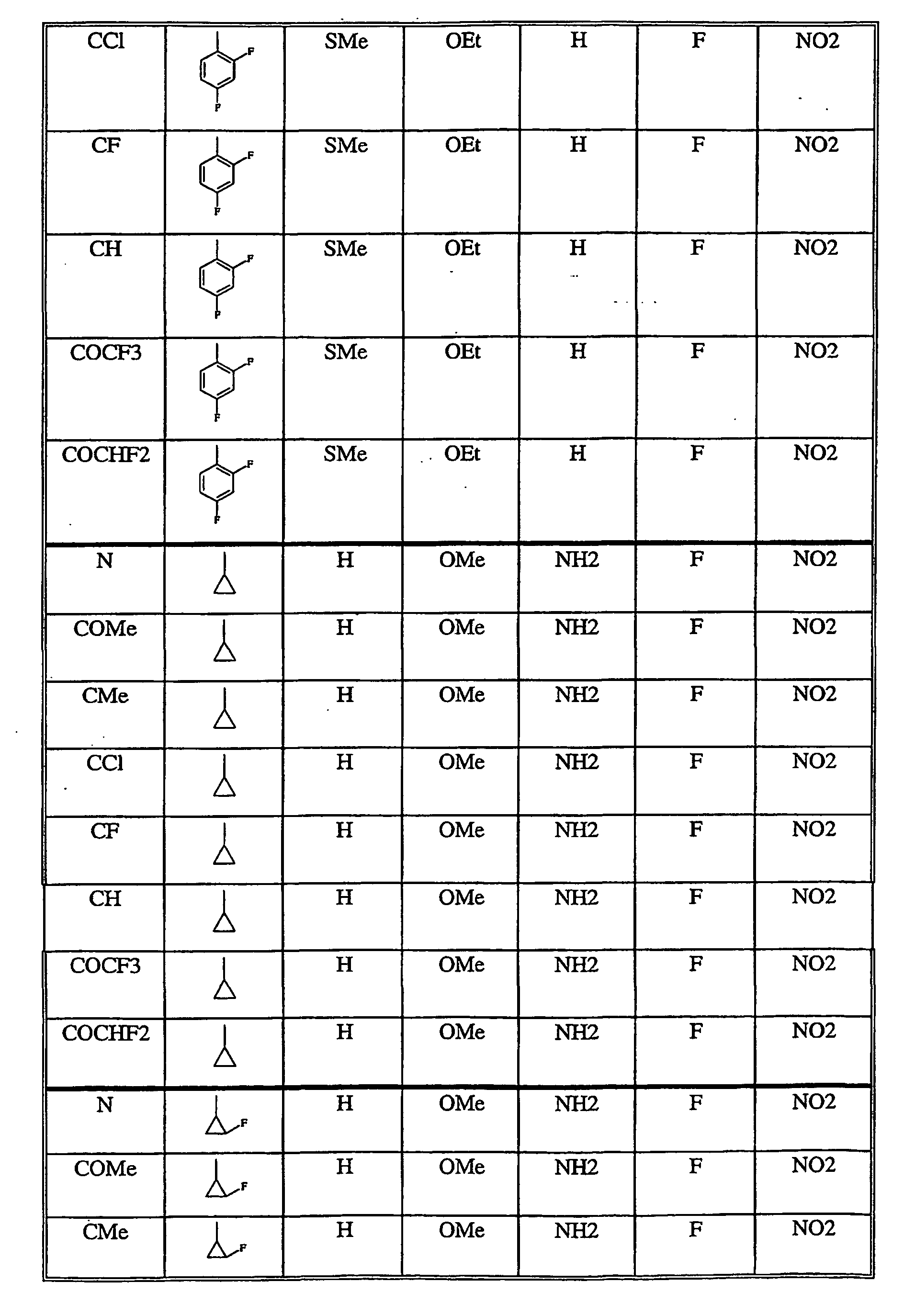

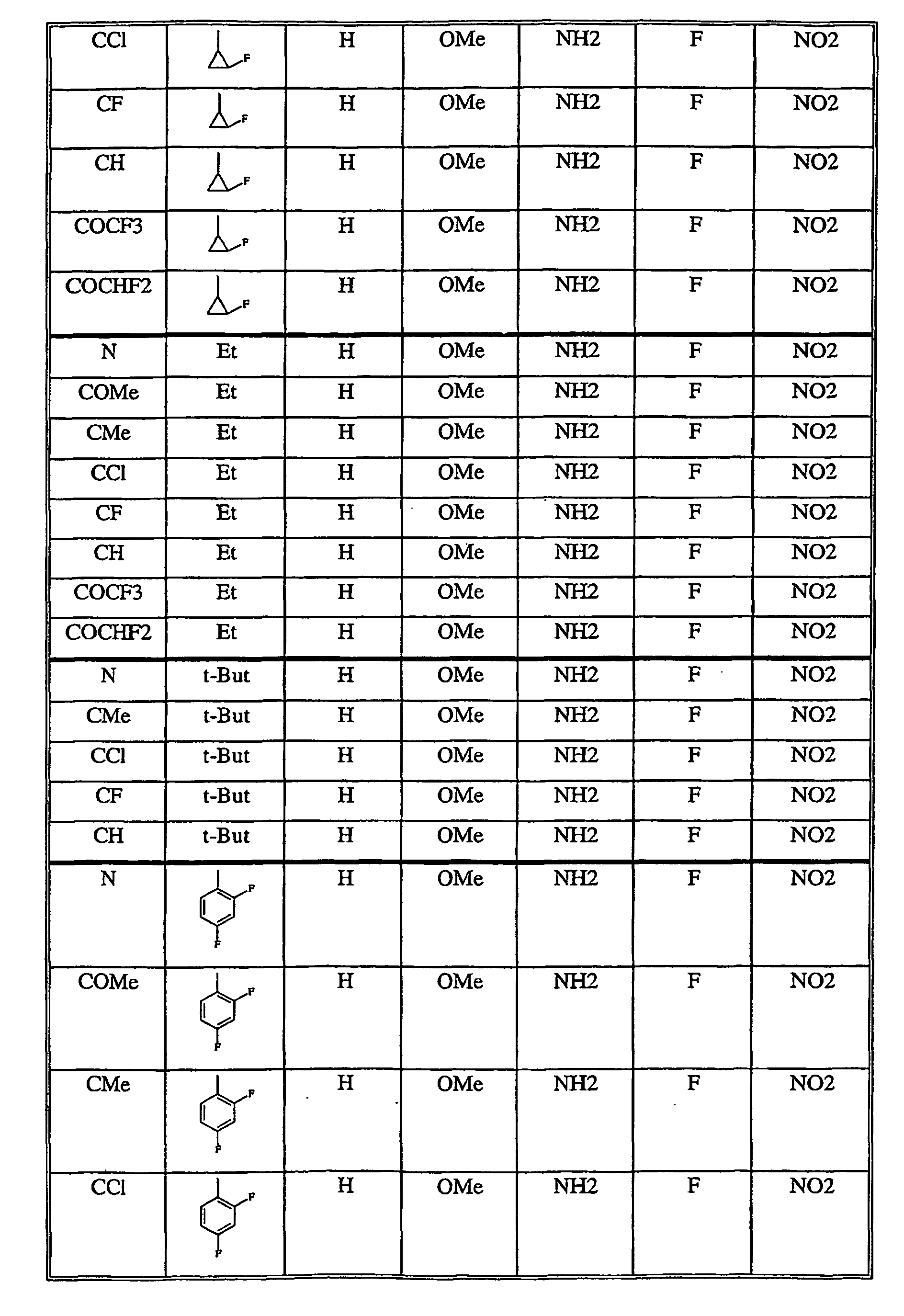

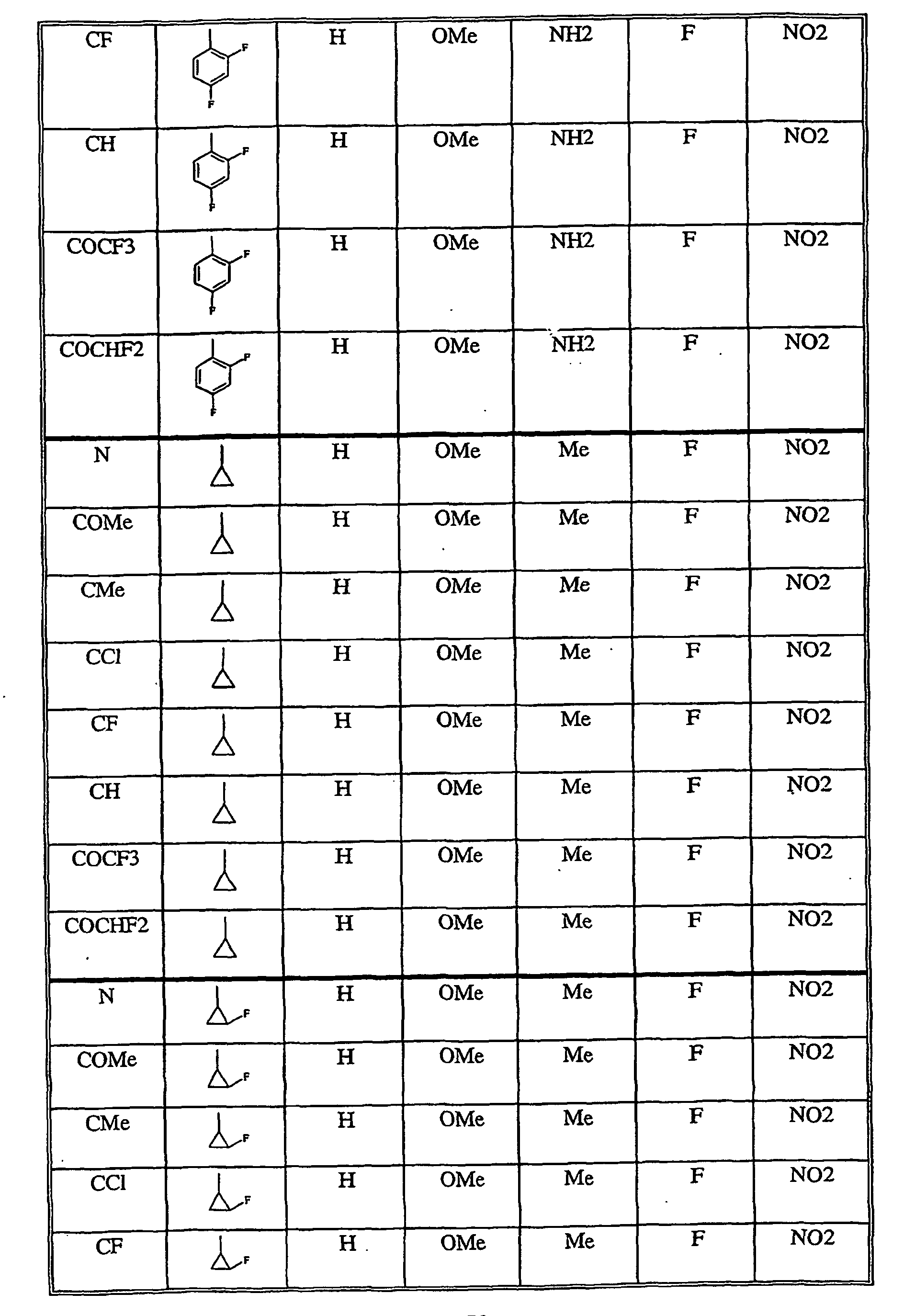

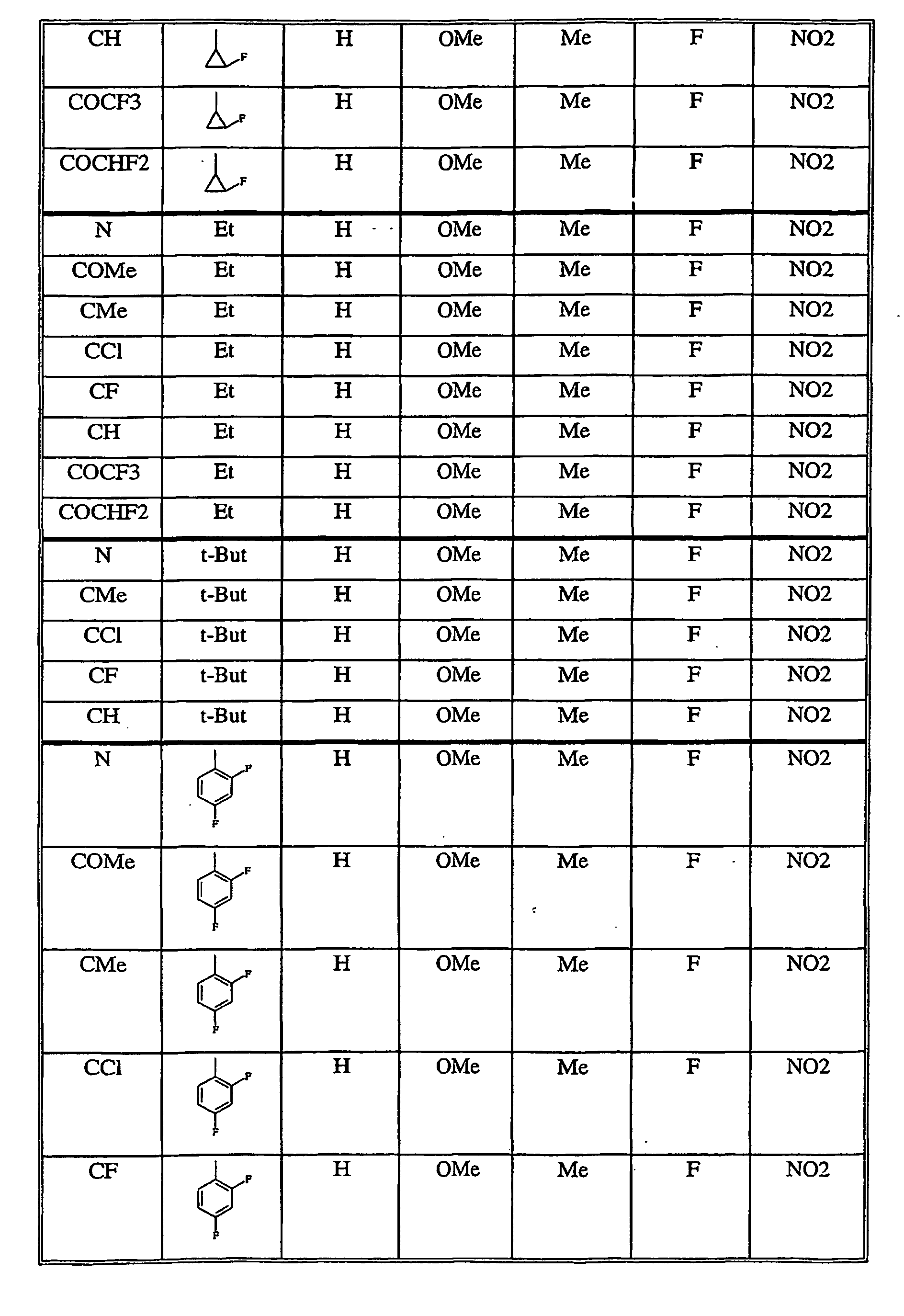

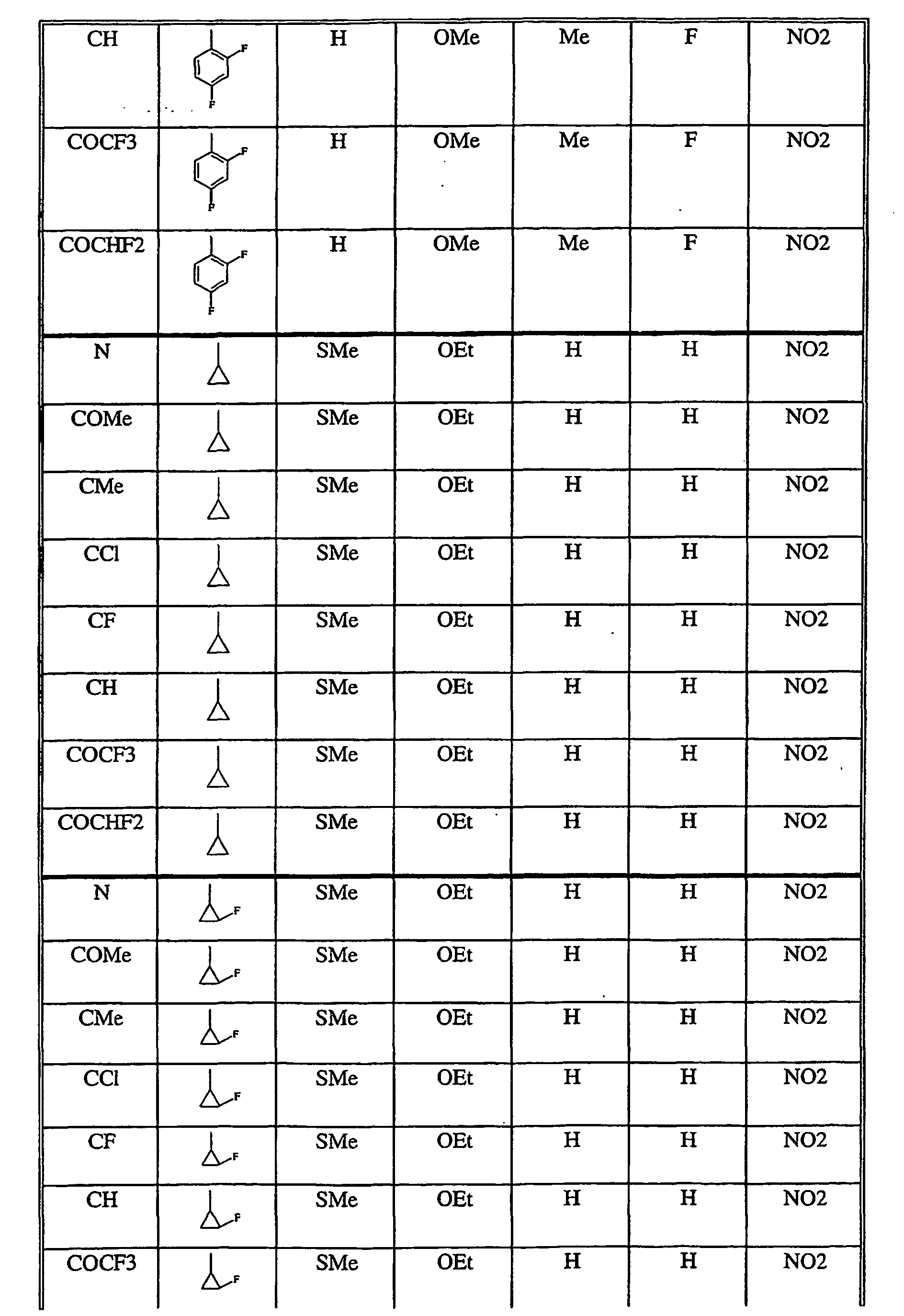

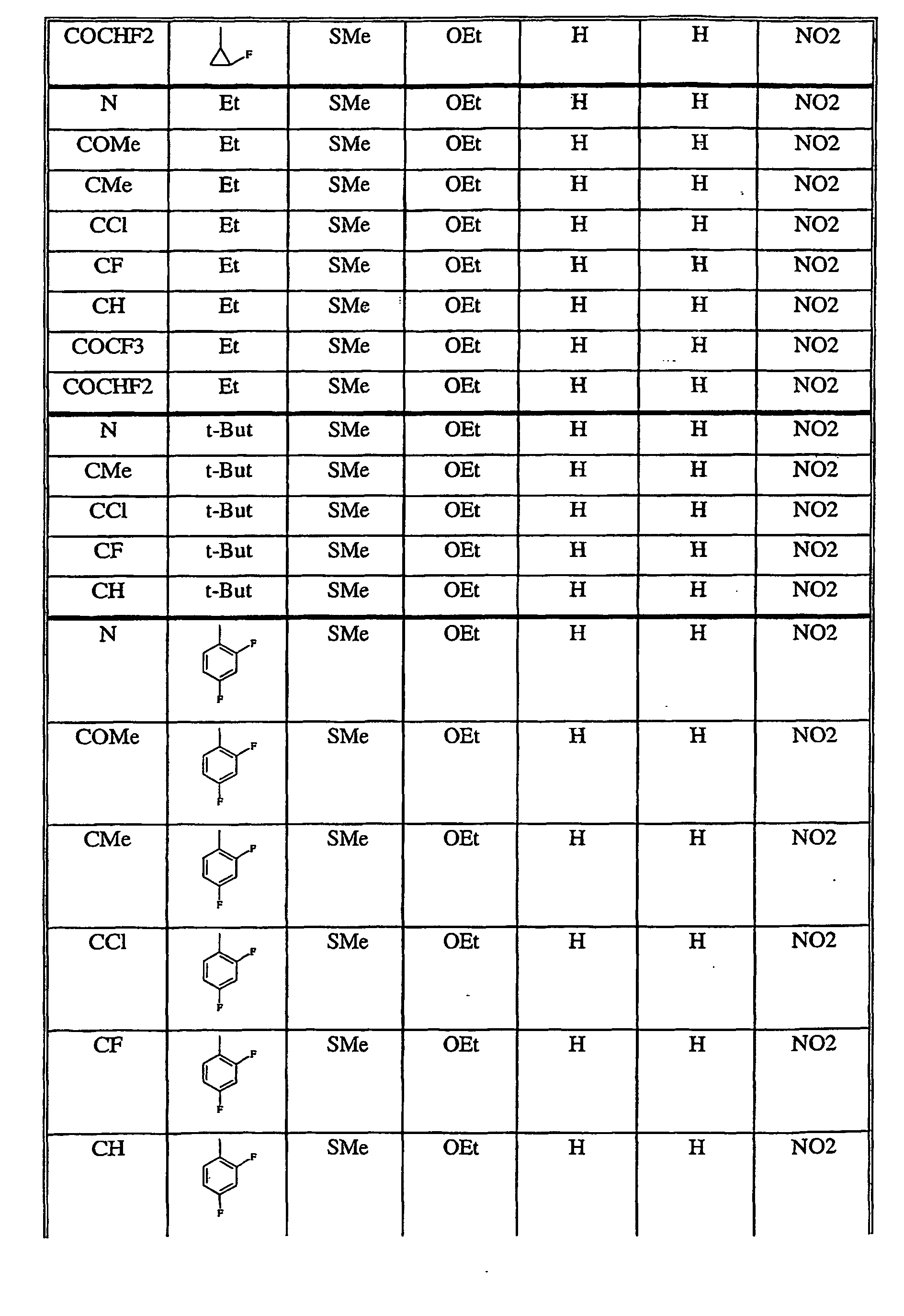

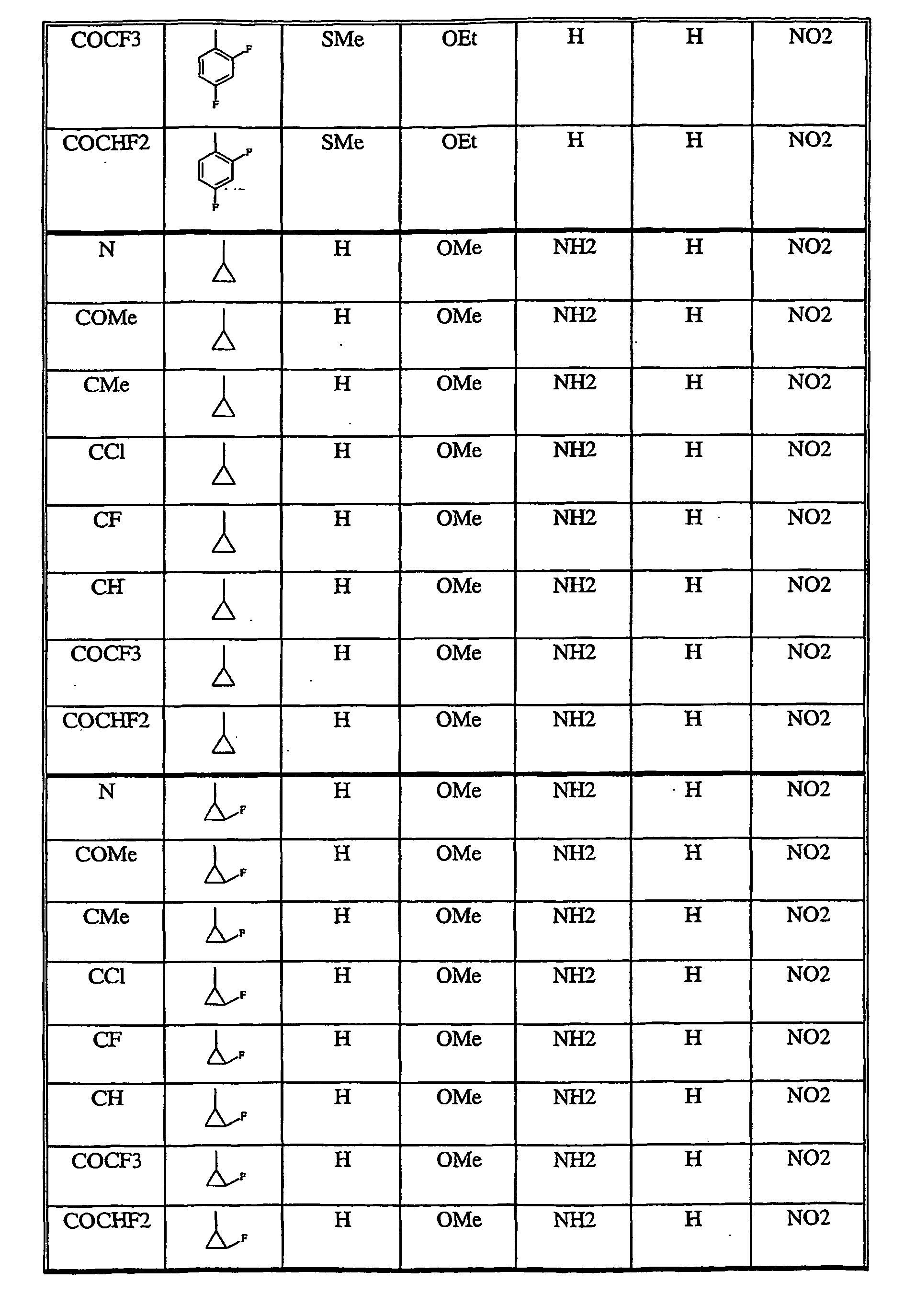

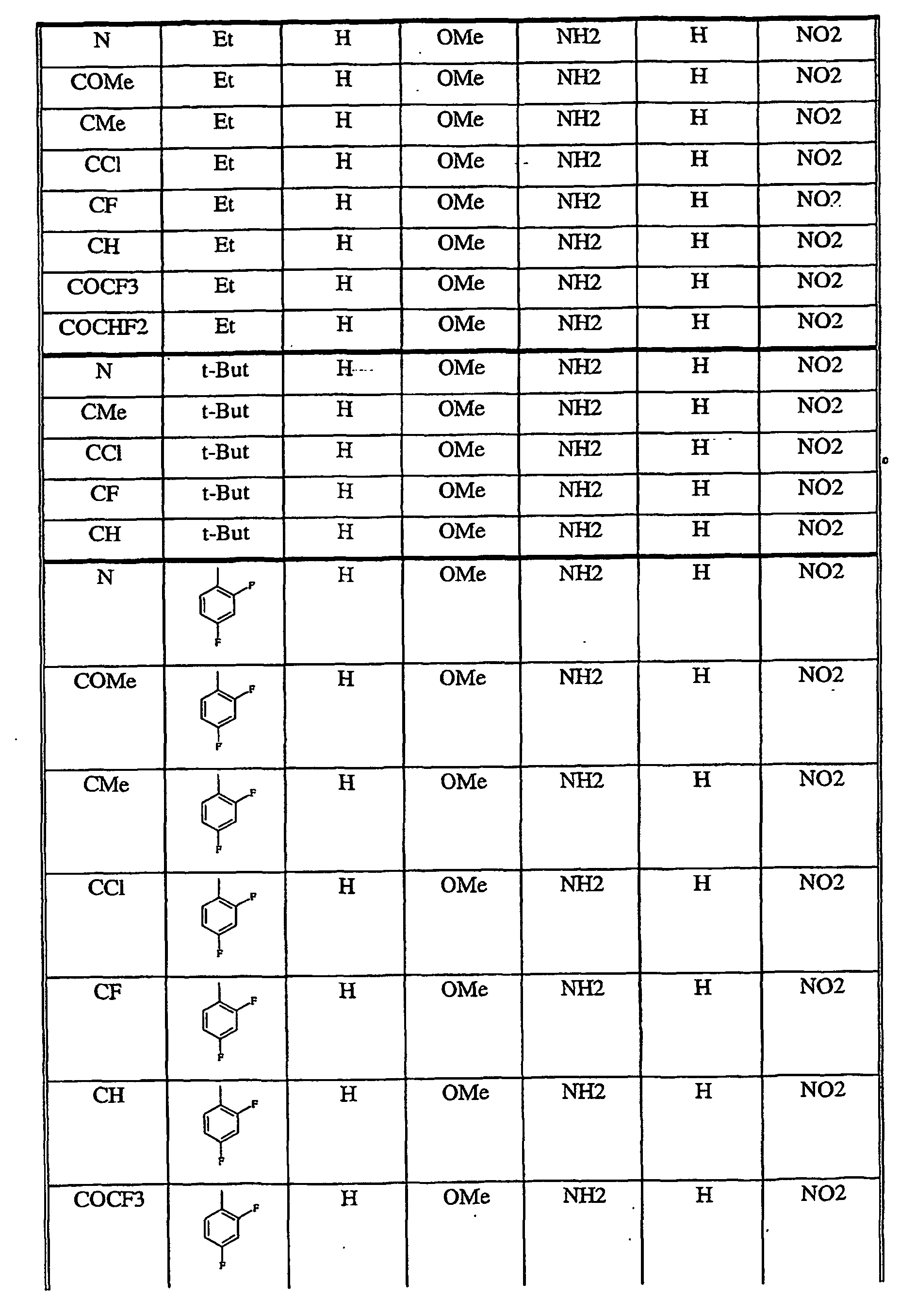

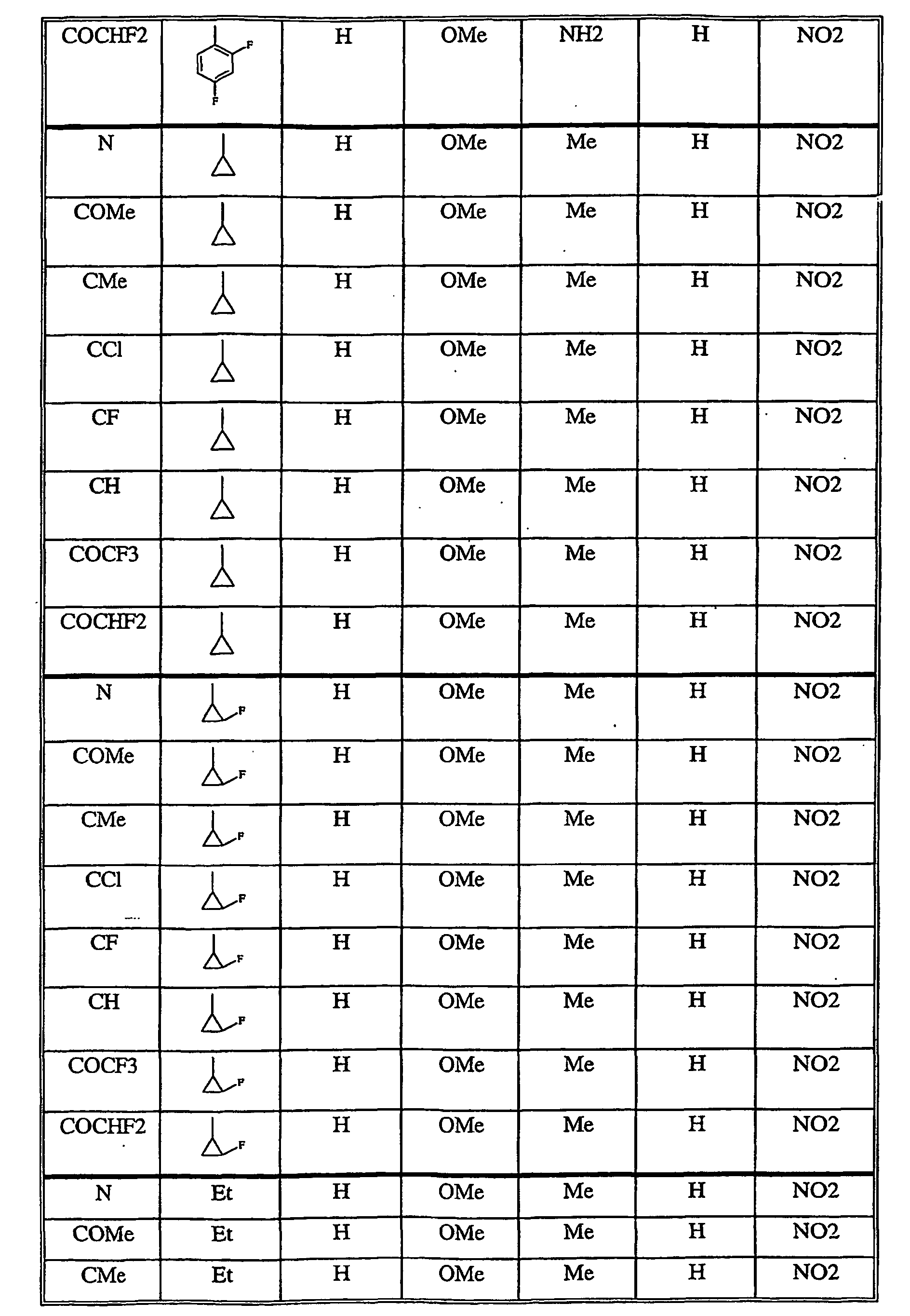

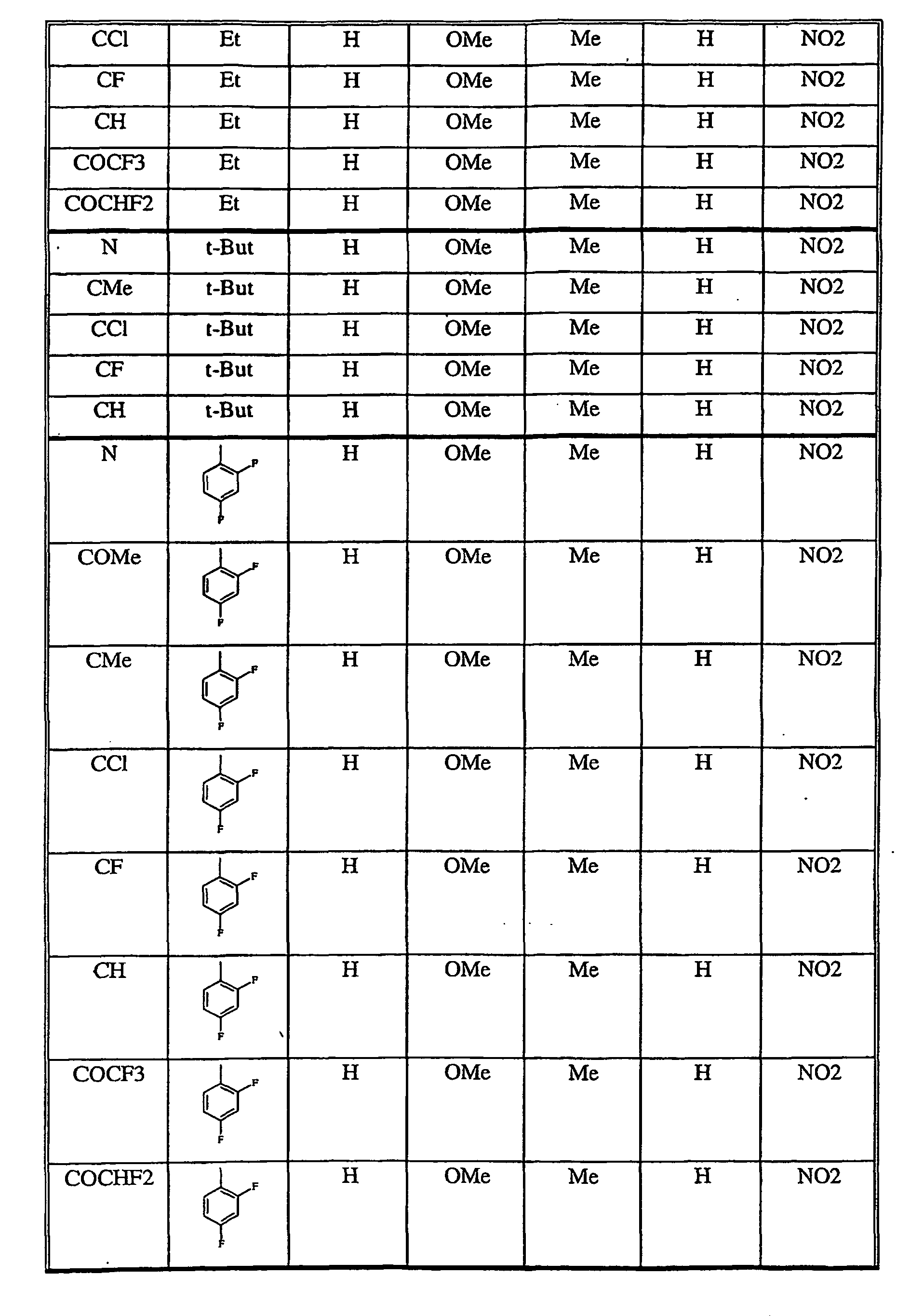

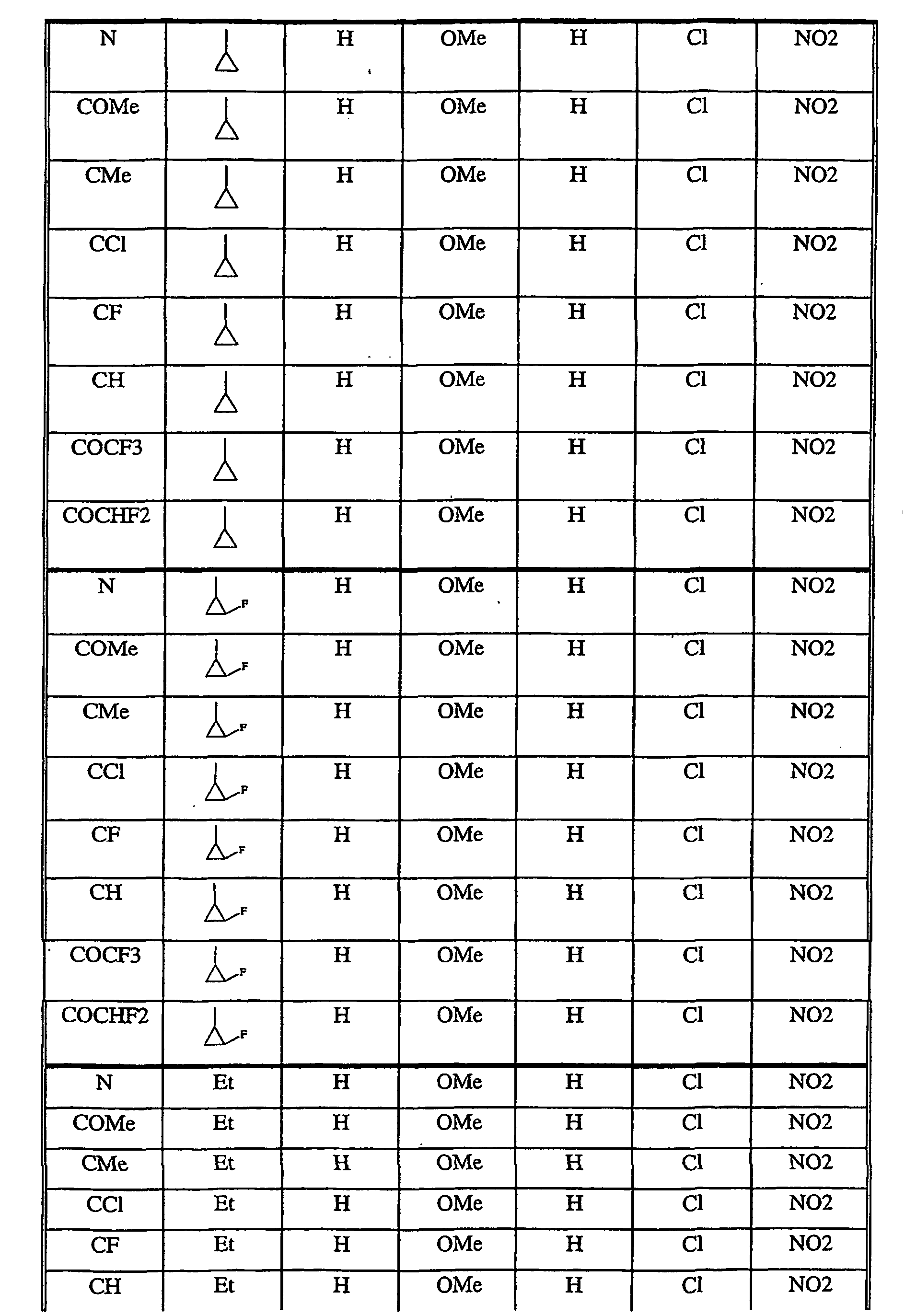

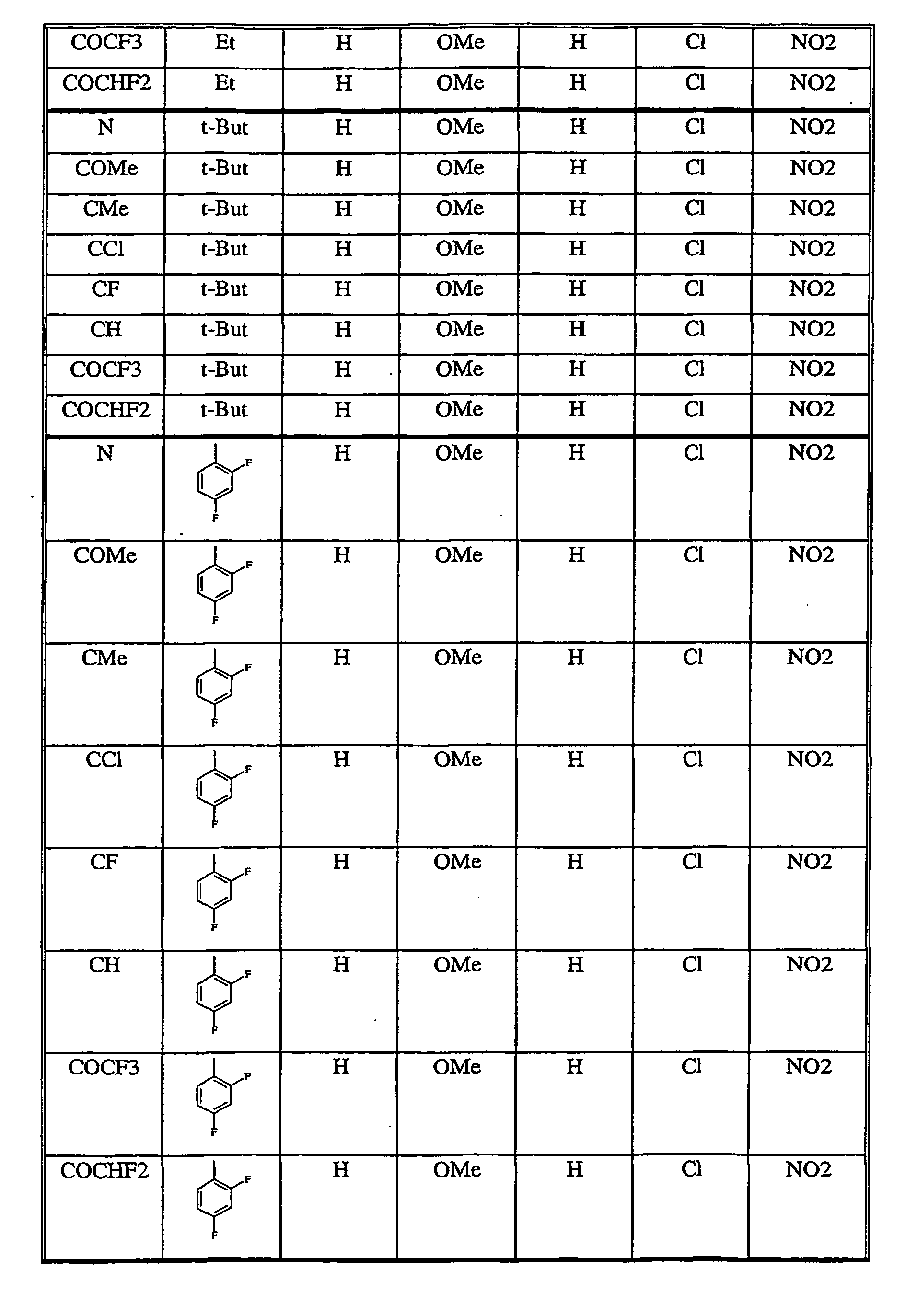

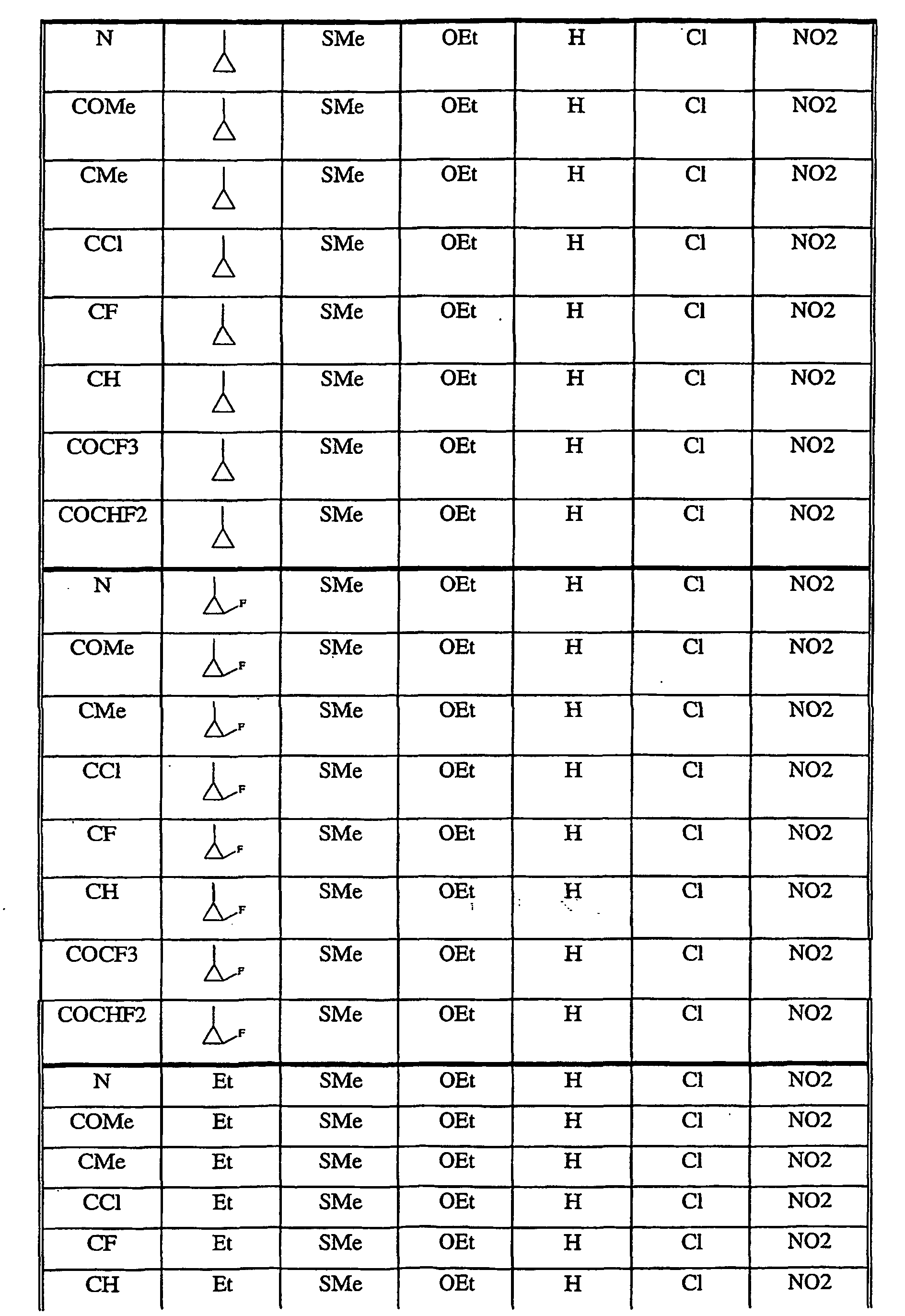

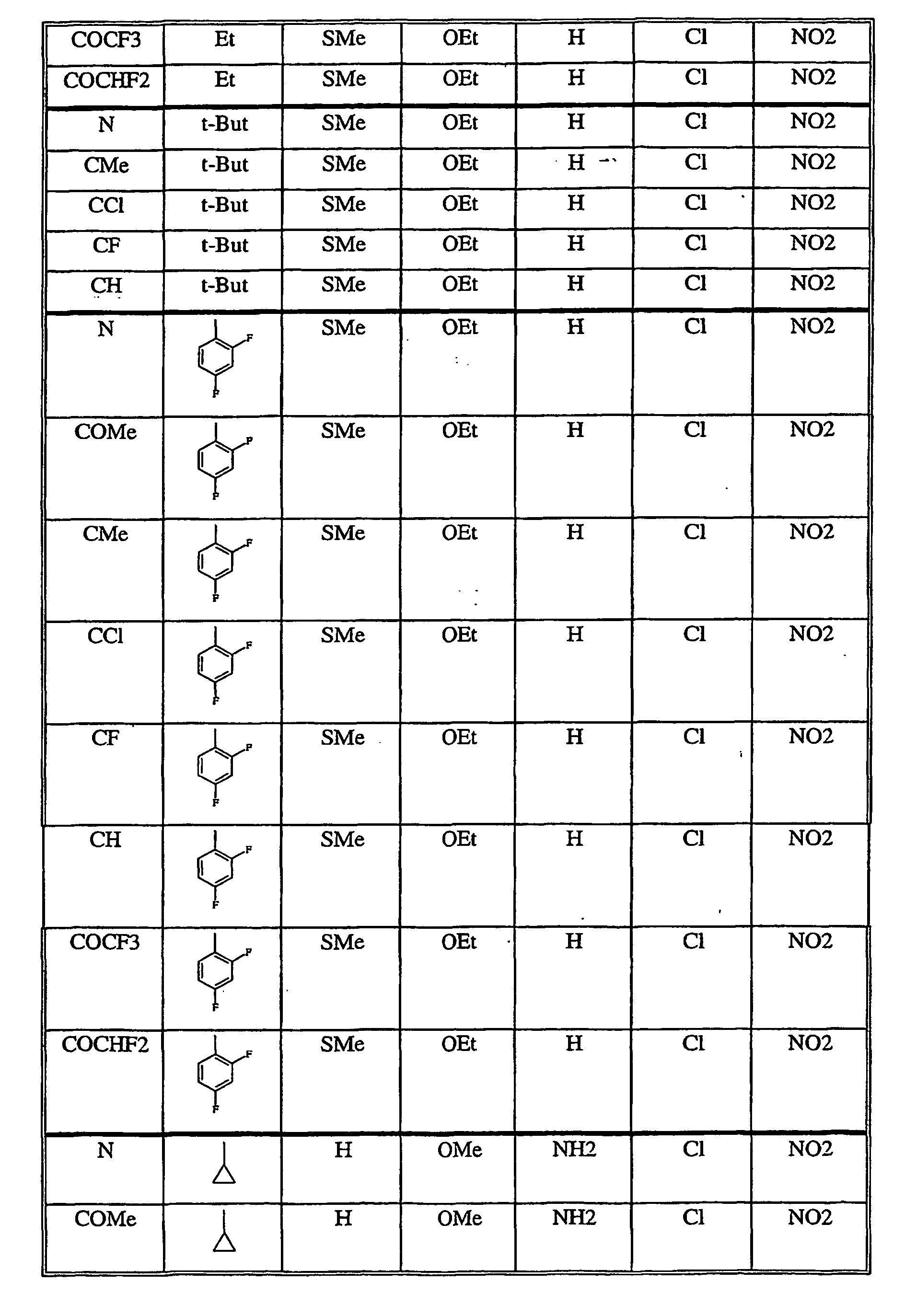

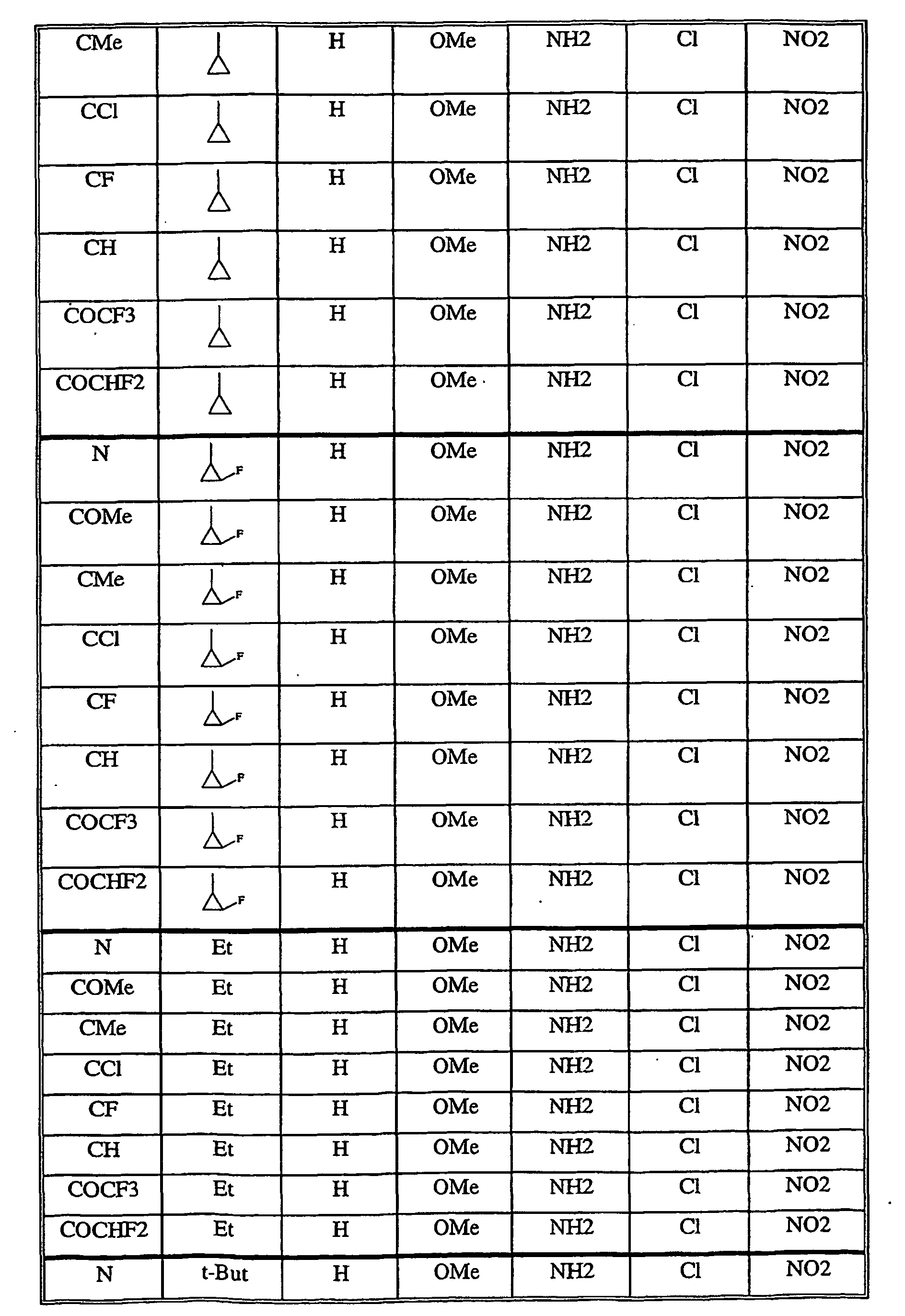

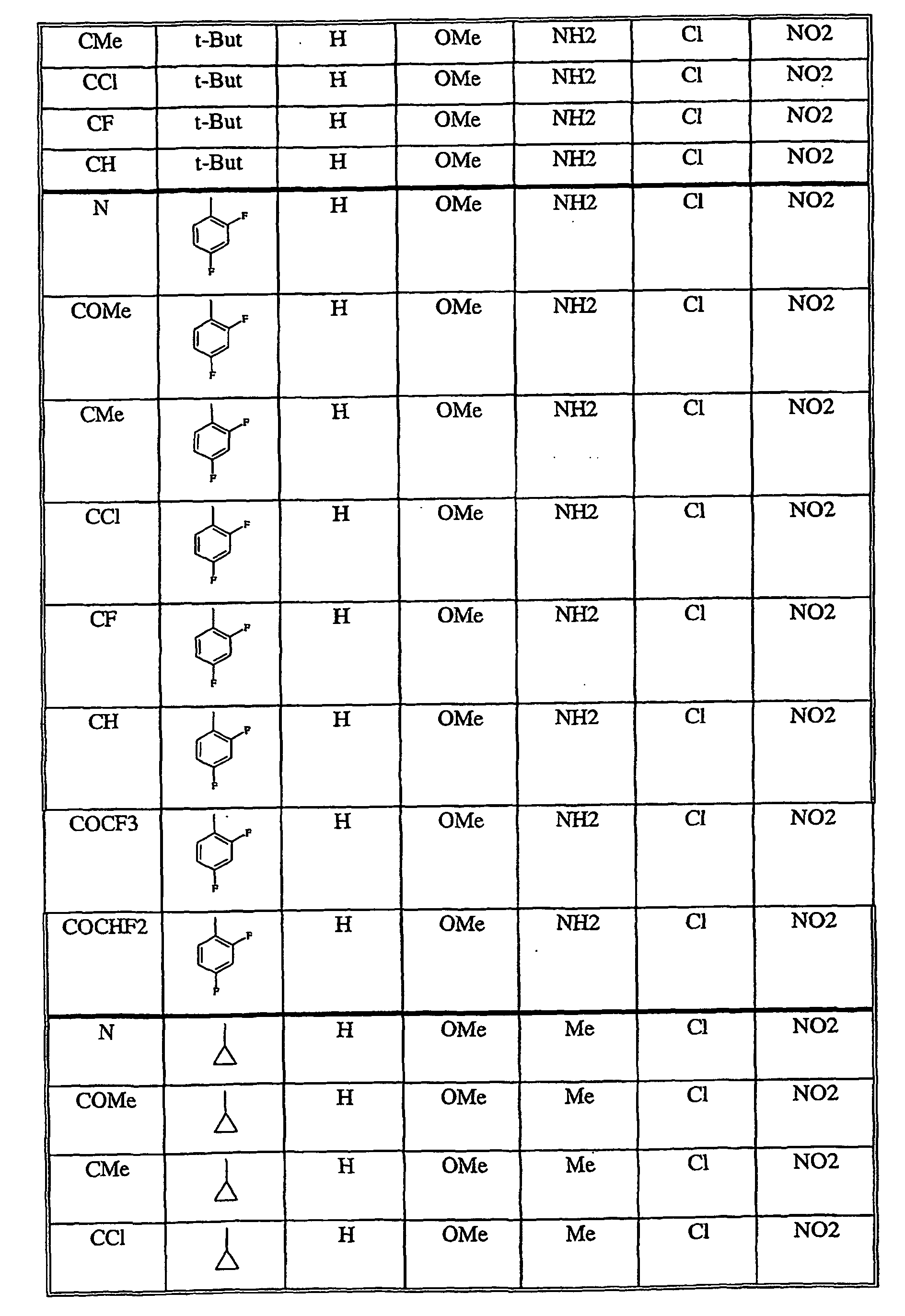

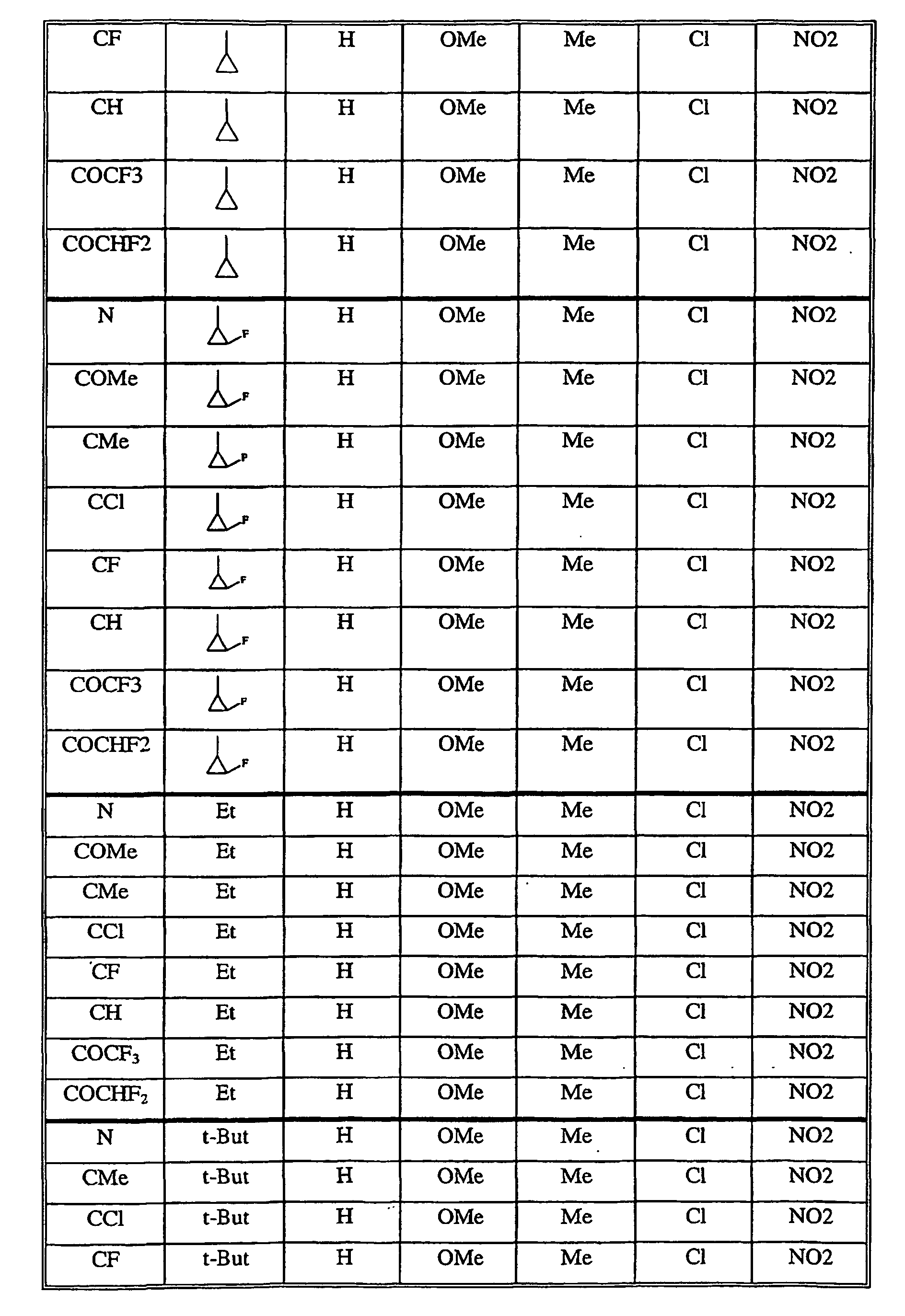

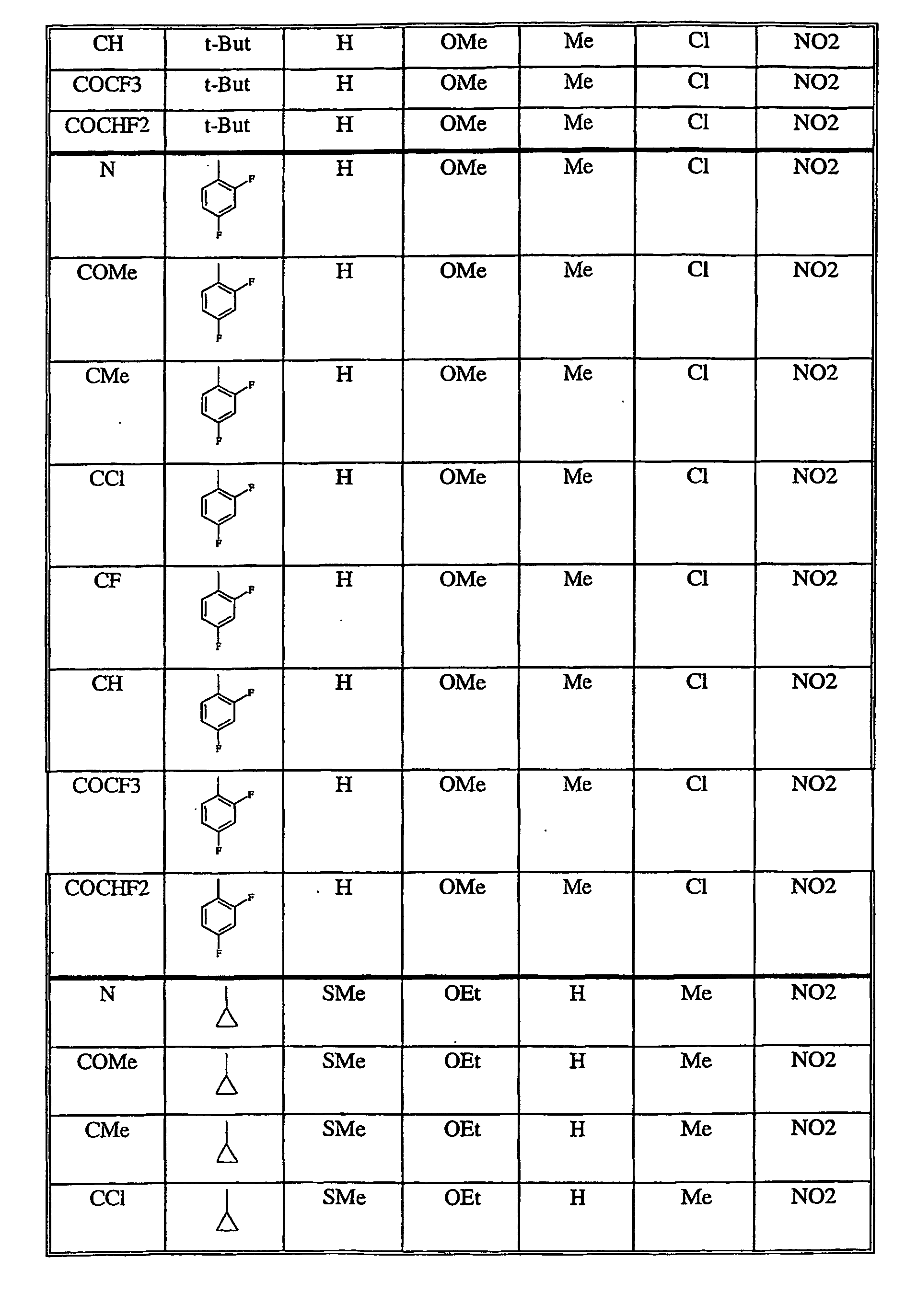

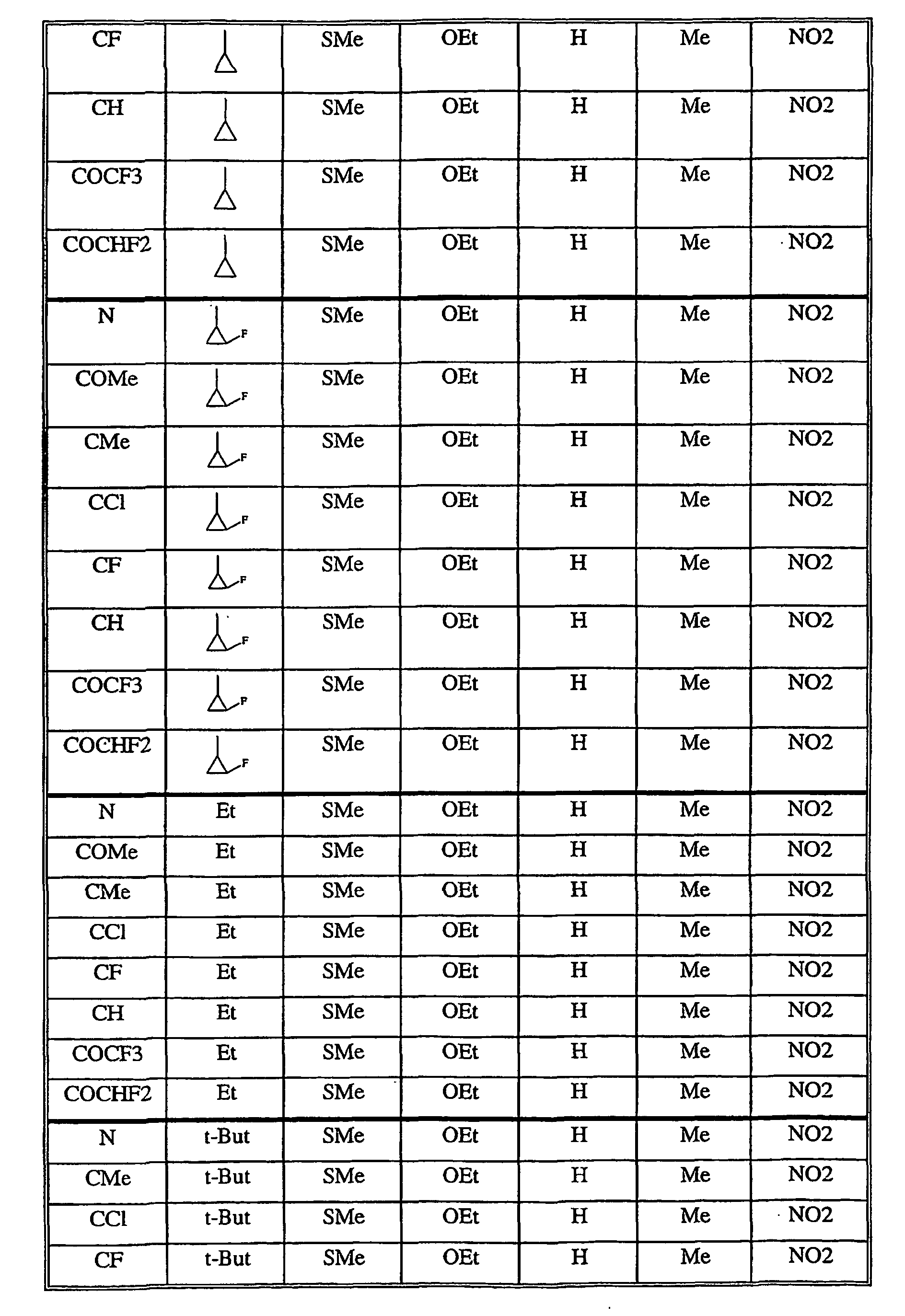

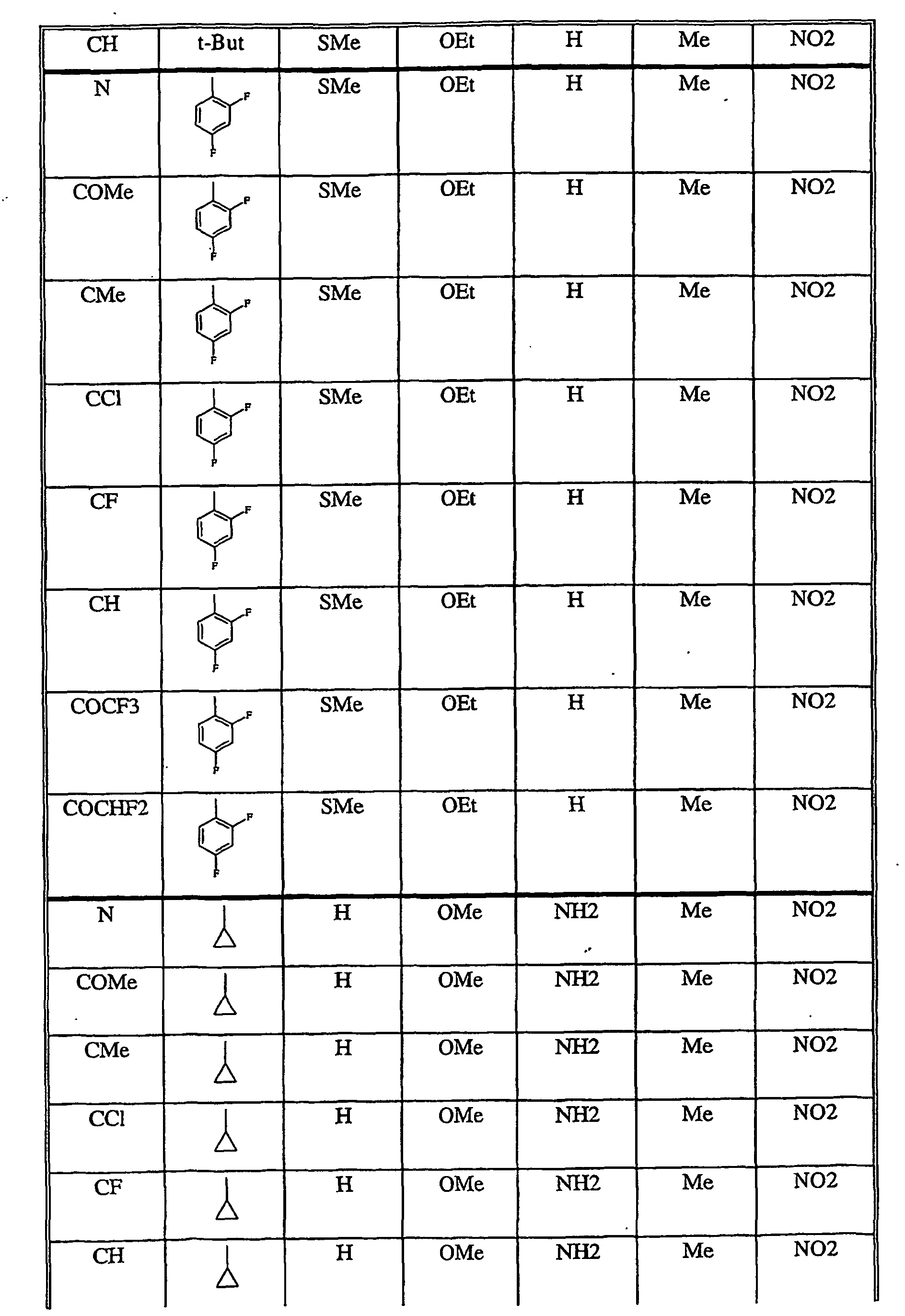

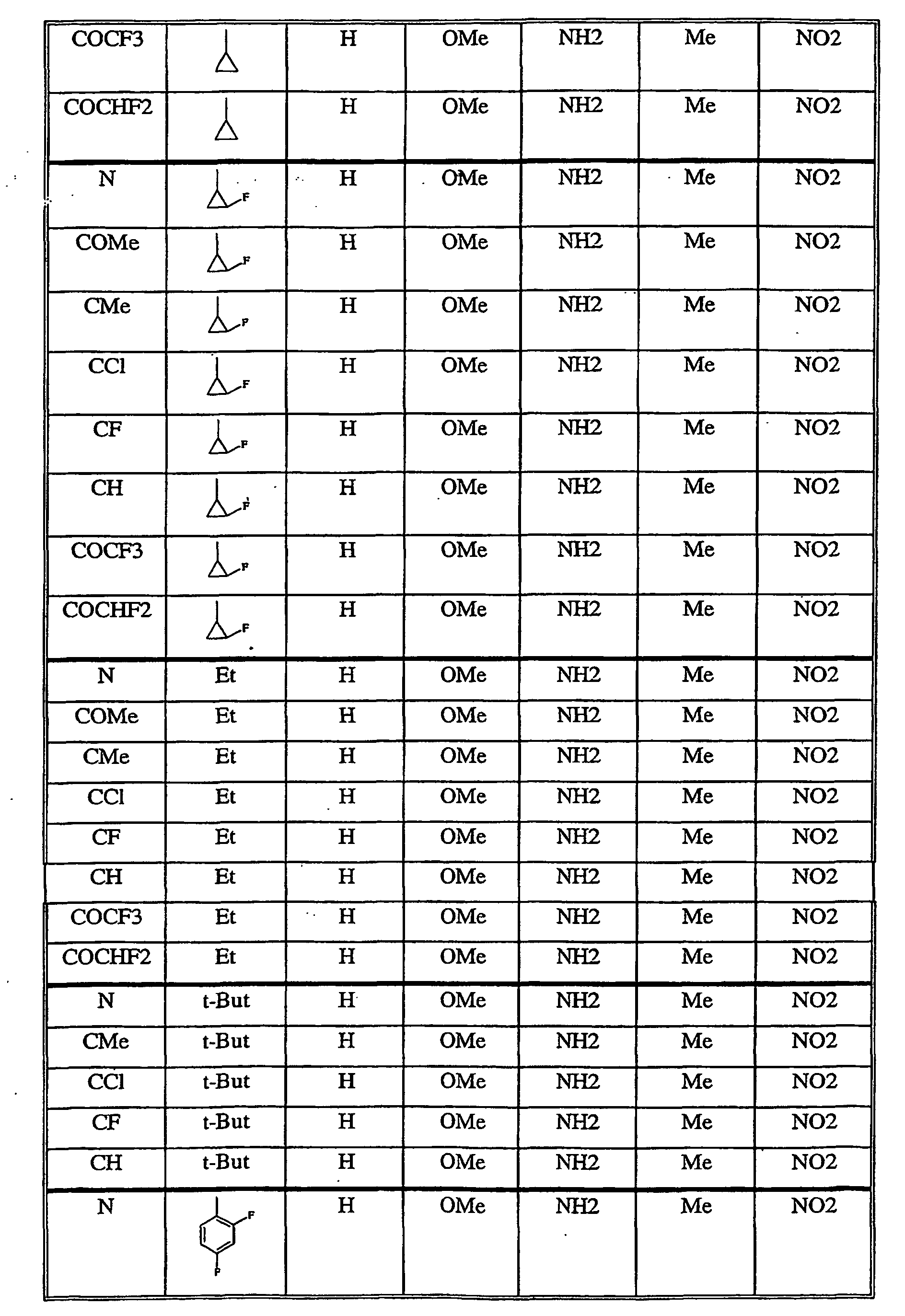

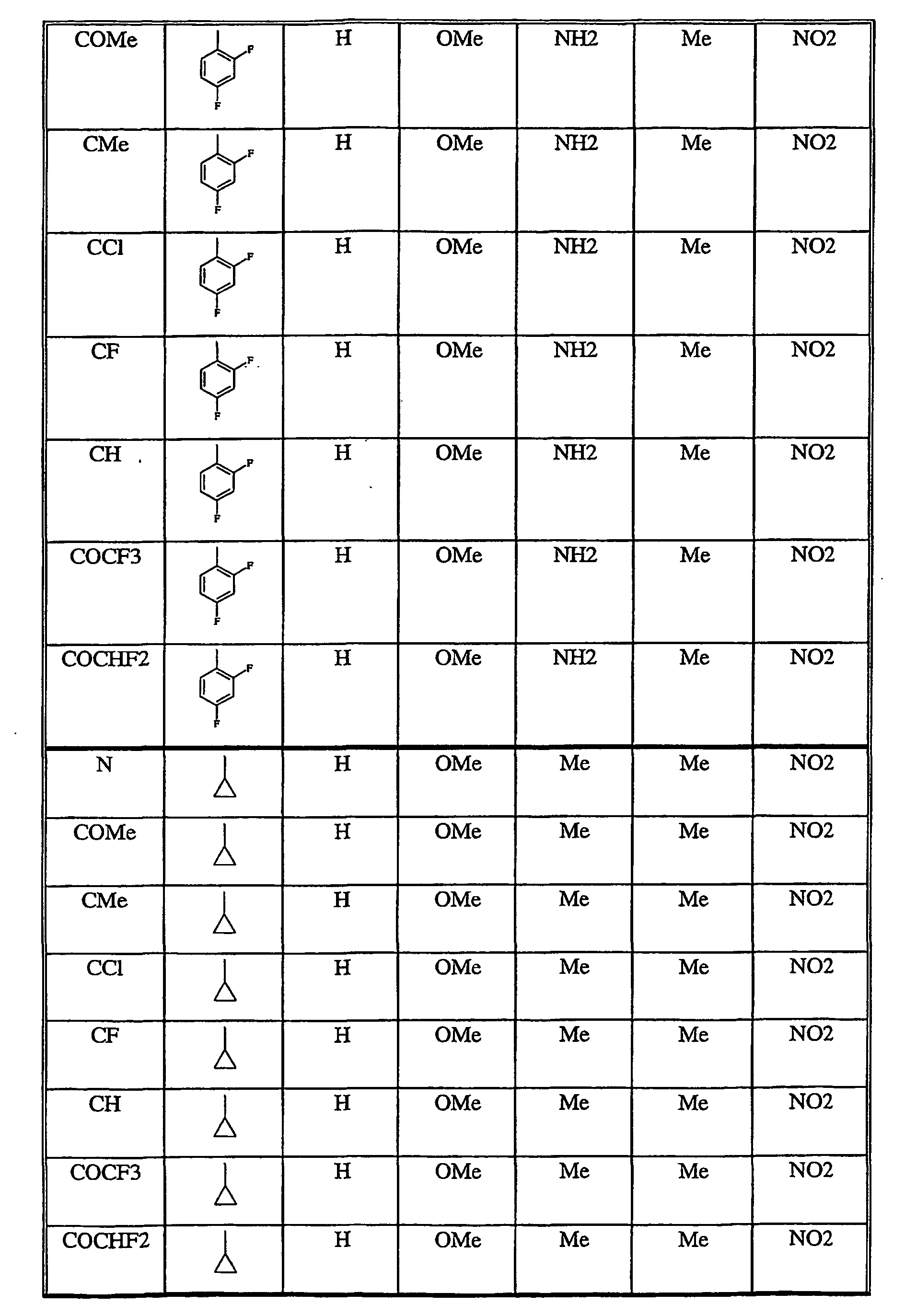

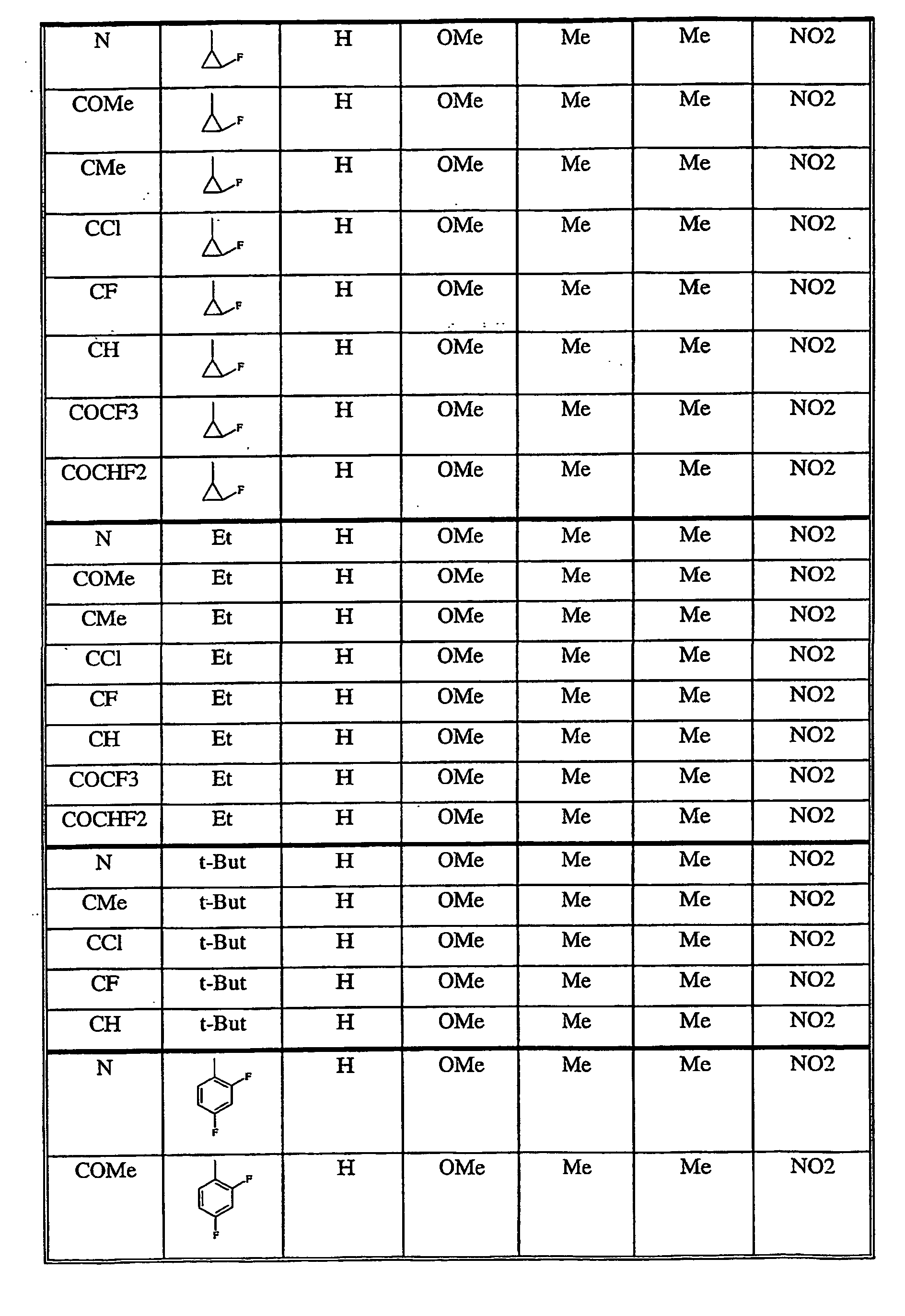

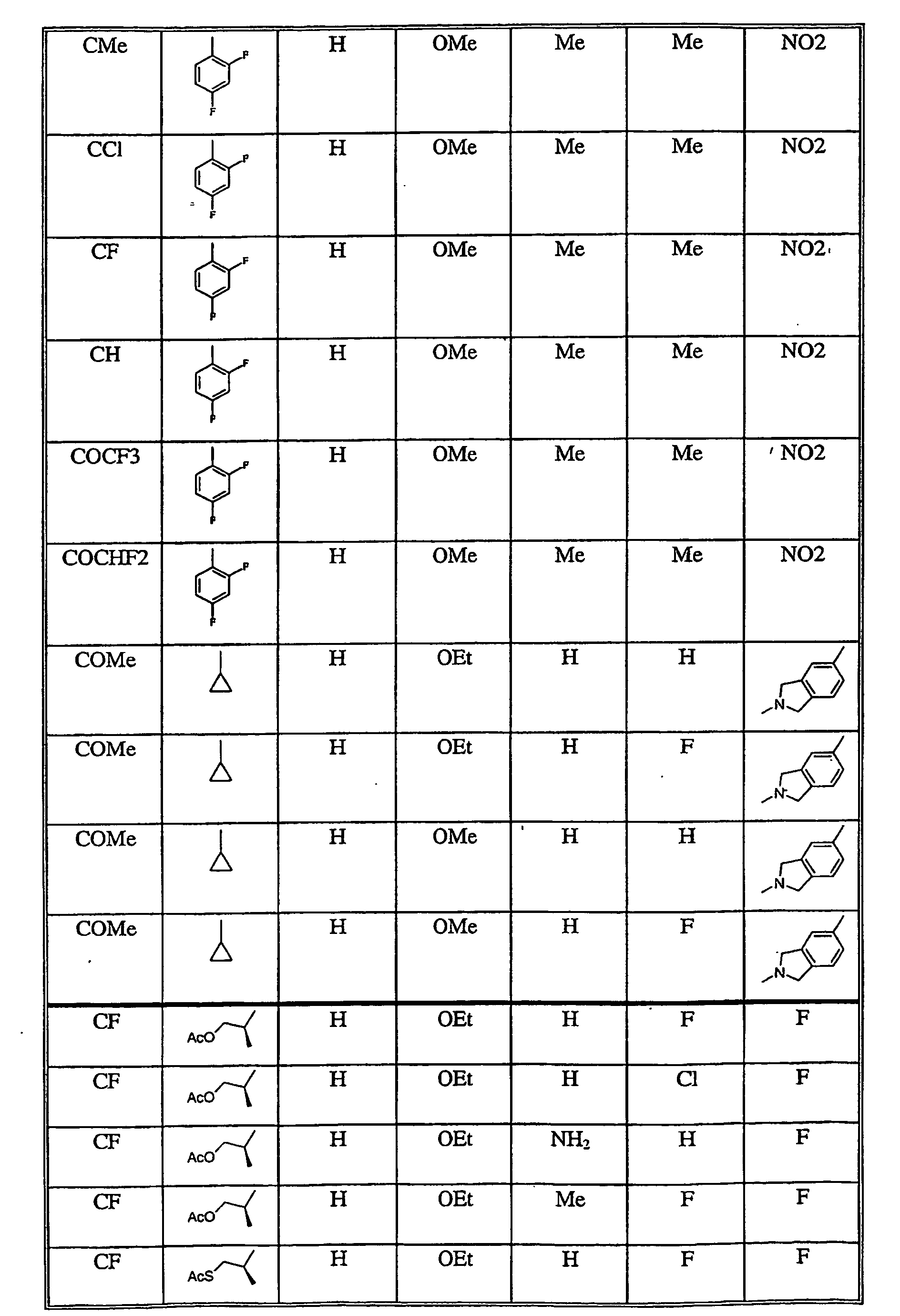

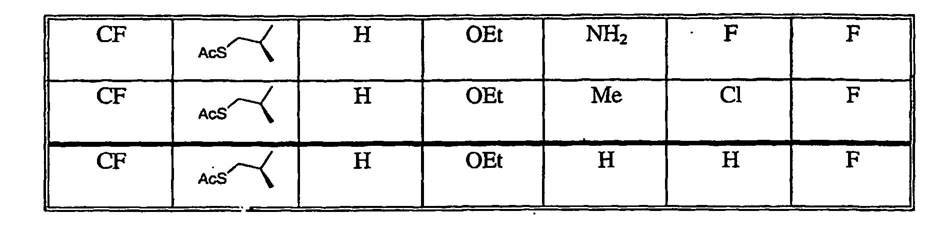

With regard to Formula (B) compounds, 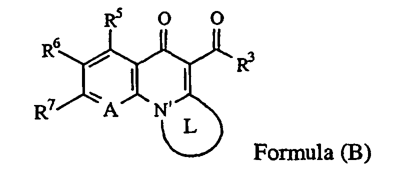 R¹ and R² of Formula (I) join to form ring L, which is a mono- or bicyclic heterocycle comprising N'.

Preferred compounds of Formula (B) made according to the present process are described in Table B.

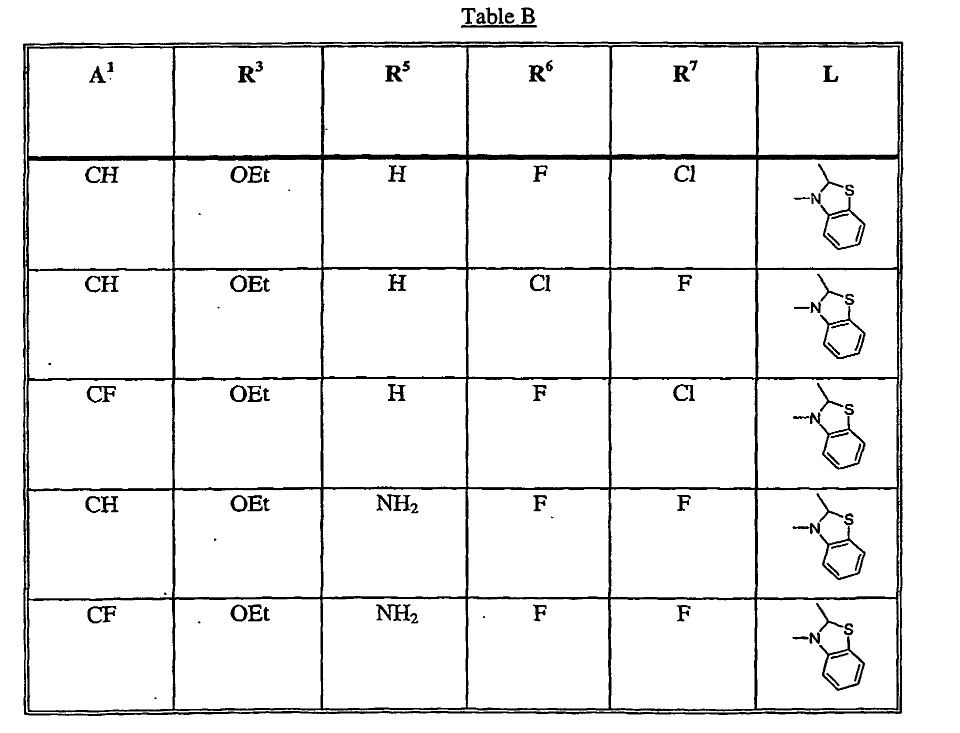

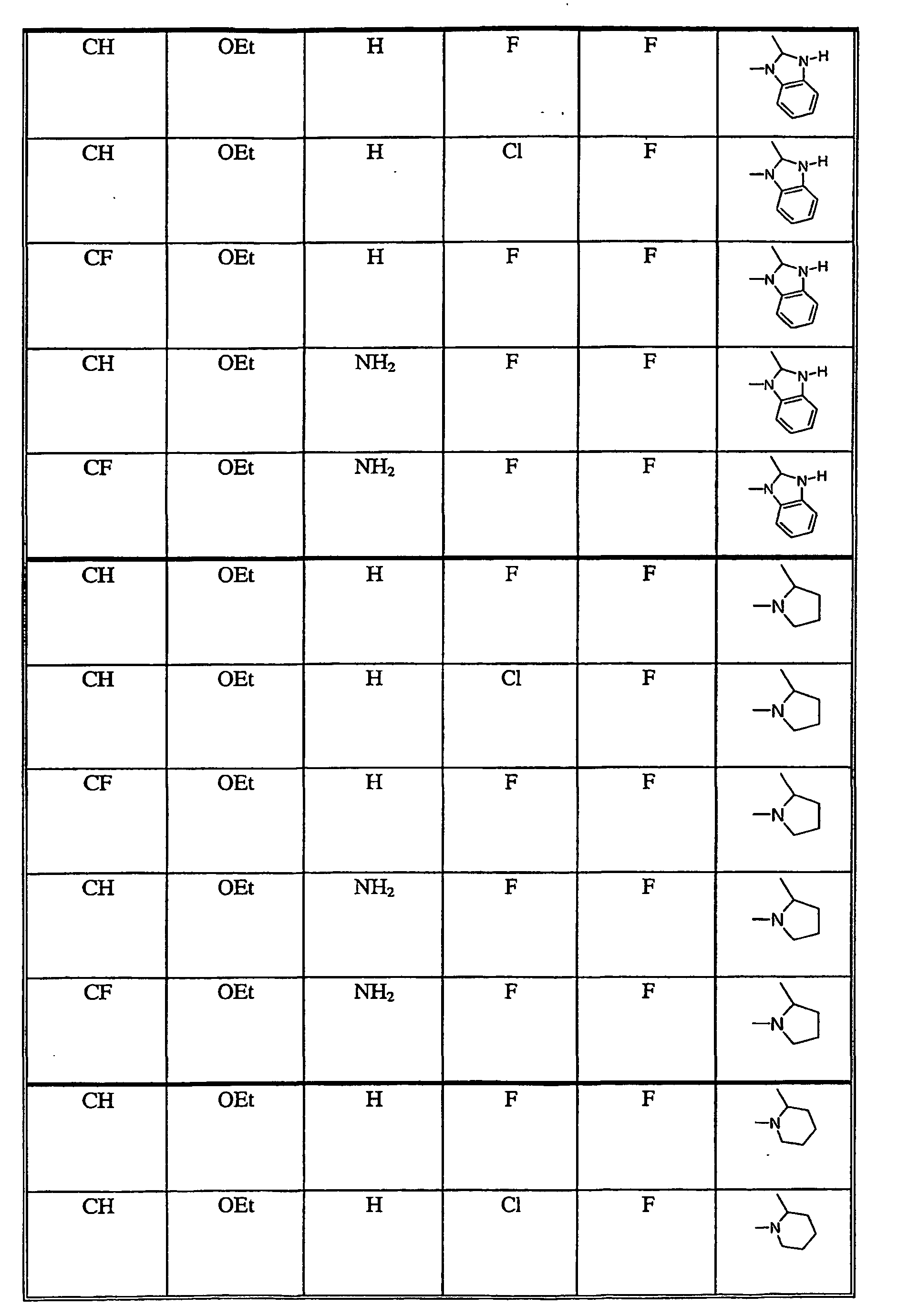

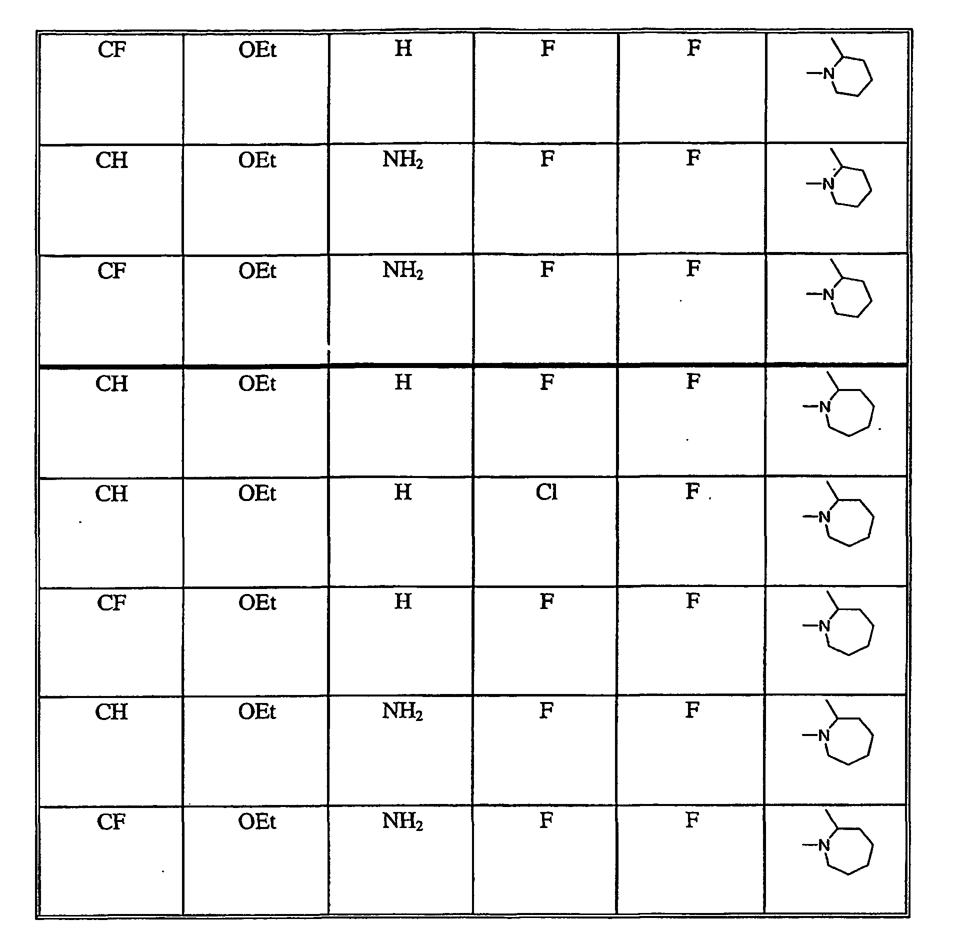

With regard to Formula (C) 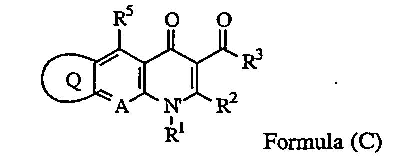 R⁶ and R⁷ of Formula (I) join to form ring Q, which is a 5- or 6-membered carbocyclic or heterocyclic ring.
Preferred compounds of Formula (C) made according to the present process are described in Table C.

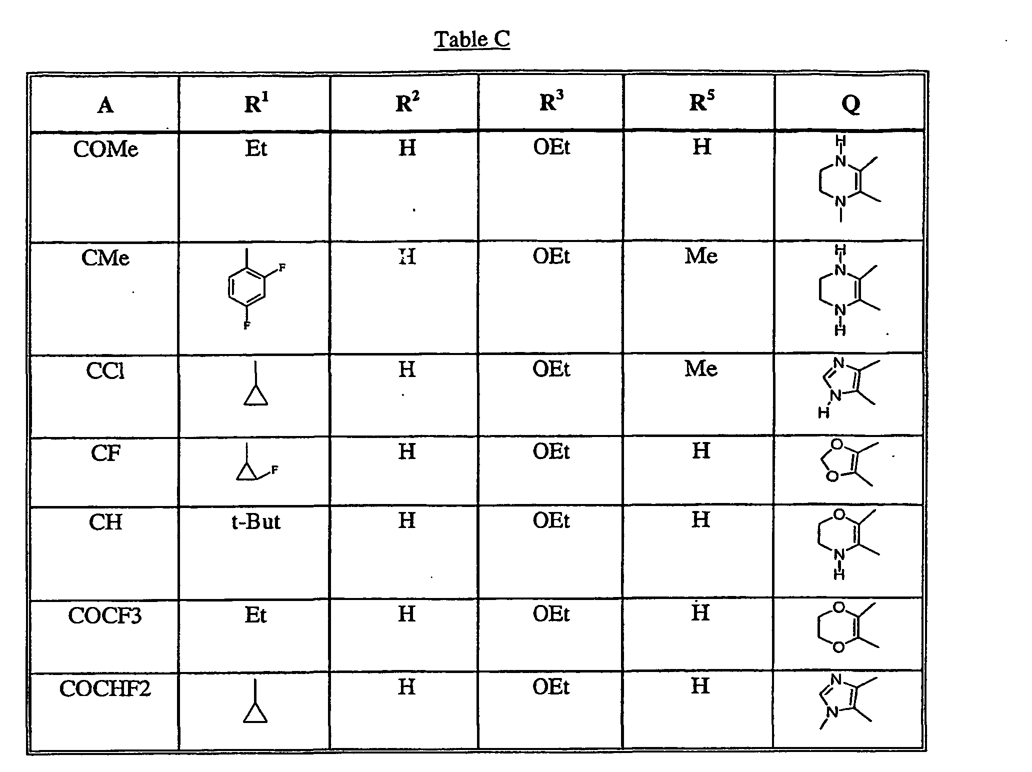

### III. Process Conditions:

The above subject invention process step utilizes a silylating agent that is an organosilicon reagent, which is defined above.

In the key process step, the molar ratio of the organosilicon reagent to reactant (i.e., compound of Formula (A)) is preferably from about 0.5:1 to about 12:1, more preferably from about 1:1 to about 4: 1. It will be recognized that these process conditions are merely preferred ranges and is it possible to use both lower and higher molar ratios and still benefit from the inventive process.

The subject invention process step is preferably carried out in an aprotic solvent or combination of solvents. Preferred solvents in which the process step is carried out include, but are not limited to, acetonitrile, N-methylpyrrolidinone (NMP), dimethylformide, N,N-dimethylacetamide, toluene, xylene, tetrahydrofuran, dioxane, 1,2-dimethoxyethane, diglyme; more preferred solvents include acetonitrile, toluene and NMP. Mixtures of one or more solvents may be utilized.

The temperature at which the subject process step is carried out is preferably from about - 50°C to about 250°C, more preferably from about -10 °C to about 160°C, more preferably still from about 20°C to about 140°C. The pressure at which the subject reaction step is carried is preferably from about 0.5 atm to about 50 atm, more preferably from about 0.8 atm to about 10 atm, more preferably still from about 1 atm to about 2 atm. Also preferred is that the process step be carried out at about ambient temperature and pressure, or at about reflux temperature and ambient pressure. Again, these process conditions are merely representative and should not be interpreted as in anyway limiting the processes claimed below.

### IV. Specific Synthetic Examples

The following are exemplary, but are not meant to be limiting, regarding variations of the subject invention process step.

### Example 1

Preparation of Ethyl-1-cyclopropyl-1,4-dihydro-8-methoxy-4-oxo-quinoline-3-carboxylate: 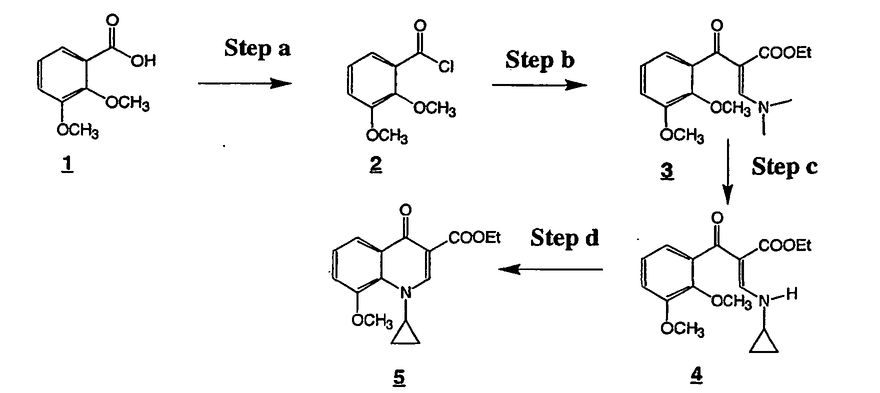
**Step a**: To a solution of 2,3-dimethoxybenzoic acid (20 g) **1** in dichloromethane (100 ml) is added oxalyl chloride (34.83 g) followed by 2 drops of anhydrous DMF. The mixture is stirred at room temperature for 1 hr, then heated to reflux for 4h. The solvent is removed by evaporation to give 2,3-dimethoxy benzoyl chloride **2**.
**Step b:** Product **2** is dissolved in anhydrous acetonitrile (20 mL) and is introduced to a stirred solution of triethyl amine (38.3 mL) and ethyl dimethylaminoacrylate (17.29 g) in acetonitrile (130 mL). The mixture is stirred at room temperature for 5 minutes, and then heated to reflux until the reaction goes to completion.
**Step c**: To the reaction mixture product of Step b, cyclopropylamine (19.01 mL) is added at ambient temperature and stirred until the reaction is complete. The solvent is evaporated, and the residue is diluted with ethyl acetate, washed with water and brine, dried over anhydrous sodium sulfate and concentrated under reduced pressure to furnish product **4**.
**Step d:** Product **4** is dissolved in anhydrous acetonitrile (150 mL). N,O-bis(trimethylsilyl)acetamide (115 g) is added. The solution is stirred at room temperature for 0.5 h and heated to reflux. Heating is continued until the reaction is complete. The reaction mixture is concentrated to an oily residue, poured into water, extracted with ethyl acetate, and the solvent removed to furnish product **5**.

### Example 2

Ethyl-1-cyclopropyl-1,4-dihydro-7,8-dimethoxy-4-oxoquinoline-3-carboxylate **10** is prepared by a process similar to that of Example 1 from commercially available 2,3,4-trimethoxy benzoic acid **6**.

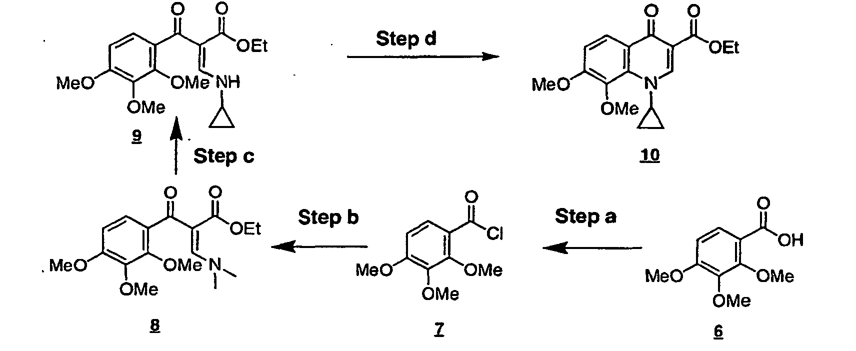

### Example 3

Ethyl-1-cyclopropyl-1,4-dihydro-8-methoxy-7-nitro-4-oxoquinoline-3-carboxylate **16** is prepared by a process similar to that of Example 1 from 4-nitro-3,4-dimethoxy benzoic acid **12**. The 4-nitro-3,4-dimethoxy benzoic acid is prepared from **11** according to literature procedures. (See, e.g., J. Org. Chem. **42,** (6) 1068-1070 (1977) and J. Heterocyclic Chemistry **33**, 1171 (1996).)

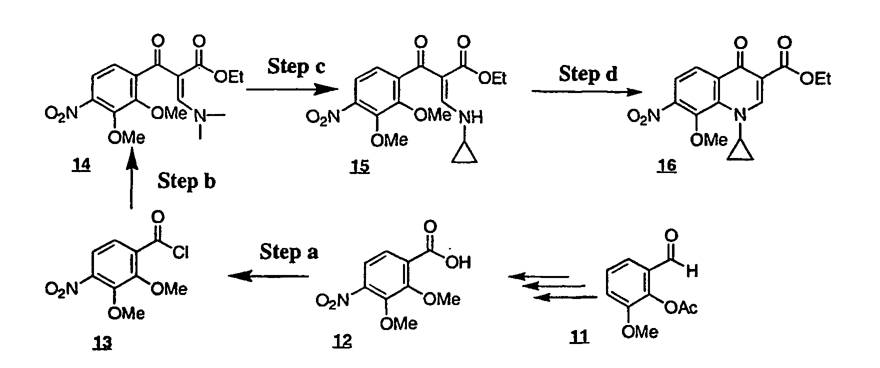

### Example 4

Ethyl-1-ethyl-1,4-dihydro-8-methoxy-8-bromoquinolone carboxylate **21** is prepared by a process similar to that of Example 1 from 4-bromo-3,4-dimethoxy benzoic acid **17****.** The 4-bromo-2,3-dimethoxy benzoic acid is prepared according to a literature method. (See e.g., J. Org. Chem. 42(6), 1068-70 (1977).)

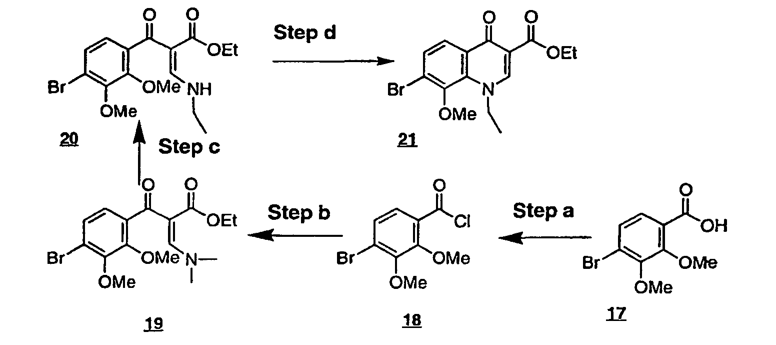

### Example 5:

Ethyl-1-cyclopropyl-1,4-dihydro-8-methoxy-7-fluoroquinolone carboxylate **27** is prepared by a process similar to that of Example 1 from 4-fluoro-3-methoxy-2-methylthio benzoic acid **23** or 4-fluoro-2,3-dimethoxybenzoic acid **62**. The starting benzoic acids are prepared from 4-fluoro-3-methoxy benzoic acid **22** by a procedure similar to that disclosed in the literature. (See, e.g., US Patent No. 5,334, 753, which is incorporated herein by reference.)

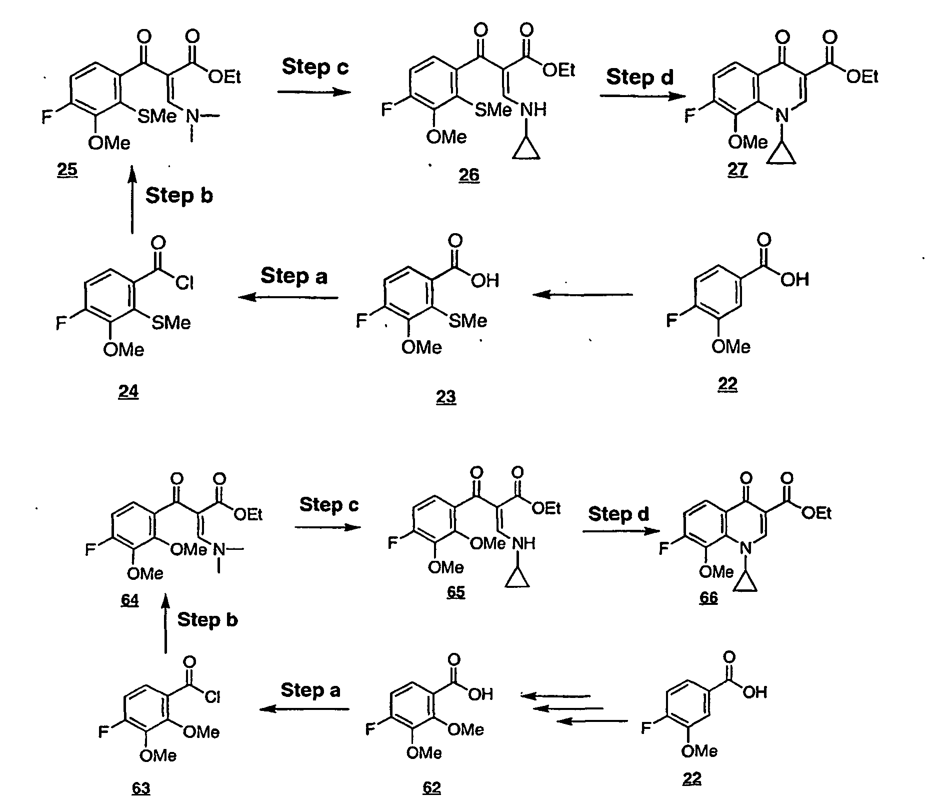

### Example 6

Ethyl-1,4-dihydro-1-(4-fluorophenyl)-8-fluoro-7-piperidinyl-1,4-dihydro-4-oxo-3-quiniline carboxylic acid **32** is prepared by a process similar to that of Example 1 from 3-fluoro-2-methoxy-4-piperidinyl benzoic acid **28.** The starting material **28** is prepared from 2,3,4-trifluorobenzoic acid by sequential displacement of ortho and para fluorine groups with methoxy and piperidinyl groups by a procedure similar to that reported in literature. (See e.g., Tetrahedron Letters **37** (36) 6439-6442 (1996).)

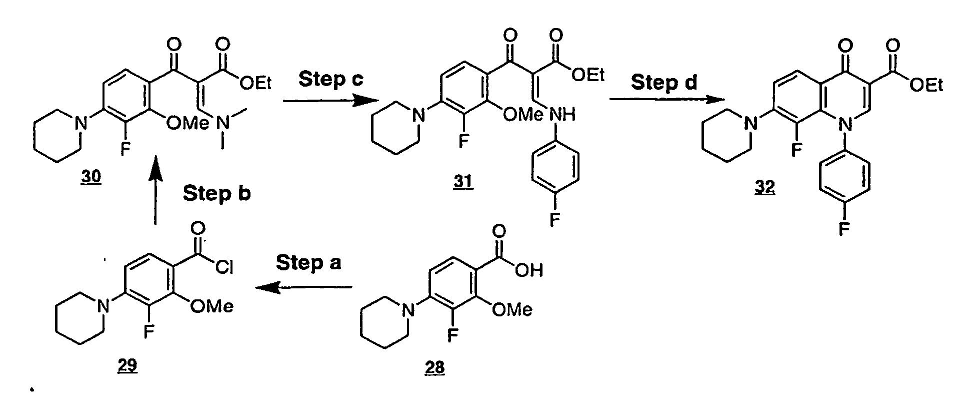

### Example 7

Ethyl-1-cyclopropyl-7-isoindoline-5-yl)-8-methoxy-1,4-dihydro-4-oxoquinoline-3-carboxylate **30** is prepared by a process similar to Step d of Example 1 from the corresponding acrylate derivative **29.** This acrylate derivative **29** is prepared by methods depicted in the literature. (See e.g., PCT Application No WO 97/29102.)

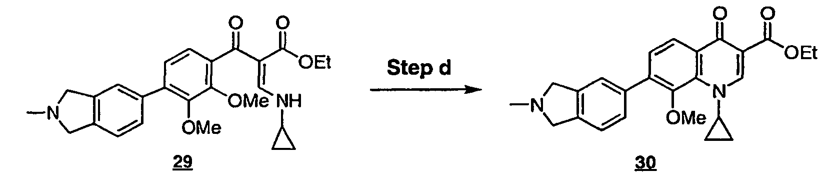

### Example 8

Ethyl 2-chloro-3-nitro-5,12-dihydro-5-oxobenzothiazolo[3,2-a]quinoline-6-carboxy-late **33** is prepared by a process similar to Step d of Example 1 from its cyclization precursor **32****.** **32** is prepared by reacting 2-chlorobenzothiazole **34** with ethyl-2-methoxy-4-chloro-5-nitrobenzoyl acetate **31** in the presence of sodium hydride.

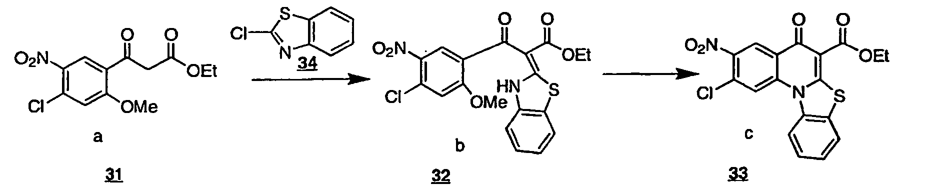

### Examples 9-11

Cyclization precursors **37****,** **40** and **43** are prepared by condensing ethyl 2-methoxy-4-chloro-5-fluoro benzoylacetate **35** with appropriate imino ethers **36****,** **39** and **42****,** respectively. The cyclization is carried out as described in Example 1 Step d to produce **38****,** **41** and **44****,** respectively.

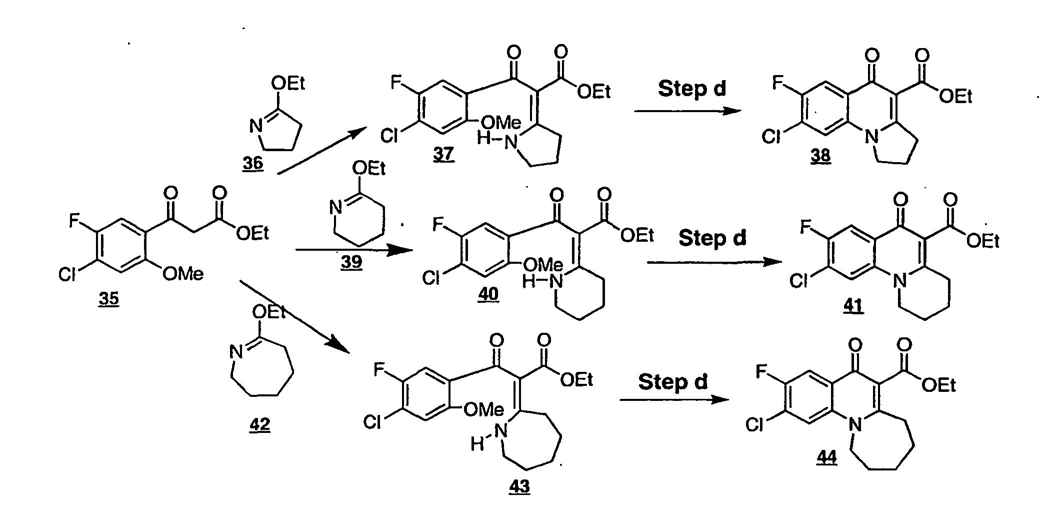

### Example 12

Ethyl 1,4-dihydro-4-oxo-6-nitro-7-chloro-1H-benz[d]imidazolo[2,3-a]quinoline-3-carboxylate **50** is prepared form cyclization precursor **49** as described in Step d of Example 1. The cyclization precursor **49** is prepared form 2-methoxy-4-chloro-5-nitrobenzoic acid **45** as shown below using similar procedures reported in literature. (See e.g., J. Med. Chem. **36** (11) 1580-1596 (1993).)

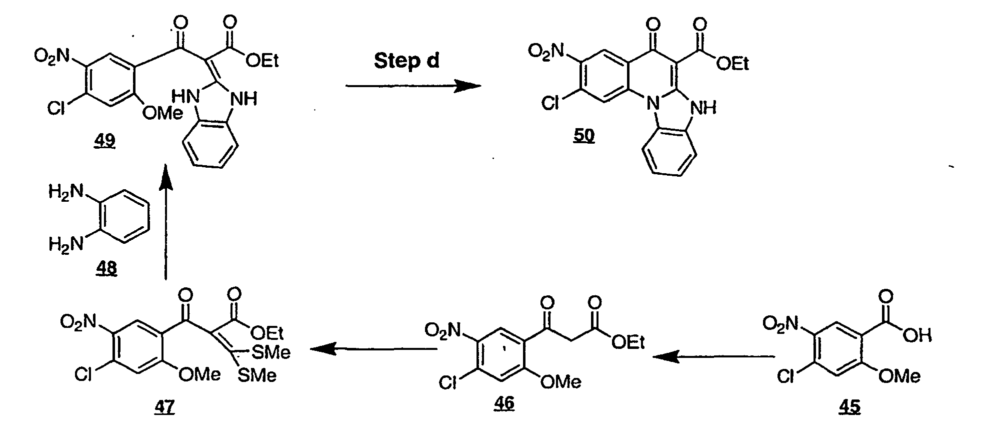

### Example 13

(-)-9,10-Difluoro-2,3-dihydro-3(S)-methyl-7-oxo-7H-pyrido[1,2,3-de]-1,4-benzox-aine-6-carboxylic acid **56** is prepared from (+)-Ethyl 2-(2-methoxy-3,4,5-trifluorobenzoyl)-3-[(1-acetoxyprop-2(S)-yl)amino]acrylate **54** by first doing Step d as in Example 1, followed by refluxing the resulting reaction mixture with a 10% aq. KOH solution. 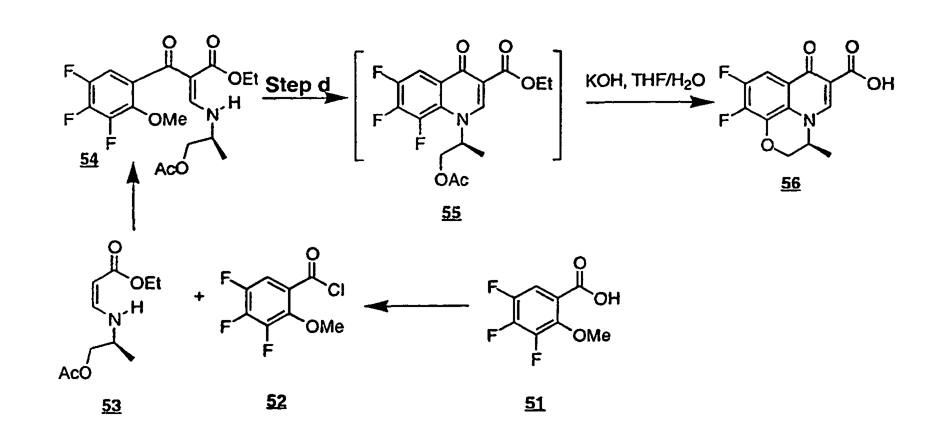 The cyclization precursor **54** is prepared from 2-methoxy-3,4,5-trifluorobenzoyl chloride **51** as shown by using literature procedures. (See E.g., in Heterocycles **45** (1), 137-145 (1997).)

### Example 14

Ethyl ester of oxolinic acid **61** is prepared by a process similar to that of Example 1 from 2-methoxy-4,5-(methylenedioxy)benzoic acid **57** as shown below. In Step c, ethylamine is used instead of cyclopropylamine.

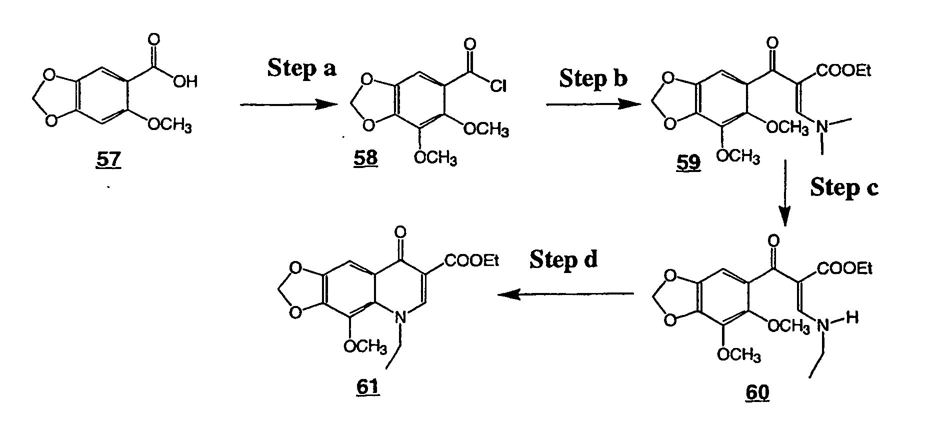

### Example 15:

Ethyl-1-cyclopropyl-1,4-dihydro-8-methoxy-7-fluoroquinolone carboxylate **66** is prepared by a process similar to that of Example 1 from 4-fluoro-3-methoxy-2-phenylthio benzoic acid **62****.** The benzoic acid **62** is prepared from 4-fluoro-3-methoxy benzoic acid **22** by a procedure similar to that disclosed in literature. (See, e.g., US Patent No. 5,334, 753, which is incorporated herein by reference.)

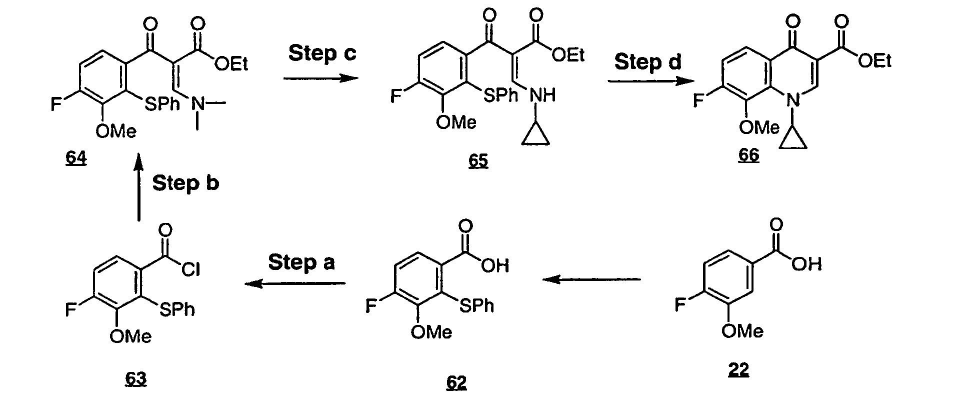

## Claims

1. A process for making a compound having a structure according to Formula (I), or an optical isoazer, diastereomer or enantiomer thereof, or a pharmaceutically-acceptable salt, hydrate, or biohydrolyzable ester, amide or imide thereof: 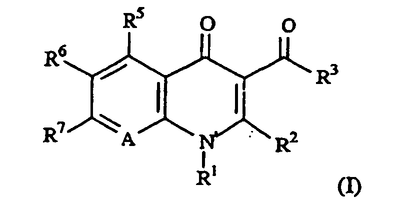 the process comprising reacting one or more organosilicon reagents with a compound having a structure according to Formula (A): 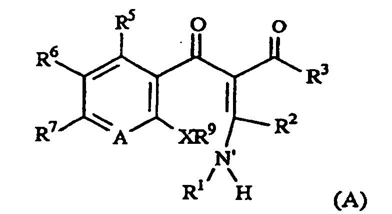 wherein with regard to Formula (I) and Formula (A):
(A)
(1) A is N or C-R⁸, where R⁸ is selected from hydrogen, C₁ to C₁₅ alkyl, aryl, halo, a heterocyclic ring, amino, C₁ to C₁₅ alkylamino, arylamino, C₁ to C₁₅ alkoxy, nitro, cyano, aryloxy, esters of hydroxy, C₁ to C₁₅ alkylthio, arylthio, aryloxy, esters of thio, C₁ to C₁₅ alkylsulfonyl, arylsulfonyl, C₁ to C₁₅ alkylphosphonyl, arylphosphonyl, C₁ to C₁₅ alkylacyl, arylacyl, and aryl esters and amides of carboxy;
(2) R⁷ is selected from hydrogen, C₁ to C₁₅ alkyl, aryl, a heterocyclic ring, amino, C₁ to C₁₅ alkylamino, arylamino, halo, nitro, cyano, C₁ to C₁₅ alkoxy, aryloxy, esters of hydroxy, C₁ to C₁₅ alkylthio, arylthio, esters of thio, C₁ to C₁₅ alkylsulfonyl, arylsulfonyl, C₁ to C₁₅ alkylphosphonyl, arylphosphonyl, C₁ to C₁₅ alkylacyl, arylacyl, and C₁ to C₁₅ alkyl and aryl esters and amides of carboxy;
(₃) R⁶ is selected from hydrogen, halo, C₁ to C₁₅ alkyl, aryl, a heterocyclic ring, amino, C₁ to C₁₅ alkylamino, arylamino, nitro, cyano, alkoxy, aryloxy, esters of hydroxy, C₁ to C₁₅ alkylthio, arylthio, esters of thio, C₁ to C₁₅ alkylsulfonyl, arylsulfonyl, C₁ to C₁₅ alkylphosphonyl, arylphosphonyl, C₁ to C₁₅ alkylacyl, arylacyl, and C₁ to C₁₅ alkyl and aryl esters and amides of carboxy;
(4) R⁵ is selected from hydrogen, C₁ to C₁₅ alkyl, aryl, cyano, a heterocyclic ring, amino, C₁ to C₁₅ alhylamino, arylamino, C₁ to C₁₅ alkylacyl, arylacyl, and aryl esters and amides of carboxy;
(5) R¹ is selected from a 3 to about 17 membered carbocyclic ring, a heterocyclic ring, C₁ to C₆ alkyl, C₁ to C₆ alkene, C₁ to C₆ alkyne and -CH(R¹⁰)(R¹¹) where R¹⁰ is selected from C₁ to C₆ alkyl and phenyl and R¹¹ is -CH₂Y(O=)CR¹² where R¹² is selected from C₁ to C₆ alkyl and phenyl and Y is selected from -NH-, -O- and -S-;
(6) R² is selected from hydrogen, C₁ to C₁₅ alkyl, aryl, a heterocyclic ring, C₁ to C₁₅ alkylthio and arylthio; and
(7) R³ is selected from hydrogen, C₁ to C₁₅ alkoxy, aryloxy, C₁ to C₁₅ alkyl and aryl; or
(B) R¹ and R² can join to form a 5- or 6-membered carbocyclic or heterocyclic ring, where A, R³, R⁵, R⁶, R⁷ and R⁸, if present, are as described in (A); or
(C) R⁶ and R⁷ can join to form a 5- or 6-membered carbocyclic or heterocyclic ring, where A, R¹, R², R³, R⁵ and R⁸, if present, are as described in (A);
and wherein with regard to Formula (A):
(D) X is selected from -O- and -S- and R⁹ is selected from C₁-C₁₀ alkyl, aryl and heteroaryl;
(E) Wherein the alkyl, heteroalkyl, cycloalkyl and heterocycloalkyl groups above may be optionally substituted with from 1 to 4 substituents, but not so as to form:
(1) Enols (OH attached to a carbon bearing a double bond);
(2) Amino groups attached to a carbon bearing a double bond (except for vinylogous amides);
(3) More than one hydroxy, amino, or amido attached to a single carbon (except where two nitrogen atoms are attached to a single carbon atom and all three atoms are member atoms within a heterocycloalkyl ring);
(4) Hydroxy, amino, or amido attached to a carbon that also has a heteroatom attached to it;
(5) Hydroxy, amino, or amido attached to a carbon that also has a halogen attached to it.

2. The process of Claim 1 wherein R⁹ in Formula (A) is selected from C₁ - C₄ alkyl and phenyl; preferably R⁹ is selected from unsubstituted C₁ - C₂ alkyl and unsubstituted phenyl

3. The process of Claim 1 wherein R⁹ in Formula (A) is selected from C₁ - C₄ alkoxy, thio (C₁ - C₄) alkyl, amyloxy and thioaryl; preferably R⁹ is selected from methoxy, ethoxy, propoxy, -SCH₃, -SCH₂CH₃, -SCH₂CH₂CH₃, phenoxy and -S(C₆H₅).

4. The process of any of Claims 1- 3 wherein none of R¹, R², R⁶, or R⁷ join together to form a ring fused to the A-containing or N'-containing rings.

5. The process of any of Claims 1 - 3 wherein R¹ and R² join to form a 5- or 6-membered carbocyclic or heterocyclic ring.

6. The process of any of Claims 1 - 3 wherein R⁶ and R⁷ join to form a 5- or 6-membered carbocyclic or heterocyclic ring.

7. The process according to claim 1
wherein with regard to Formula (I) and Formula (A):
(A)
(1) A is N or C-R⁸, where R⁸ is selected from hydrogen, halo, C₁-C₄ alkyl, phenyl, C₁-C₄ alkoxy, C₁-C₄ alkylthio, and phenoxy;
(2) R⁷ is selected from hydrogen, halo, nitro, C₁ to C₄ alkyl, unsubstituted amino, C₁ to C₄ mono- or di-alkylamino, phenyl, naphthyl, a heterocyclic ring having one ring with 5 or 6 ring atoms or two fused rings with 8-10 ring atoms, C₁ to C₄ alkylthio, phenylthio, phenoxy and C₁ to C₄ esters of hydroxy;
(3) R⁶ is selected from hydrogen, halo, nitro, C₁ to C₄ alkylamino, C₁ to C₄ alkoxy, and C₁ to C₄ esters of hydroxy;
(4) R⁵ is selected from hydrogen, halo, C₁ to C₄ alkyl, phenyl, amino and C₁ to C₄ mono- or diallcylamino;
(5) R¹ is selected from C₁-C₄ alkyl, C₃-C₆ cycloalkyl and aryl.
(6) R² is selected from hydrogen, C₁-C₄ alkyl, C₁-C₄ alkylthio and phenyl; and
(7) R³ is selected from hydrogen, about C₁-C₄ alkoxy and phenoxy.

8. The process of any of Claims 1 - 7 wherein the molar ratio of the organosilicon reagent to the compound of Formula (A) is from 0.5:1 to 12:1, more preferably from 1:1 to 4:1.

9. The process of any of Claims 1 - 8
wherein:
(A) the organosilicon reagent is selected from the group consisting of chlorotrimethylsilane, N,O-bis(trimethylsilyl)acetamide, N,O-bis(trimethylsilyl)trifluoroacetamide, 1,3-bis(trimethylsilyl)urea, 1,1,1,3,3,3-hexamethyldisilazane, N-methyl-N-trimethylsilyltrifluoroacetamide, 1-trimethylsilylimidazole, trimethylsilyl trifluoromethanesulfonate, tert-butyldimethylchlorosilane, 1-(tert-butyldimethylsilyl)imidazole, ethyl(trimethylsilyl)acetate, N-tert-butyldimethyl-N-methyltrifluoroacetamide, tertbutyldimethylsilyl trifluoromethanesulfonate, tert-butyldiphenylchlorosilane, tert-butyl-methoxyphenylbromosilane, dimethylphenylchlomsilane, triethylchlorosilane, triethylsilyl trifluoromethane-sulfonate, and triphenylchlorosilane, and mixtures thereof; preferably the organosilicon reagent is selected from N,O-bis(trimethylsilyl)acetamide, N,O-bis(trimethylsilyl)trifluoroacetamide, N-methyl-N-trimethylsilyltrifluoroacetamide and tert-butyldiphenylchlorosilane, and mixtures thereof;
(B) the organosilicon reagent and the compound of Formula (A) are reacted at a temperature of from -50°C to 250°C; preferably from -10°C to 160°C; more preferably from 20°C to 140°C; and
(C) the organosilicon reagent and the compound of Formula (A) are reacted at a pressure of from 0.5 atm to 50 atm; preferably 0.8 atm to 10 atm; more preferably from 1 atm to 2 atm.

10. The process of any of Claims 1- 9 wherein the organosilicon reagent and the compound of Formula (A) are reacted in a solvent selected from acetonitrile, N-methylpyrrolidinone (NMP), dimethylformide, N,N-dimethylacetamide, toluene, xylene, tetrahydrofuran, dioxane, 1,2-dimethoxyethane, diglyme; more preferred solvents include acetonitrile, toluene, NMP, and mixtures of any of the foregoing.

## Patentansprüche

1. Verfahren für die Herstellung einer Verbindung mit einer Struktur nach Formel (I) oder eines optischen Isomers, Diastereomers oder Enantiomers davon oder eines pharmazeutisch akzeptablen Salzes, Hydrats oder biologisch hydrolysierbaren Esters, Amids oder Imids davon: 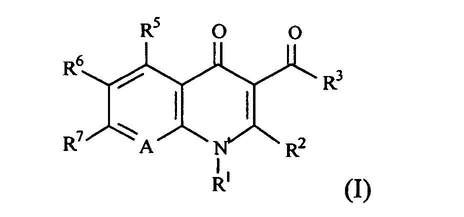 wobei das Verfahren das Umsetzen eines oder mehrerer Organosilicium-Reagentien mit einer Verbindung umfasst, die eine Struktur nach Formel (A) hat: 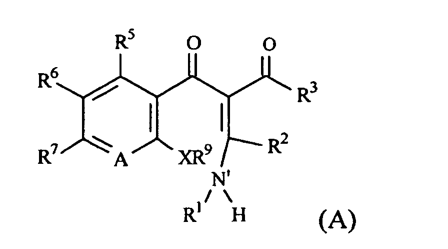 worin hinsichtlich Formel (I) und Formel (A):
(A)
(1) A N oder C-R⁸ ist, wo R⁸ ausgewählt ist aus Wasserstoff, C₁- bis C₁₅-Alkyl, Aryl, Halo, einem heterocyclischen Ring, Amino, C₁-bis C₁₅-Alkylamino, Arylamino, C₁- bis C₁₅-Alkoxy, Nitro, Cyano, Aryloxy, Estern von Hydroxy, C₁- bis C₁₅-Alkylthio, Arylthio, Aryloxy, Estern von Thio, C₁- bis C₁₅-Alkylsulfonyl, Arylsulfonyl, C₁- bis C₁₅-Alkylphosphonyl, Arylphosphonyl, C₁- bis C₁₅-Alkylacyl, Arylacyl und Arylestern und Amiden von Carboxy;
(2) R⁷ ausgewählt ist aus Wasserstoff, C₁- bis C₁₅-Alkyl, Aryl, einem heterocyclischen Ring, Amino, C₁- bis C₁₅-Alkylamino, Arylamino, Halo, Nitro, Cyano, C₁- bis C₁₅-Alkoxy, Aryloxy, Estern von Hydroxy, C₁- bis C₁₅-Alkylthio, Arylthio, Estern von Thio, C₁- bis C₁₅-Alkylsulfonyl, Arylsulfonyl, C₁- bis C₁₅-Alkylphosphonyl, Arylphosphonyl, C₁- bis C₁₅-Alkylacyl, Arylacyl und C₁- bis C₁₅-Alkyl- und Arylestern und Amiden von Carboxy;
(3) R⁶ ausgewählt ist aus Wasserstoff, Halo, C₁- bis C₁₅-Alkyl, Aryl, einem heterocyclischen Ring, Amino, C₁- bis C₁₅-Alkylamino, Arylamino, Nitro, Cyano, Alkoxy, Aryloxy, Estern von Hydroxy, C₁- bis C₁₅-Alkylthio, Arylthio, Estern von Thio, C₁- bis C₁₅-Alkylsulfonyl, Arylsulfonyl, C₁- bis C₁₅-Alkylphosphonyl, Arylphosphonyl, C₁- bis C₁₅-Alkylacyl, Arylacyl und C₁- bis C₁₅-Alkyl- und Arylestern und Amiden von Carboxy;
(4) R⁵ ausgewählt ist aus Wasserstoff, C₁- bis C₁₅-Alkyl, Aryl, Cyano, einem heterocyclischen Ring, Amino, C₁- bis C₁₅-Alkylamino, Arylamino, C₁- bis C₁₅-Alkylacyl, Arylacyl und Arylestern und Amiden von Carboxy;
(5) R¹ ausgewählt ist aus einem 3- bis etwa 17-gliedrigen carbocyclischen Ring, einem heterocyclischen Ring, C₁- bis C₆-Alkyl, C₁-bis C₆-Alken, C₁- bis C₆-Alkin und -CH(R¹⁰)(R¹¹), wo R¹⁰ ausgewählt ist aus C₁- bis C₆-Alkyl und Phenyl und R¹¹ -CH₂Y(O=)CR¹² ist wo R¹² ausgewählt ist aus C₁- bis C₆-Alkyl und Phenyl und Y ausgewählt ist aus -NH-, -O- und -S-;
(6) R² ausgewählt ist aus Wasserstoff, C₁- bis C₁₅-Alkyl, Aryl, einem heterocyclischen Ring, C₁- bis C₁₅-Alkylthio und Arylthio und
(7) R³ ausgewählt ist aus Wasserstoff, C₁- bis C₁₅-Alkoxy, Aryloxy, C₁- bis C₁₅-Alkyl und Aryl oder
(B) R¹ und R² sich verbinden können, um einen 5- oder 6-gliedrigen carbocyclischen oder heterocyclischen Ring zu bilden, wo A, R³, R⁵, R⁶, R⁷ und R⁸, wenn vorhanden, wie in (A) beschrieben sind; oder
(C) R⁶ und R⁷ sich verbinden können, um einen 5- oder 6-gliedrigen carbocyclischen oder heterocyclischen Ring zu bilden, wo A, R¹, R², R³, R⁵ und R⁸, wenn vorhanden, wie in (A) beschrieben sind;
und worin hinsichtlich Formel (A):
(D) X ausgewählt ist aus -O- und -S- und R⁹ ausgewählt ist aus C₁-C₁₀-Alkyl, Aryl und Heteroaryl;
(E) worin die obigen Alkyl-, Heteroalkyl-, Cycloalkyl- und Heterocycloalkylgruppen wahlweise mit von 1 bis 4 Substituenten substituiert sein können, aber nicht so, dass sie bilden:
(1) Enole (OH, gebunden an einen Kohlenstoff, der eine Doppelbindung trägt),
(2) Aminogruppen, gebunden an einen Kohlenstoff, der eine Doppelbindung trägt (außer vinyloge Amide);
(3) mehr als ein Hydroxy, Amino oder Amido, die an einen einzelnen Kohlenstoff gebunden sind (außer, wo zwei Stickstoffatome an ein einzelnes Kohlenstoffatom gebunden sind und alle drei Atome Atomglieder innerhalb eines Heterocycloalkylrings sind);
(4) Hydroxy, Amino oder Amido, gebunden an einen Kohlenstoff, an den auch ein Heteroatom gebunden ist;
(5) Hydroxy, Amino oder Amido, gebunden an einen Kohlenstoff, an den auch ein Halogen gebunden ist.

2. Verfahren nach Anspruch 1, worin R⁹ in Formel (A) ausgewählt ist aus C₁-C₄-Alkyl und Phenyl; R⁹ vorzugsweise ausgewählt ist aus nicht-substituiertem. C₁-C₂-Alkyl und nicht-substituiertem Phenyl

3. Verfahren nach Anspruch 1, worin R⁹ in Formel (A) ausgewählt ist aus C₁-C₄-Alkoxy, Thio(C₁-C₄)alkyl, Amyloxy und Thioaryl; R⁹ vorzugsweise ausgewählt ist aus Methoxy, Ethoxy, Propoxy, -SCH₃, -SCH₂CH₃, -SCH₂CH₂CH₃, Phenoxy und -S(C₆H₅).

4. Verfahren nach einem der Ansprüche 1-3, worin weder R¹, R², R⁶ noch R⁷ sich verbinden, um einen Ring zu bilden, der mit den A-enthaltenden oder N'-enthaltenden Ringen kondensiert.

5. Verfahren nach einem der Ansprüche 1-3, worin R¹ und R² sich verbinden, um einen 5- oder 6-gliedrigen carbocyclischen oder heterocyclischen Ring zu bilden.

6. Verfahren nach einem der Ansprüche 1-3, worin R⁶ und R⁷ sich verbinden, um einen 5- oder 6-gliedrigen carbocyclischen oder heterocyclischen Ring zu bilden.

7. Verfahren nach Anspruch 1,
worin hinsichtlich Formel (I) und Formel (A):
(A)
(1) AN oder C-R⁸ ist, wo R⁸ ausgewählt ist aus Wasserstoff, Halo, C₁-C₄-Alkyl, Phenyl, C₁-C₄-Alkoxy, C₁-C₄-Alkylthio und Phenoxy;
(2) R⁷ ausgewählt ist aus Wasserstoff, Halo, Nitro, C₁- bis C₄-Alkyl, nicht-substituiertem Amino, C₁- bis C₄-Mono- oder Dialkylamino, Phenyl, Naphthyl, einem heterocyclischen Ring, der einen Ring mit 5 oder 6 Ringatomen oder zwei kondensierte Ringe mit 8-10 Ringatomen aufweist, C₁- bis C₄-Alkylthio, Phenylthio, Phenoxy und C₁- bis C₄-Estern von Hydroxy;
(3) R⁶ ausgewählt ist aus Wasserstoff, Halo, Nitro, C₁- bis C₄-Alkylamino, C₁- bis C₄-Alkoxy und C₁- bis C₄-Estern von Hydroxy;
(4) R⁵ ausgewählt ist aus Wasserstoff, Halo, C₁- bis C₄-Alkyl, Phenyl, Amino und C₁- bis C₄-Mono- oder Dialkylamino;
(5) R¹ ausgewählt ist aus C₁-C₄-Alkyl, C₃-C₆-Cycloalkyl und Aryl.
(6) R² ausgewählt ist aus Wasserstoff, C₁-C₄-Alkyl, C₁-C₄-Alkylthio und Phenyl und
(7) R³ ausgewählt ist aus Wasserstoff, etwa C₁-C₄-Alkoxy und Phenoxy.

8. Verfahren nach einem der Ansprüche 1-7, worin das Molverhältnis des Organosilicium-Reagens zur Verbindung von Formel (A) von 0,5:1 bis 12:1, mehr bevorzugt von 1:1 bis 4:1 ist.

9. Verfahren nach einem der Ansprüche 1-8
worin
(A) das Organosilicium-Reagens ausgewählt ist aus der Gruppe, bestehend aus Chlortrimethylsilan, N,O-Bis(trimethylsilyl)acetamid, N,O-Bis(trimethylsilyl)trifluoracetamid, 1,3-Bis(trimethylsilyl)carbamid, 1,1,1,3,3,3-Hexamethyldisilazan, N-Methyl-N-trimethylsilyltrifluoracetamid, 1-Trimethylsilylimidazol, Trimethylsilyltrifluormethansulfonat, tert-Butyldimethylchlorsilan, 1-(tert-Butyldimethylsilyl)imidazol, Ethyl(trimethylsilyl)acetat, N-tert-Butyldimethyl-N-methyltrifluoracetamid, tert-Butyldimethylsilyltrifluormethansulfonat, tert-Butyldiphenylchlorsilan, tert-Butylmethoxyphenylbromsilan, Dimethylphenylchlorsilan, Triethylchlorsilan, Triethylsilyltrifluormethansulfonat und Triphenylchlorsilan und Mischungen davon; das Organosilicium-Reagens vorzugsweise ausgewählt ist aus N,O-Bis(trimethylsilyl)acetamid, N,O-Bis(trimethysilyl)trifluoracetamid, N-Methyl-N-trimethylsilyltrifluoracetamid und tert-Butyldiphenylchlorsilan und Mischungen davon;
(B) das Organosilicium-Reagens und die Verbindung der Formel (A) bei einer Temperatur von -50 °C bis 250 °C; vorzugsweise von -10 °C bis 160 °C; mehr bevorzugt von 20 °C bis 140 °C umgesetzt werden, und
(C) das Organosilicium-Reagens und die Verbindung der Formel (A) bei einem Druck von 0,5 atm bis 50 atm; vorzugsweise 0,8 atm bis 10 atm; mehr bevorzugt von 1 atm bis 2 atm umgesetzt werden.

10. Verfahren nach einem der Ansprüche 1-9, worin das Organosilicium-Reagens und die Verbindung der Formel (A) in einem Lösungsmittel umgesetzt werden, ausgewählt aus Acetonitril, N-Methylpyrrolidinon (NMP), Dimethylformid, N,N-Dimethylacetamid, Toluen, Xylen, Tetrahydrofuran, Dioxan, 1,2-Dimethoxyethan, Diglyme; mehr bevorzugte Lösungsmittel Acetonitril, Toluen, NMP und Mischungen beliebiger der oben stehenden umfassen.

## Revendications

1. Procédé pour préparer un composé ayant une structure selon la Formule (I), ou un isomère optique, un diastéréomère ou un énantiomère de celui-ci, ou un sel, hydrate ou ester biohydrolysable, amide ou imide acceptable en pharmacie de celui-ci: 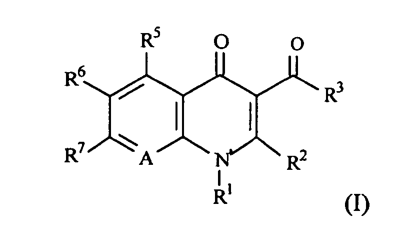 le procédé comprenant la réaction d'un ou plusieurs réactifs organosiliciés avec un composé ayant une structure selon la Formule (A): 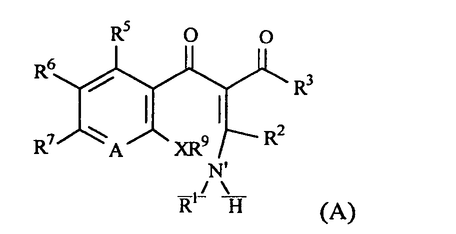 où, en ce qui concerne la Formule (I) et la Formule (A):
(A)
(1) A est N ou C-R⁸, où R⁸ est choisi parmi les hydrogène, alkyle en C₁ à C₁₅, aryle, halo, un hétérocycle, amino, alkylamino en C₁ à C₁₅, arylamino, alkoxy en C₁ à C₁₅ nitro, cyano, aryloxy, esters hydroxy, alkylthio en C₁ à C₁₅, arylthio, aryloxy, esters de thio, alkylsulfonyl- en C₁ à C₁₅, arylsulfonyl-, alkylphosphonyl- en C₁ à C₁₅, arylphosphonyl-, alkylacyl- en C₁ à C₁₅, arylacyl-, et aryl-esters et amides carboxyliques;
(2) R⁷ est choisi parmi les hydrogène, alkyle en C₁ à C₁₅, aryle, un hétérocycle, amino, alkylamino en C₁ à C₁₅, arylamino, halo, nitro, cyano, alkoxy en C₁ à C₁₅, aryloxy, esters d'hydroxy, alkylthio en C₁ à C₁₅, arylthio, esters de thio, alkylsulfonyl- en C₁ à C₁₅, arylsulfonyl-, alkylphosphonyl- en C₁ à C₁₅, arylphosphonyl-, alkylacyl- en C₁ à C₁₅, arylacyl-, et alkyl- en C₁ à C₁₅ et aryl-esters et amides carboxyliques;
(3)R⁶ est choisi parmi les hydrogène, halo, alkyl en C₁ à C₁₅, aryle, un hétérocycle, amino, alkylamino en C₁ à C₁₅, arylamino, nitro, cyano, alcoxy, aryloxy, esters d'hydroxy, alkylthio en C₁ à C₁₅, arylthio, esters de thio, alkylsulfonyl- en C₁ à C₁₅, arylsulfonyl-, alkylphosphonyl- en C₁ à C₁₅, arylphosphonyl-, alkylacyl- en C₁ à C₁₅, arylacyl, et alkyl- en C₁ à C₁₅ et aryl-esters et amides carboxyliques;
(4)R⁵ est choisi parmi les hydrogène, alkyl en C₁ à C₁₅, aryle, cyano, un hétérocycle, amino, alkylamino en C₁ à C₁₅, arylamino, alkylacyl- en C₁ à C₁₅, arylacyl-, et aryl-esters et amides carboxyliques;
(5)R¹ est choisi parmi un carbocycle de 3 à environ 17 chaînons, un hétérocycle, alkyle en C₁ à C₆, alcène en C₁ à C₆, alcyne en C₁ à C₆ et -CH(R¹⁰)(R¹¹) où R¹⁰ est choisi parmi l'alkyle en C₁ à C₆ et le phényle et R¹¹ est -CH₂Y(O=)CR¹² où R¹² est choisi parmi l'alkyle en C₁ à C₆ et le phényle et Y est choisi parmi -NH-, -0- et -S-;
(6)R² est choisi parmi les hydrogène, alkyle en C₁ à C₁₅, aryle, un hétérocycle, alkylthio en C₁ à C₁₅ et arylthio; et
(7)R³ est choisi parmi les hydrogène, alkoxy en C₁ à C₁₅, aryloxy, alkyle en C₁ à C₁₅ et aryle; ou
(B) R¹ et R² peuvent être joints pour former un carbocycle ou hétérocycle à 5 ou 6 chaînons, où A, R³, R⁵, R⁶, R⁷ et R⁸, s'ils sont présents, sont tels que décrits en (A); ou
(C) R⁶ et R⁷ peuvent être joints pour former un carbocycle ou hétérocycle à 5 ou 6 chaînons, où A, R¹, R², R³, R⁵ et R⁸, s'ils sont présents, sont tels que décrits en (A);
et où, en ce qui concerne la formule (A):
(D) X est choisi parmi -O et -S et R⁹ est choisi parmi un alkyle en C₁ à C₁₀, un aryle et un hétéroaryle;
(E) Où les groupes alkyle, hétéroalkyle, cycloalkyle et hétérocycloalkyle ci-dessus peuvent être éventuellement substitués par de 1 à 4 substituants, mais pas de façon à former:
(1) des énols (OH rattaché à un carbone portant une double liaison);
(2) des groupes amino rattachés à un carbone portant une double liaison (à l'exception des amides de type vinylique);
(3) plus d'un hydroxy, amino ou amido rattaché à un seul carbone (sauf lorsque deux atomes d'azote sont rattachés à un seul atome de carbone et tous les trois atomes sont des atomes membres à l'intérieur d'un cycle hétérocycloalkyle);
(4) un hydroxy, amino ou amido rattaché à un carbone auquel est aussi rattaché un hétéroatome;
(5) un hydroxy, amino ou amido rattaché à un carbone auquel est aussi rattaché un halogène.

2. Procédé selon la Revendication 1, dans lequel R⁹ dans la Formule (A) est choisi parmi un alkyle en C₁ à C₄ et un phényle; de préférence R⁹ est choisi parmi un alkyle en C₁ à C₂ non substitué et un phényle non substitué.

3. Procédé selon la Revendication 1, dans lequel R⁹ dans la formule (A) est choisi parmi un alcoxy en C₁ à C₄, thioalkyle (en C₁ à C₄), amyloxy et thioaryle; de préférence R⁹ est choisi parmi les méthoxy, éthoxy, propoxy, -SCH₃, -SCH₂CH₃, -SCH₂CH₂CH₃, phénoxy et -S(C₆H₅).

4. Procédé selon l'une quelconque des Revendications 1 à 3, dans lequel aucun de R¹, R², R⁶ ou R⁷ n'est joint ensemble pour former un cycle condensé aux cycles contenant A ou contenant N'.

5. Procédé selon l'une quelconque des Revendications 1 à 3, dans lequel R¹ et R² sont joints pour former un cycle carbocyclique ou hétérocyclique à 5 ou 6 chaînons.

6. Procédé selon l'une quelconque des Revendications 1 à 3, dans lequel R⁶ et R⁷ sont joints pour former un cycle carbocyclique ou hétérocyclique à 5 ou 6 chaînons.

7. Procédé selon la Revendication 1,
dans lequel, en ce qui concerne la formule (I) et la formule (A):
(A)
(1) A est N ou C-R⁸, où R⁸ est choisi parmi les hydrogène, alkyle en C₁ à C₄, phényle, alkoxy en C₁ à C₄, alkylthio en C₁ à C₄, et phénoxy;
(2) R⁷ est choisi parmi les hydrogène, halo, nitro, alkyl en C₁ à C₄, amino non substitué, mono- ou di-alkylamino en C₁ à C₄, phényle, naphthyle, un hétérocycle ayant un cycle avec 5 ou 6 atomes sur le cycle ou deux cycles condensés avec 8-10 atomes sur les cycles, alkylthio en C₁ à C₄, phénylthio, phénoxy et esters hydroxyliques en C₁ à C₄;
(3)R⁶est choisi parmi les hydrogène, halo, nitro, alkylamino en C₁ à C₄, alkoxy en C₁ à C₄, et esters hydroxyliques en C₁ à C₄;
(4)R⁵est choisi parmi les hydrogène, halo, alkyle en C₁ à C₄, phényle, amino et mono- ou di-alkylamino en C₁ à C₄;
(5)R¹ est choisi parmi les alkyle en C₁ à C₄, cycloalkyle en C₃ à C₆ et aryle.
(6)R² est choisi parmi les hydrogène, alkyle en C₁ à C₄, alkylthio en C₁ à C₄ et phényle; et
(7)R³ est choisi parmi les hydrogène, alcoxy en environ C₁ à C₄ et phénoxy.

8. Procédé selon l'une quelconque des revendications 1 à 7, dans lequel le rapport molaire du réactif organosilicié au composé de Formule (A) est de 0,5/1 à 12/1, plus préférablement de 1/1 à 4/1.

9. Procédé selon l'une quelconque des Revendications 1 à 8, dans lequel:
(A) le réactif organosilicié est choisi dans le groupe constitué par le chlorotriméthylsilane, le N,O-bis(triméthylsilyl)acétamide, le N,Obis(triméthylsilyl)trifluoroacétamide, la 1,3-bis(triméthylsilyl)urée, le 1,1,1,3,3,3-hexaméthyldisilazane, le N-méthyl-N-triméthylsilyltrifluoroacétamide, le 1-triméthylsilylimidazole, le trifluorométhanesulfonate de triméthylsilyle, le tert-butyldiméthylchlorosilane, le 1-(tert-butyldiméthylsilyl)imidazole, l'acétate d'éthyl(triméthylsilyle), le N-tert-butyldiméthyl-N-méthyltrifluoroacétamide, le trifluorométhanesulfonate de tert-butyldiméthylsilyle, le tert-butyldiphénylchlorosilane, le tert-butylméthoxyphénylbromosilane, le diméthylphénylchlorosilane, le triéthylchlorosilane, le trifluorométhanesulfonate de triéthylsilyle, et le triphénylchlorosilane, et leurs mélanges; de préférence le réactif organosilicié est choisi parmi le N,O-bis(triméthylsilyl)acétamide, le N,Obis(triméthysilyl)trifluoroacétamide, le N-méthyl-N-triméthylsilyltrifluoroacétamide et le tert-butyldiphénylchlorosilane, et leurs mélanges;
(B) le réactif organosilicié et le composé de Formule (A) sont mis à réagir à une température de -50 °C à 250 °C; de préférence de -10 °C à 160 °C; plus préférablement de 20 °C à 140 °C; et
(C) le réactif organosilicié et le composé de Formule (A) sont mis à réagir sous une pression de 0,5 atm à 50 atm; de préférence de 0,8 atm à 10 atm; plus préférablement de 1 atm à 2 atm.

10. Procédé selon l'une quelconque des revendications 1 à 9, dans lequel le réactif organosilicié et le composé de Formule (A) sont mis à réagir dans un solvant choisi parmi l'acétonitrile, la N-méthylpyrrolidinone (NMP), le diméthylformamide, le N,N-diméthylacétamide, le toluène,le xylène, le tétrahydrofurane, le dioxane, le 1,2-diméthoxyéthane, le diglyme; les solvants davantage préférés incluent l'acétonitrile, le toluène, la NMP, et les mélanges de n'importe lesquels des précédents.
